# EUROPEAN PATENT APPLICATION

(11) **EP 4 282 861 A1**
(43) Date of publication of application: **29.11.2023**
(21) Application number: 22742734.1
(22) Date of filing: 05.01.2022
(51) Int. Cl.: C07D 263/57, C07D 213/16, C07D 215/06, C07D 307/91, C07D 333/76, C07D 277/66, C07C 15/24, C07C 25/18, C07F 7/08, H01L 51/00

(54) **ORGANIC COMPOUND AND ORGANIC ELECTROLUMINESCENT DEVICE COMPRISING SAME**

(30) Priority: 22.01.2021 KR 20210009036
(71) Applicant: Lapto Co., Ltd., Gyeonggi-do 13215 (KR)
(72) Inventor: KIM, Gyu-ri, Seongnam-si Gyeonggi-do 13215 (KR); KIM, Gyung-woo, Seongnam-si Gyeonggi-do 13215 (KR); JEONG, Yun-cho, Seongnam-si Gyeonggi-do 13215 (KR); HAN, Kap-jong, Seongnam-si Gyeonggi-do 13215 (KR); OH, Eu-gene, Seongnam-si Gyeonggi-do 13215 (KR)
(74) Representative: Hamann, Jean-Christophe
(86) International application number: PCT/KR2022/000115
(87) International publication number: WO 2022/158758

(57) **Abstract**

Provided is an organic compound which contributes to substantial improvements in the luminous efficiency and viewing angle of an organic electroluminescent device. An organic electroluminescent device according to the present invention comprises: a first electrode; a second electrode; an organic layer disposed between the first electrode and the second electrode; and a capping layer, wherein the organic layer or the capping layer comprises an organic compound represented by chemical formula 1 according to the present invention.

## Description

### Technical Field

The present invention relates to an organic compound for use in an organic electroluminescent device and an organic electroluminescent device including the organic compound, particularly an organic electroluminescent device including a capping layer in which the organic compound is used to achieve low refractive index properties.

### Background of the Invention

With the recent trend toward large-sized displays, there is an increasing demand in the display industry for flat display devices that take up small spaces. Liquid crystal displays (LCDs) have a limited viewing angle and require additional light sources because they are not self-luminous. For these reasons, self-luminous organic light emitting diodes (OLEDs) have attracted attention as promising displays.

In 1963, Pope *et al.* have conducted the first research on carrier injection electroluminescence (EL) utilizing a single crystal of an anthracene aromatic hydrocarbon in the field of OLEDs. Based on this research, basic mechanisms such as charge injection, recombination, exciton formation, and light emission in organic materials, and luminescent properties have been understood and studied.

Particularly, various approaches aimed at increasing the luminescent efficiency of devices have been proposed, for example, in connection with changes in the structure of devices and the development of device materials (Sun, S., Forrest, S. R., Appl. Phys. Lett. 91, 263503 (2007)/Ken-Tsung Wong, Org. Lett., 7, 2005, 5361-5364).

A typical OLED display basically has a multilayer structure consisting of an anode, a hole injection layer (HIL), a hole transporting layer (HTL), an emission layer (EML), an electron transporting layer (ETL), and a cathode. That is, the OLED display has a structure in which an organic multilayer film is sandwiched between both electrodes.

In general, organic luminescence refers to a phenomenon in which organic materials are used to convert electrical energy into light energy. An organic light emitting device using organic luminescence usually has a structure including an anode, a cathode, and an organic layer interposed therebetween. The organic layer often consists of one or more layers composed of different materials to increase the efficiency and stability of the organic light emitting device. For example, the organic layer may include a hole injection layer, a hole transporting layer, an emission layer, an electron transporting layer, and an electron injection layer.

When a voltage is applied between the two electrodes of the organic light emitting device, holes and electrons are injected into the organic layer from the anode and the cathode, respectively. The holes recombine with the electrons in the organic layer to form excitons. The excitons fall to the ground state to emit light. Such organic light emitting devices are known to have many advantages, including self-luminescence, high luminance, high efficiency, low driving voltage, wide viewing angle, high contrast, and fast response.

Materials for organic layers of organic light emitting devices can be classified into light emitting materials and charge transporting materials, for example, hole injection materials, hole transporting materials, electron transporting materials, and electron injection materials, by their functions.

Light emitting materials include blue, green, and red light emitting materials that emit different colors of light and yellow and orange light emitting materials that are necessary to obtain more natural colors. Other light emitting materials are host/dopant systems that can be used to achieve high color purity and luminescent efficiency through energy transfer. The principle is that when a small amount of a dopant, whose energy band gap is smaller and whose luminescent efficiency is higher than those of a host as a main material for an emission layer, is introduced into the emission layer, excitons generated from the host are transported to the dopant to emit light with high efficiency. At this time, since the wavelength of the host is shifted to the wavelength band of the dopant, light of a desired wavelength can be obtained depending on the type of the dopant.

Materials for organic layers of organic light emitting devices, for example, hole injection materials, hole transporting materials, light emitting materials, electron transporting materials, and electron injection materials, have been developed to achieve excellent characteristics of the devices. Organic light emitting devices using commercialized materials for organic layers have been recognized for their performance.

However, since the commercialization of organic light emitting devices, characteristics other than the luminescent properties of organic light emitting devices have been taken into consideration.

Most organic light emitting devices are exposed to external light sources for a long time and thus remain exposed to high-energy UV light, which continuously affects organic materials constituting the organic light emitting devices. This problem can be solved by applying UV-absorbing capping layers to organic light emitting devices to prevent their exposure to high-energy light sources.

A typical organic light emitting device is known to have a wide viewing angle but undergoes a large variation in terms of light source's spectrum depending on the viewing angle. This is due to a deviation between the total refractive index of constituent elements and materials (e.g., including a glass substrate, organic materials, and electrode materials) of the organic light emitting device and an optimal refractive index depending on the emission wavelength of the organic light emitting device.

Generally, a high refractive index is required for blue light emission and a lower refractive index is required for a longer wavelength. There is thus a need to develop a material for a capping layer that simultaneously meets the aforementioned requirements in terms of UV absorption characteristics and optimal refractive index.

The efficiency of an organic light emitting device can be generally divided into internal luminescent efficiency and external luminescent efficiency. The internal luminescent efficiency is related to the formation efficiency of excitons in the organic layer for photoconversion.

The external luminescent efficiency refers to the emission efficiency of light from the organic layer outside the organic light emitting device.

Not only high internal luminescent efficiency but also high external luminescent efficiency is required to raise the overall efficiency. Thus, there is a need to develop a compound for a capping layer (CPL) that has new functions to achieve high external luminescent efficiency and prevent many problems caused by long-term exposure to daylight. Particularly, there is a need to develop a material for a capping layer (CPL) that has an outstanding ability to absorb light in a UV wavelength band.

Unlike a bottom-emitting device having a non-resonant structure, a top-emitting device having a resonant structure is constructed such that the produced light is reflected from the anode as a reflective film and is directed toward the cathode, resulting in a significant loss of optical energy due to surface plasmon polaritons (SPPs).

Accordingly, one important approach to improve the shape and efficiency of the EL spectrum is to form a capping layer on the top cathode.

As for SPPs, four metals, including Al, Pt, Ag, and Au, are mainly used for electron emission and surface plasmons are generated on the surfaces of the metal electrodes. For example, when Ag is used for the cathode, emitted light is quenched by SPPs (light energy is lost by Ag), resulting in efficiency reduction.

In contrast, the use of a capping layer causes the generation of SPPs at the interface between a MgAg electrode and an organic material. When the organic material has a high refractive index (for example, n > 1.69 at 620 nm), transverse electric (TE) polarized light is dissipated on the surface of the capping layer in the vertical direction by evanescent waves, the wavelength of transverse magnetic (TM) polarized light traveling along the cathode and the capping layer is amplified by surface plasmon resonance, and as a result, the intensity of the peak increases, achieving high efficiency and enabling effective control over color purity.

However, there remains a need to develop materials and structures of organic light emitting devices that are necessary to improve various characteristics of the devices in a balanced manner while achieving improved efficiency and color purity of the devices.

### Object and Summary of the Invention

### Problems to be Solved by the Invention

One object of the present invention is to provide a material for a capping layer of an organic light emitting device that can be used to improve the luminescent efficiency and lifetime of the device while achieving improved viewing angle characteristics.

A further object of the present invention is to provide a highly efficient and long-lasting organic electroluminescent device including a capping layer with controlled refractive index, particularly to achieve improved light extraction efficiency.

### Means for Solving the Problems

The present invention provides an organic electroluminescent device including a first electrode, an organic layer arranged on the first electrode, a second electrode arranged on the organic layer, and a capping layer arranged on the second electrode wherein the organic layer or the capping layer includes an organic compound represented by Formula 1: 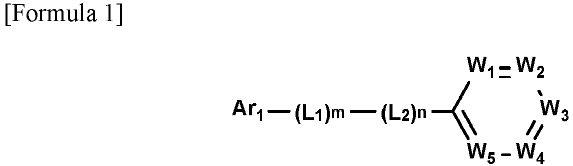 wherein W₁ to W₅ are each independently N or CRₐ, Rₐ is selected from H, F, CF₃, and Si(CH₃)₃, L₁ and L₂ are each independently selected from phenylene, pyridylene, and naphthalene groups, m is 0 or 1, n is an integer from 1 to 5, and Ar₁ is selected from a phenyl group, a phenyl group substituted with F, CF₃ or Si(CH₃)₃, a pyridyl group, a naphthyl group, a quinoline group, an isoquinoline group, a dibenzofuran group, a dibenzothiophene group, a benzoxazole group, a benzothiazole group, and a benzimidazole group.

### Effects of the Invention

The compound described herein can be used as a material for an organic layer of an organic light emitting device.

The use of the compound described herein as a material for a low refractive index capping layer of an organic light emitting device leads to an improvement in the luminous efficiency of the device and a reduction in the full width at half maximum of the emission spectrum, resulting in a significant improvement in the color purity of the device.

The organic electroluminescent device of the present invention has a structure in which a high refractive index organic thin film and a low refractive index thin film are continuously introduced on an MgAg electrode. This structure causes guided-mode resonance, achieving improved viewing angle and optical efficiency of light extracted into the air.

### Brief Description of the Drawings

Fig. 1 shows an exemplary structure of an organic light emitting device according to one embodiment of the present invention in which a first electrode 110, a hole injection layer 210, a hole transporting layer 215, an emission layer 220, an electron transporting layer 230, an electron injection layer 235, a second electrode 120, and a capping layer 300 are stacked in this order on a substrate 100.
Fig. 2 shows the refraction and absorption properties of light when organic compounds according to exemplary embodiments of the present invention were used.

### Best Mode for Carrying out the Invention

As the present invention allows for various changes and numerous embodiments, particular embodiments will be illustrated in drawings and described in detail in the written description. However, this is not intended to limit the present invention to particular modes of practice, and it is to be appreciated that all changes, equivalents, and substitutes that do not depart from the spirit and technical scope of the present invention are encompassed in the present invention.

As used herein, the term "substituted" in the definition of "substituted or unsubstituted" refers to substitution with at least one substituent selected from the group consisting of deuterium and halogen atoms and cyano, nitro, amino, hydroxyl, silyl, boron, phosphine oxide, phosphine sulfide, alkyl, haloalkyl, alkoxy, alkenyl, aryl, heteroaryl, and heterocyclic groups. The term "unsubstituted" in the same definition indicates having no substituent. Each of the substituents exemplified above may be optionally further substituted. For example, a biphenyl group may be interpreted as an aryl group or a phenyl group substituted with a phenyl group.

Examples of the halogen atoms include fluorine, chlorine, bromine, and iodine atoms.

The alkyl groups may be linear, branched or cyclic. The number of carbon atoms in each of the alkyl groups is 1 to 50, preferably 1 to 6. Examples of the alkyl groups include, but are not limited to, methyl, ethyl, n-propyl, isopropyl, n-butyl, s-butyl, t-butyl, i-butyl, 2-ethylbutyl, 3,3-dimethylbutyl, n-pentyl, i-pentyl, neopentyl, t-pentyl, cyclopentyl, 1-methylpentyl, 3-methylpentyl, 2-ethylpentyl, 4-methyl-2-pentyl, n-hexyl, 1-methylhexyl, 2-ethylhexyl, 2-butylhexyl, cyclohexyl, 4-methylcyclohexyl, 4-t-butylcyclohexyl, n-heptyl, 1-methylheptyl, 2,2-dimethylheptyl, 2-ethylheptyl, 2-butylheptyl, n-octyl, t-octyl, 2-ethyloctyl, 2-butyloctyl, 2-hexyloctyl, 3,7-dimethyloctyl, cyclooctyl, n-nonyl, n-decyl, adamantyl, 2-ethyldecyl, 2-butyldecyl, 2-hexyldecyl, 2-octyldecyl, n-undecyl, n-dodecyl, 2-ethyldodecyl, 2-butyldodecyl, 2-hexyldodecyl, 2-octyldodecyl, n-tridecyl, n-tetradecyl, n-pentadecyl, n-hexadecyl, 2-ethylhexadecyl, 2-butylhexadecyl, 2-hexylhexadecyl, 2-octylhexadecyl, n-heptadecyl, n-octadecyl, n-nonadecyl, n-eicosyl, 2-ethyleicosyl, 2-butyleicosyl, 2-hexyleicosyl, 2-octyleicosyl, n-henicosyl, n-docosyl, n-tricosyl, n-tetracosyl, n-pentacosyl, n-hexacosyl, n-heptacosyl, n-octacosyl, n-nonacosyl, and n-triacontyl groups.

As used herein, the term "cyclic hydrocarbon group" refers to any functional group or substituent derived from an alicyclic hydrocarbon. The cyclic hydrocarbon group may be a saturated cyclic hydrocarbon group having 5 to 20 ring carbon atoms.

As used herein, the term "aryl group" refers to any functional group or substituent derived from an aromatic cyclic hydrocarbon ring. The aryl group may be monocyclic or polycyclic. The number of ring carbon atoms in the aryl group may be 6 to 30, preferably 6 to 15. Examples of such aryl groups include, but are not limited to, phenyl, naphthyl, fluorenyl, anthracenyl, phenanthryl, biphenyl, terphenyl, quaterphenyl, quinquephenyl, sexiphenyl, triphenylenyl, pyrenyl, perylenyl, naphthacenyl, benzofluoranthenyl, and chrysenyl groups.

The fluorenyl group may be substituted. In this case, two substituents may be bonded to each other to form a spiro structure.

The heteroaryl group may be interrupted by one or more heteroatoms selected from O, N, P, Si, and S. The N and S atoms may be optionally oxidized and the N atom(s) may be optionally quaternized. The number of ring carbon atoms in the heteroaryl group is 2 to 30 or 2 to 20. The heteroaryl group may be monocyclic or polycyclic. For example, the polycyclic heteroaryl group may have a bicyclic or tricyclic structure.

Examples of such heteroaryl groups include, but are not limited to, thiophenyl, furanyl, pyrrolyl, imidazolyl, pyrazolyl, thiazolyl, oxazolyl, oxadiazolyl, triazolyl, pyridinyl, bipyridinyl, pyrimidinyl, triazinyl, tetrazinyl, triazolyl, tetrazolyl, acridyl, pyridazinyl, pyrazinyl, quinolinyl, quinazolinyl, quinoxalinyl, phenoxazinyl, phthalazinyl, pyridopyrimidinyl, pyridopyrazinopyrazinyl, isoquinolinyl, cinnolinyl, indolyl, isoindolyl, indazolyl, carbazolyl, N-arylcarbazolyl, N-heteroarylcarbazolyl, N-alkylcarbazolyl, benzoxazolyl, benzoimidazolyl, benzothiazolyl, benzocarbazolyl, benzothiophenyl, benzoisothiazolyl, benzoisoxazolyl, dibenzothiophenyl, benzofuranyl, phenanthrolinyl, phenanthridinyl, thiazolyl, isoxazolyl, oxadiazolyl, thiadiazolyl, isothiazolyl, phenothiazinyl, benzodioxolyl, dibenzosilolyl, dibenzofuranyl, and isobenzofuranyl groups. An N-oxide aryl group may correspond to the monocyclic or polycyclic heteroaryl group. The N-oxide aryl group may be, for example, a quaternary salt such as a pyridyl N-oxide or quinolyl N-oxide group but is not limited thereto.

As used herein, the term "silyl group" is intended to include alkylsilyl and aryl silyl groups. Examples of such silyl groups include, but are not limited to, trimethylsilyl, triethylsilyl, t-butyldimethylsilyl, vinyldimethylsilyl, propyldimethylsilyl, triphenylsilyl, diphenylsilyl, and phenylsilyl groups.

As used herein, the term "boron group" is intended to include alkyl boron and aryl boron groups. Examples of such boron groups include, but are not limited to, trimethyl boron, triethyl boron, t-butyl dimethyl boron, triphenyl boron, diphenyl boron, and phenyl boron groups.

The alkenyl groups may be linear or branched. The number of carbon atoms in each of the alkeyl groups is 2 to 30, preferably 2 to 10, but is not particularly limited thereto. Examples of the alkenyl groups include, but are not limited to, vinyl, 1-butenyl, 1-pentenyl, 1,3-butadienyl, allyl, styrenyl, and styrylvinyl groups.

As used herein, the term "adjacent group" may mean a substituent on an atom directly attached to an atom substituted with the corresponding substituent, another substituent on an atom substituted with the corresponding substituent or a substituent disposed sterically closest to the corresponding substituent. For example, the two methyl groups in 1,2-dimethylbenzene can be interpreted as "adjacent groups" and the two ethyl groups in 1,1-diethylcyclopentene can be interpreted as "adjacent groups".

The present invention provides an organic compound for use in an organic layer and/or a capping layer.

According to one embodiment of the present invention, the organic compound is represented by Formula 1: 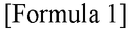 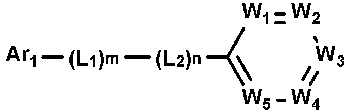 wherein W₁ to W₅ are each independently N or CRₐ, Rₐ is selected from H, F, CF₃, and Si(CH₃)₃, L₁ and L₂ are each independently selected from phenylene, pyridylene, and naphthalene groups, m is 0 or 1, n is an integer from 1 to 5, and Ar₁ is selected from a phenyl group, a phenyl group substituted with F, CF₃ or Si(CH₃)₃, a pyridyl group, a naphthyl group, a quinoline group, an isoquinoline group, a dibenzofuran group, a dibenzothiophene group, a benzoxazole group, a benzothiazole group, and a benzimidazole group.

According to one embodiment of the present invention, the organic compound of Formula 1 may be selected from compounds represented by Formulae 2 to 5: 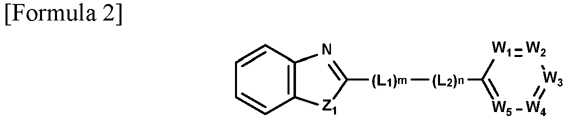 wherein Z₁ is O or S and W₁ to W₅, L₁, L₂, m, and n are as defined in Formula 1, 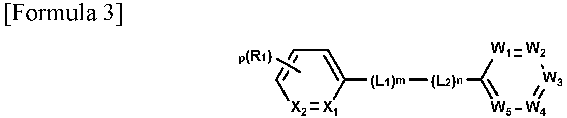 wherein X₁ and X₂ are each independently CH or N (with the proviso that at least one of X₁ and X₂ is CH), R₁ is selected from H, F, CF₃, and Si(CH₃)₃, p is an integer from 0 to 5, and W₁ to W₅, L₁, L₂, m, and n are as defined in Formula 1, 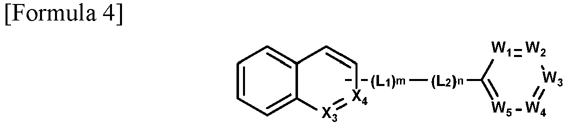 wherein X₃ and X₄ are each independently CH or N (with the proviso that at least one of X₃ and X₄ is CH) and W₁ to W₅, L₁, L₂, m, and n are as defined in Formula 1, and 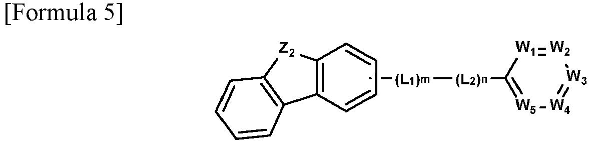 wherein Z₂ is O or S and W₁ to W₅, L₁, L₂, m, and n are as defined in Formula 1.

According to one embodiment of the present invention, the organic compound of Formula 1 may be selected from the compounds represented by Formulae 6:

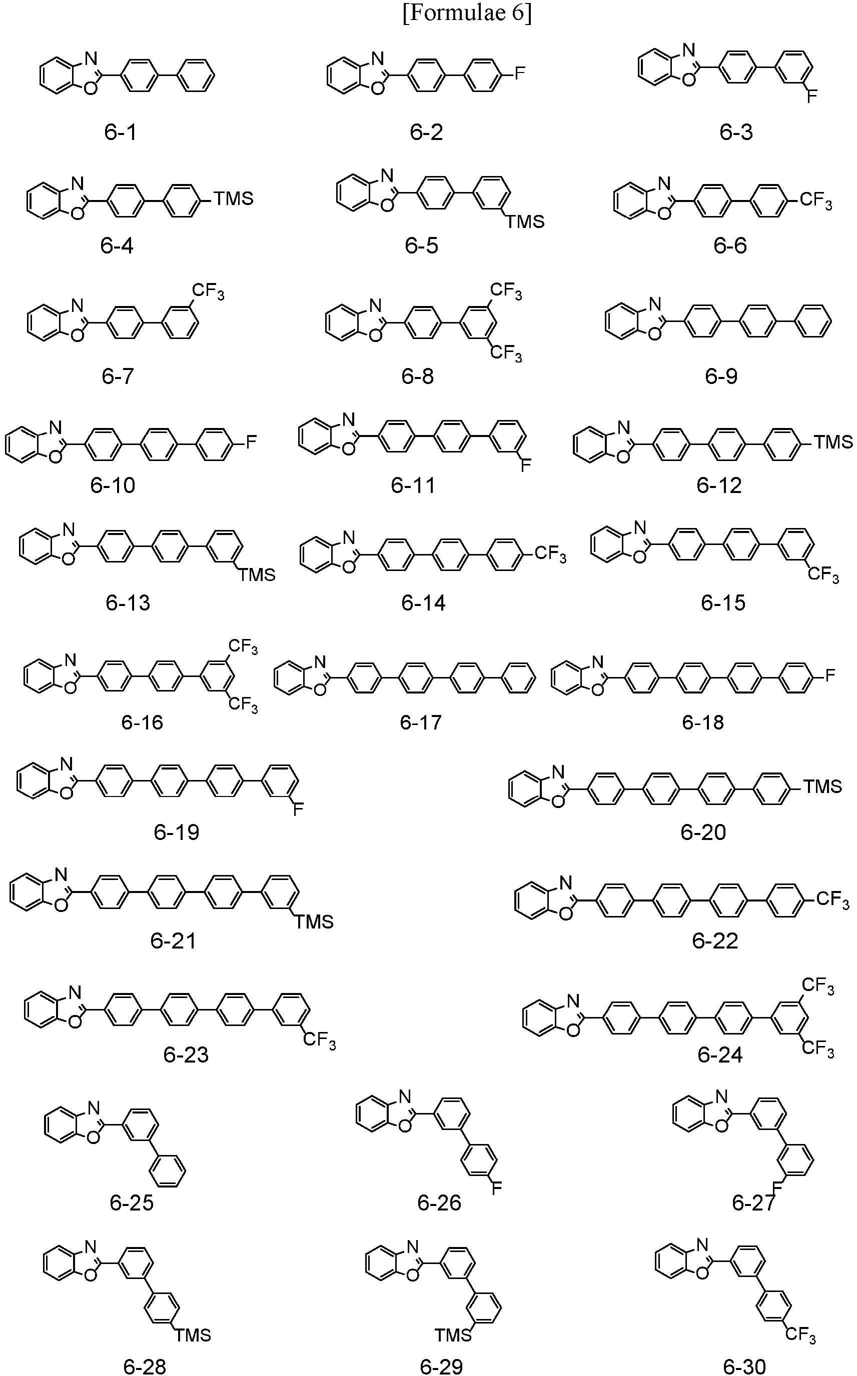

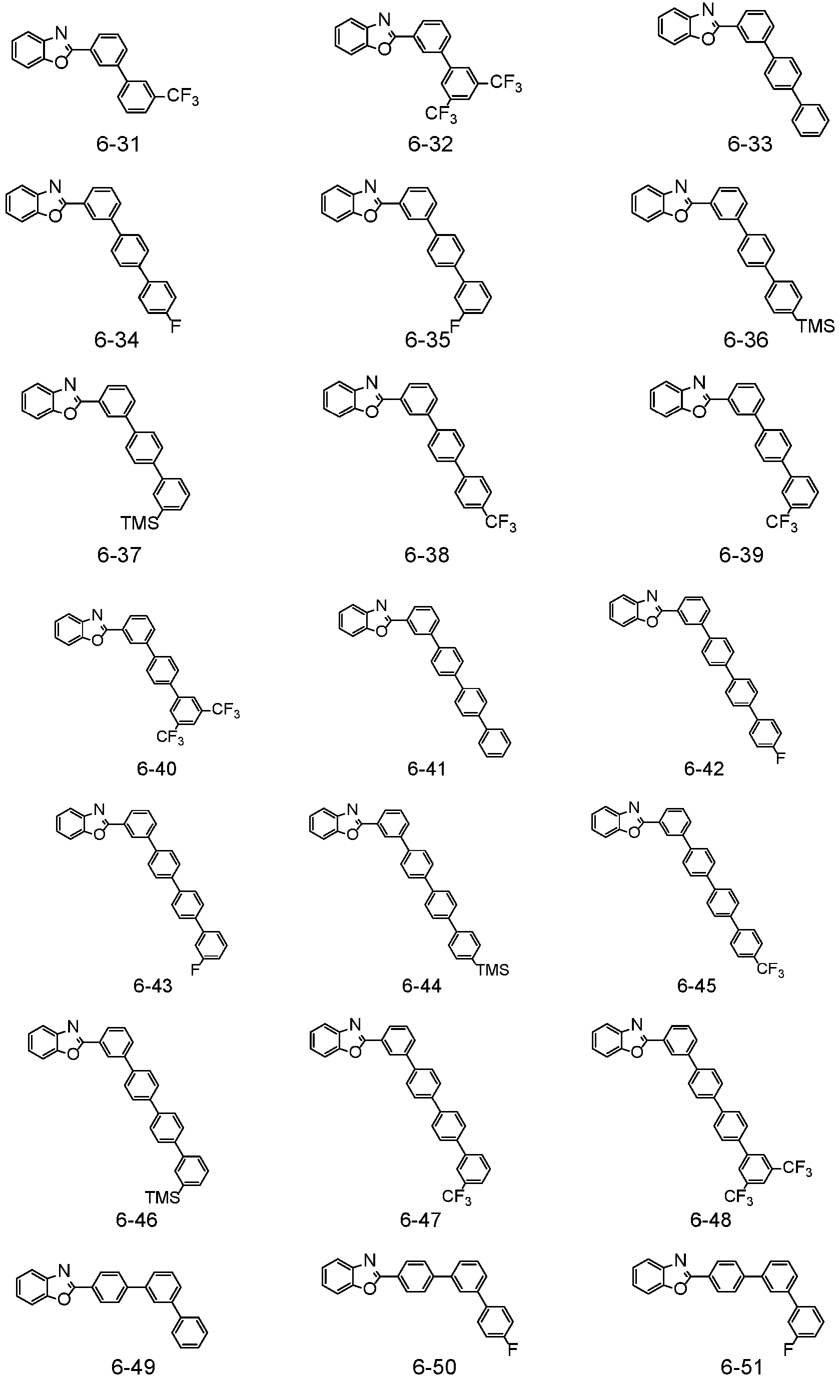

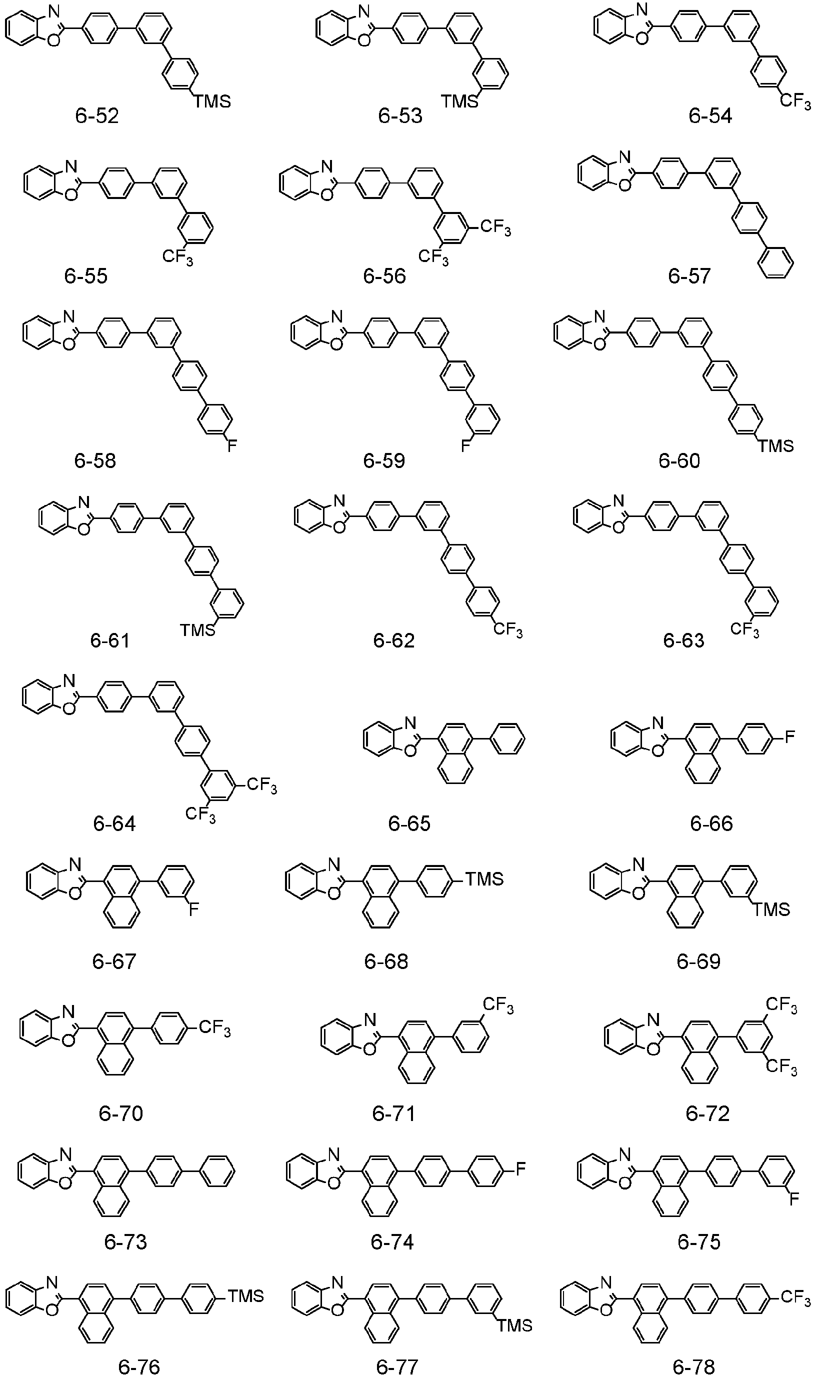

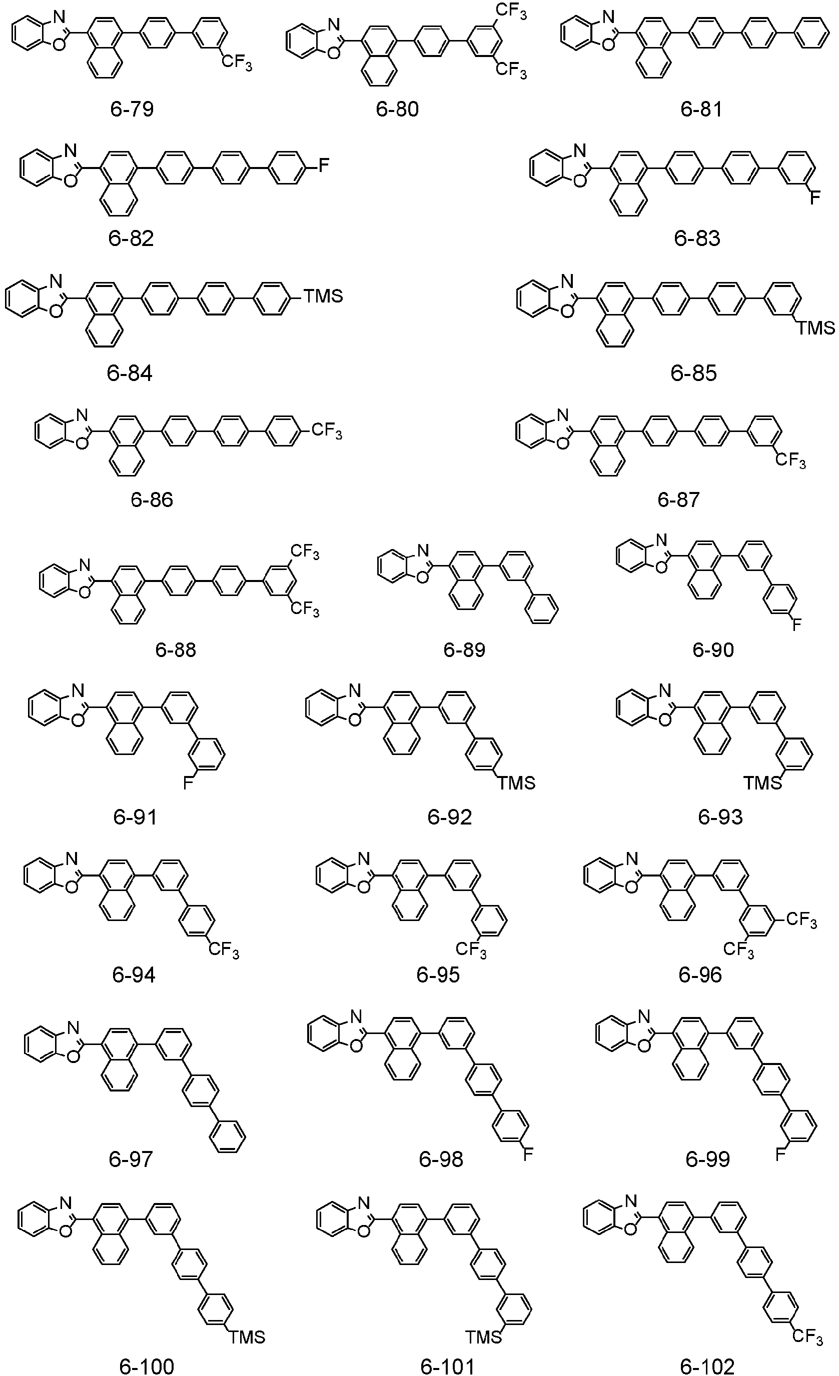

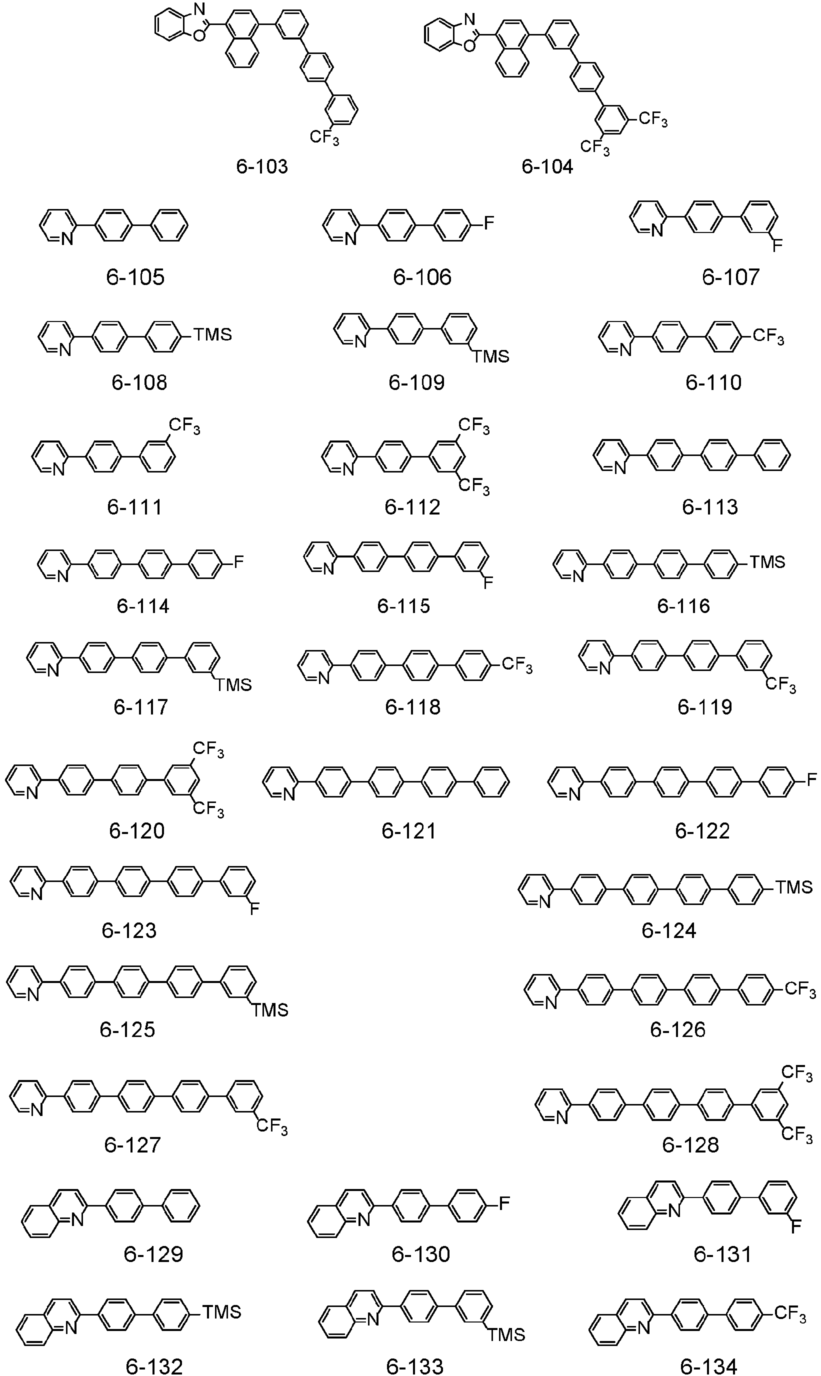

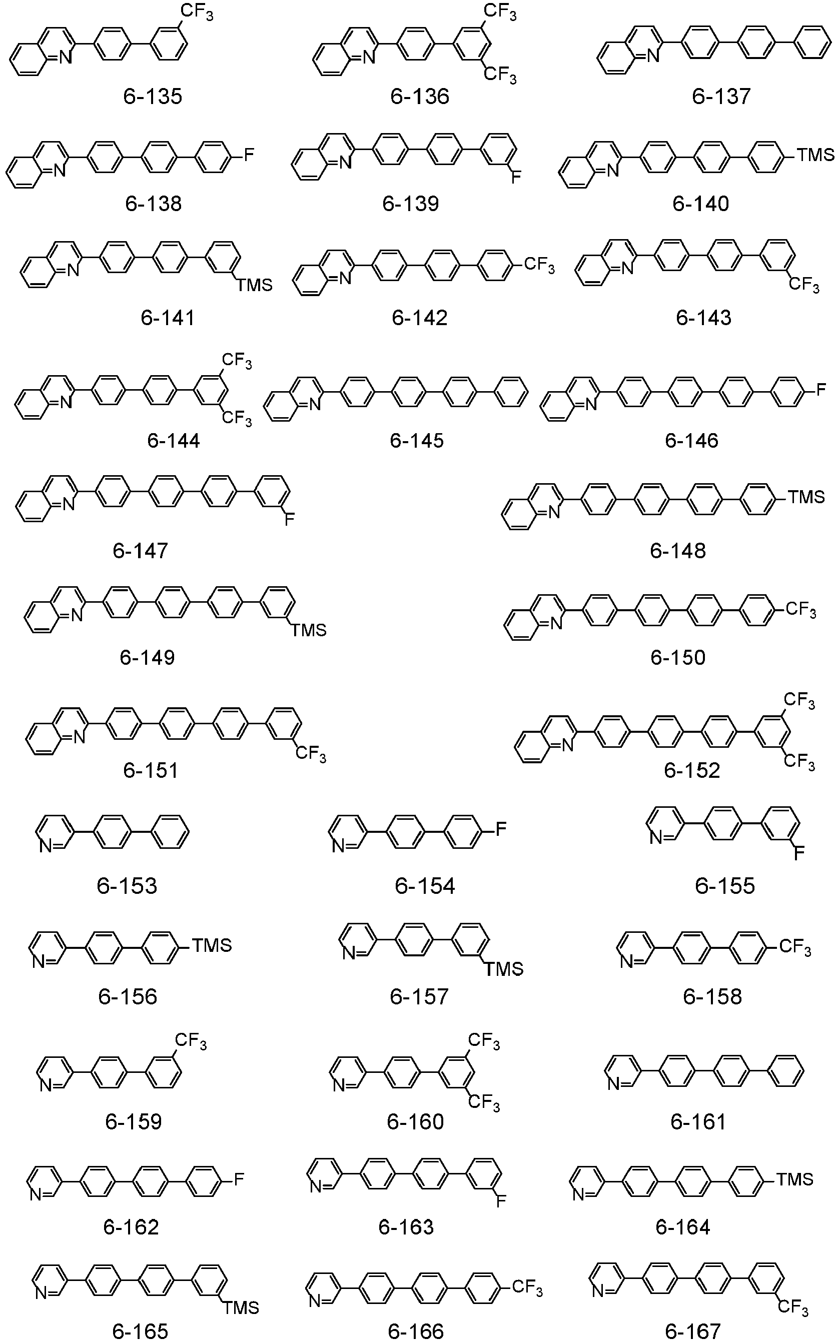

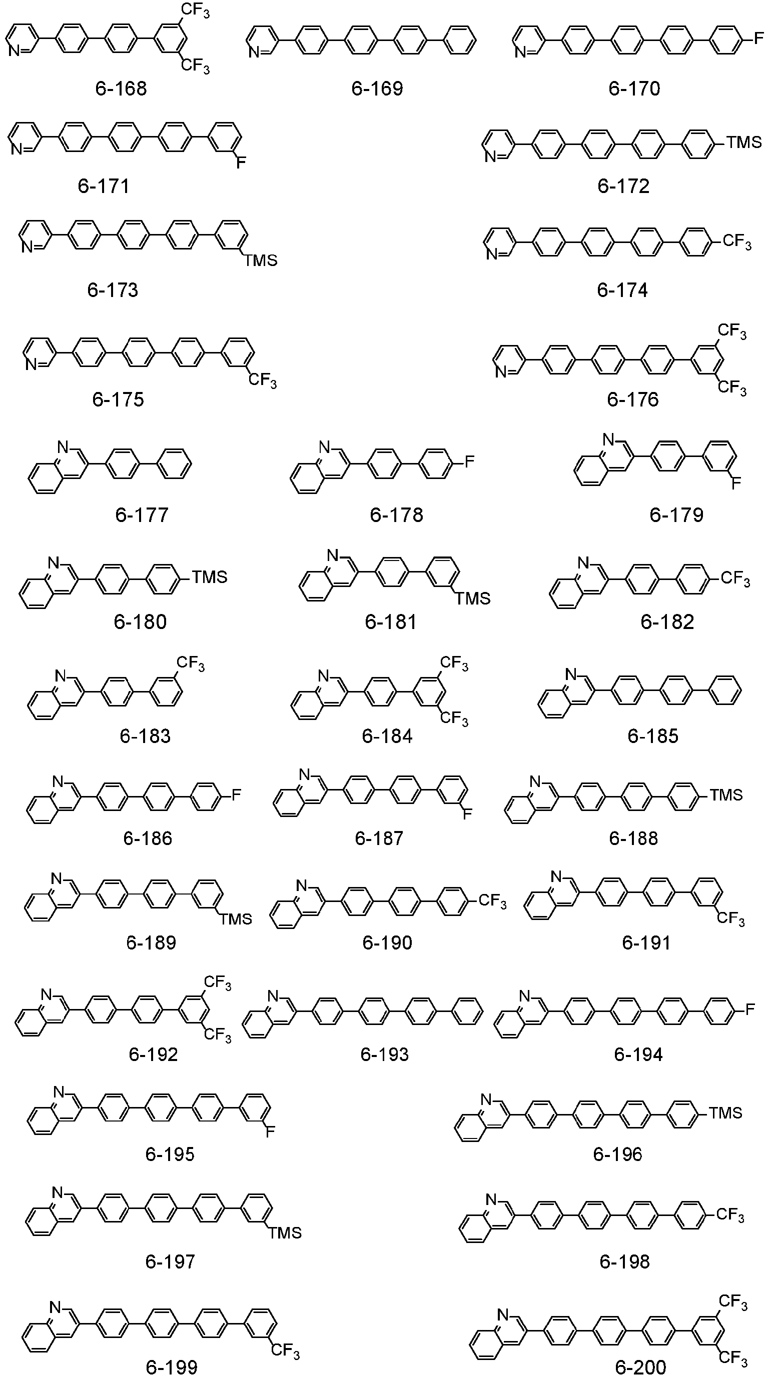

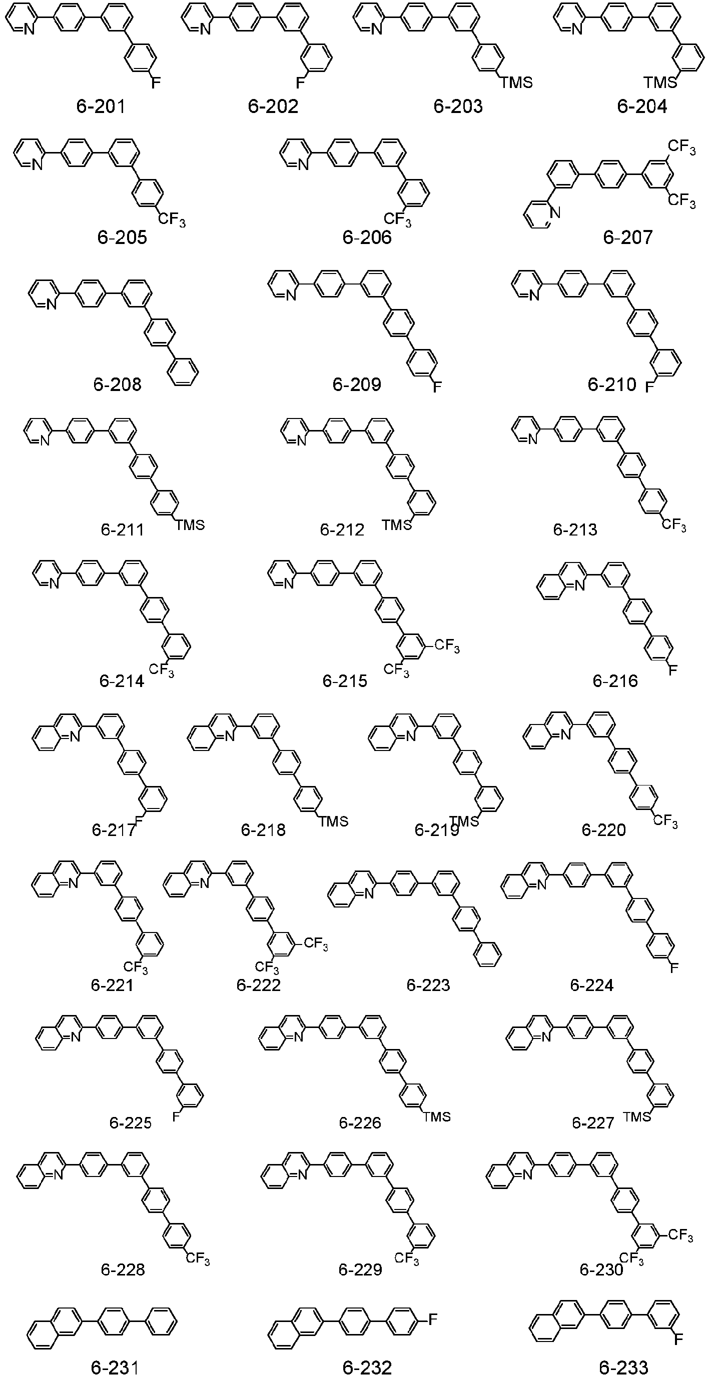

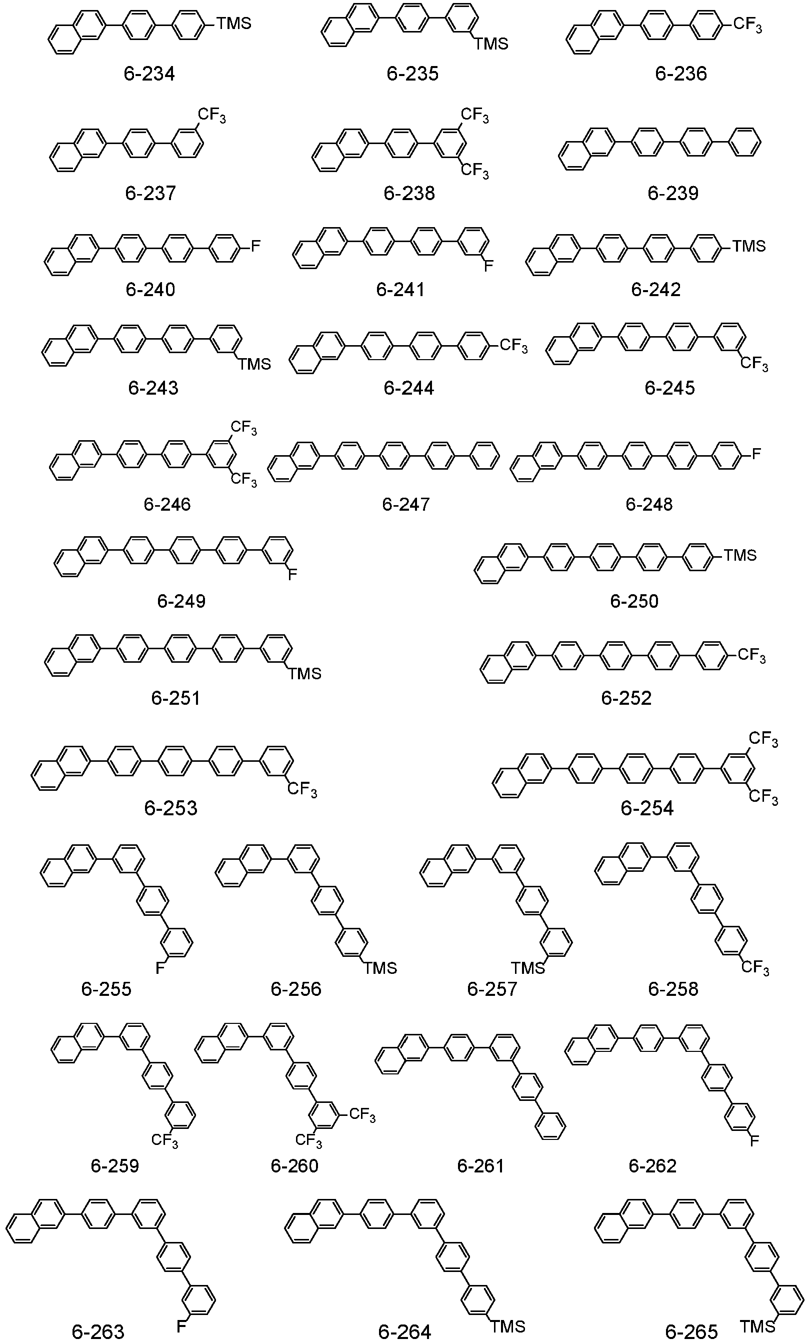

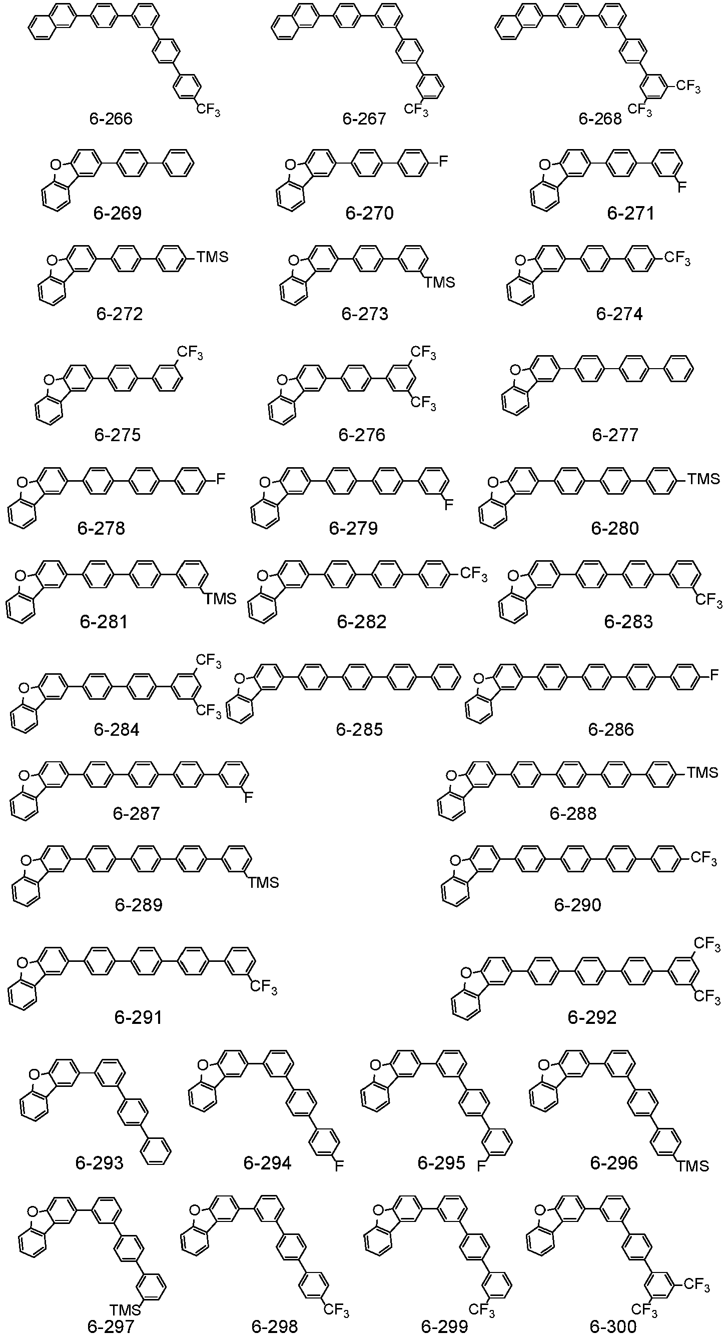

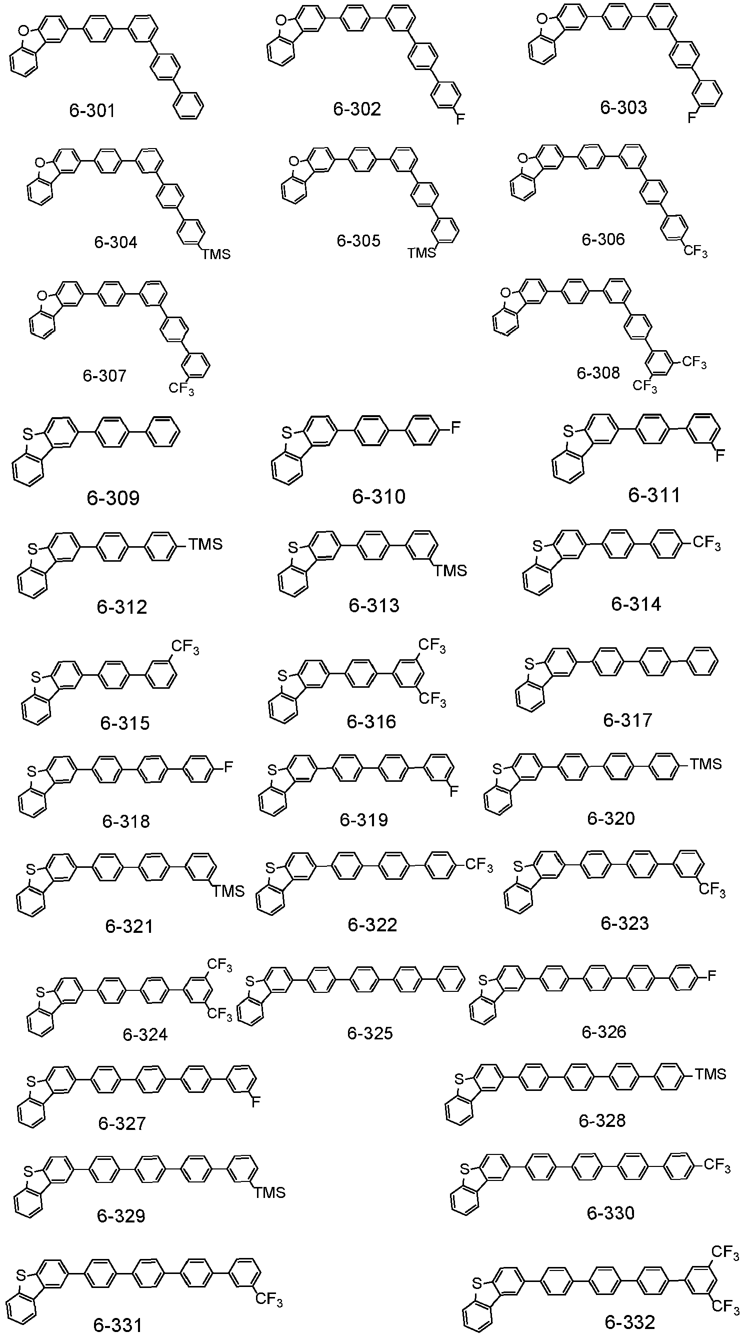

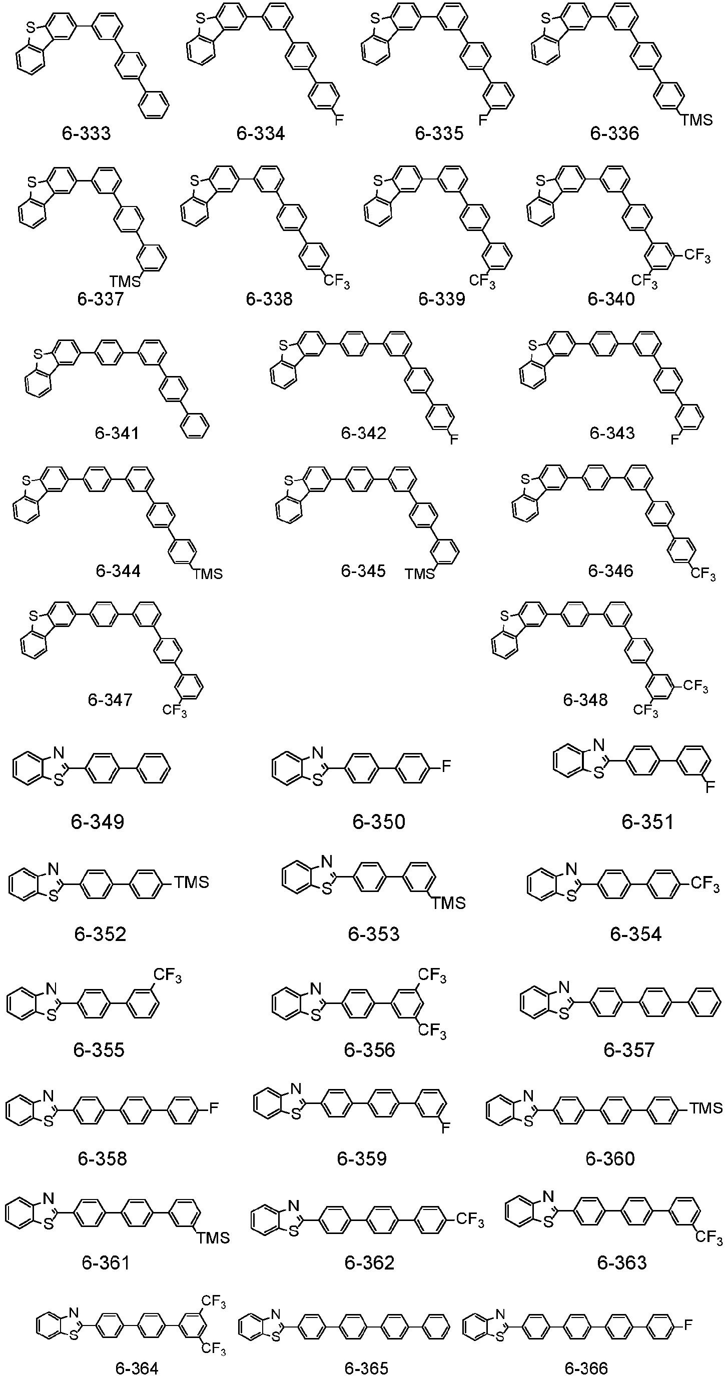

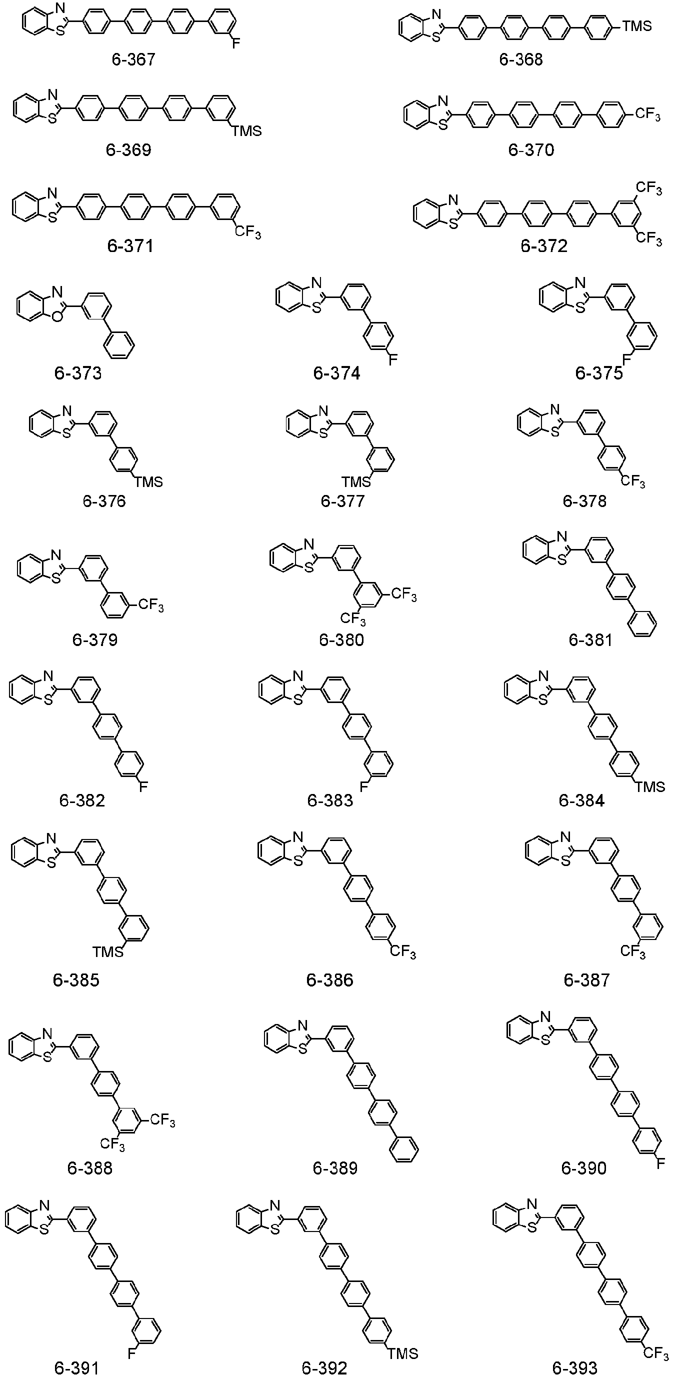

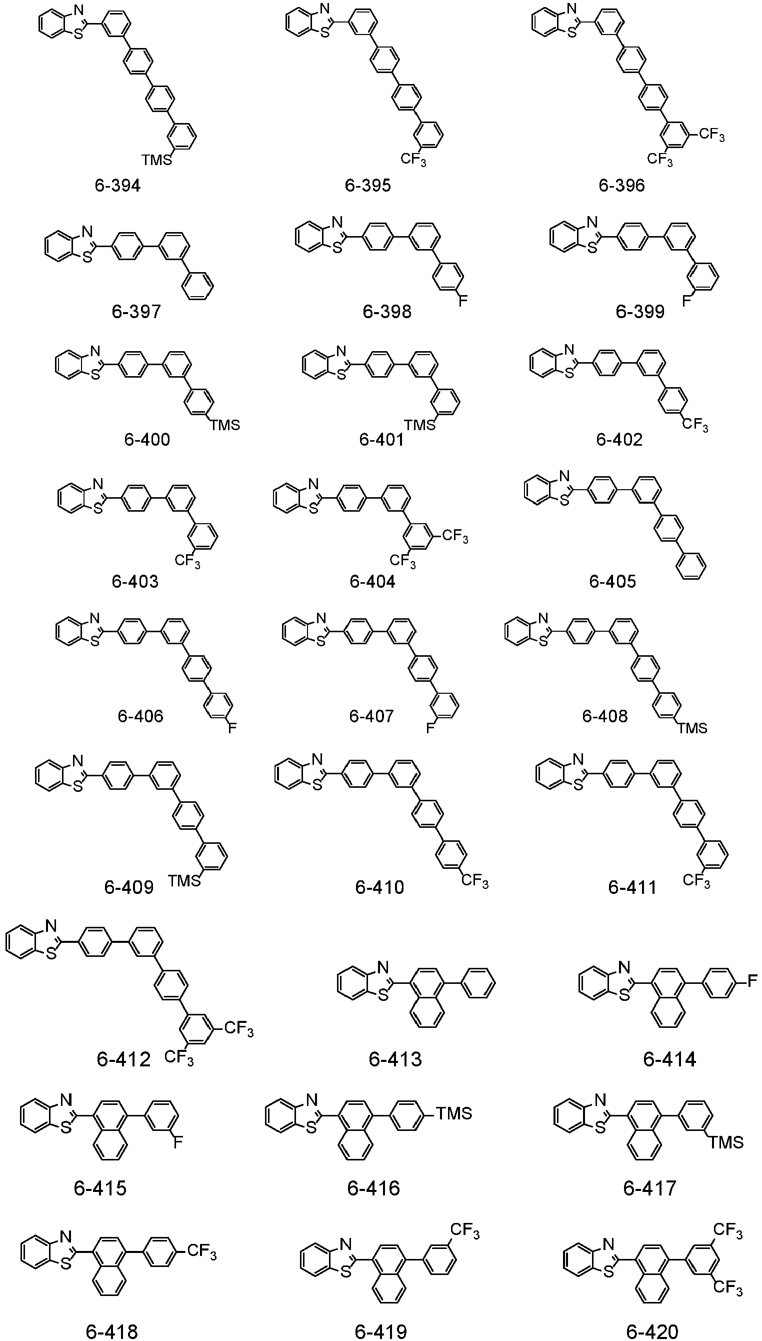

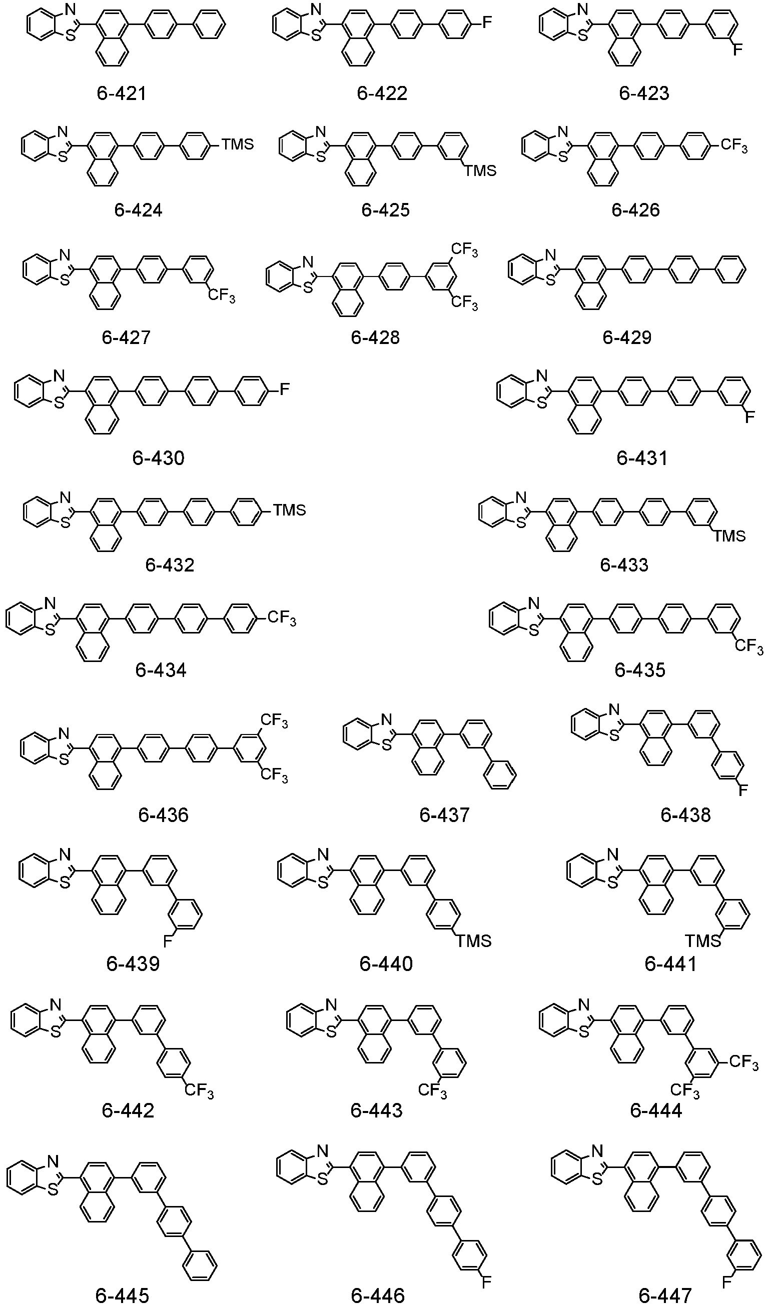

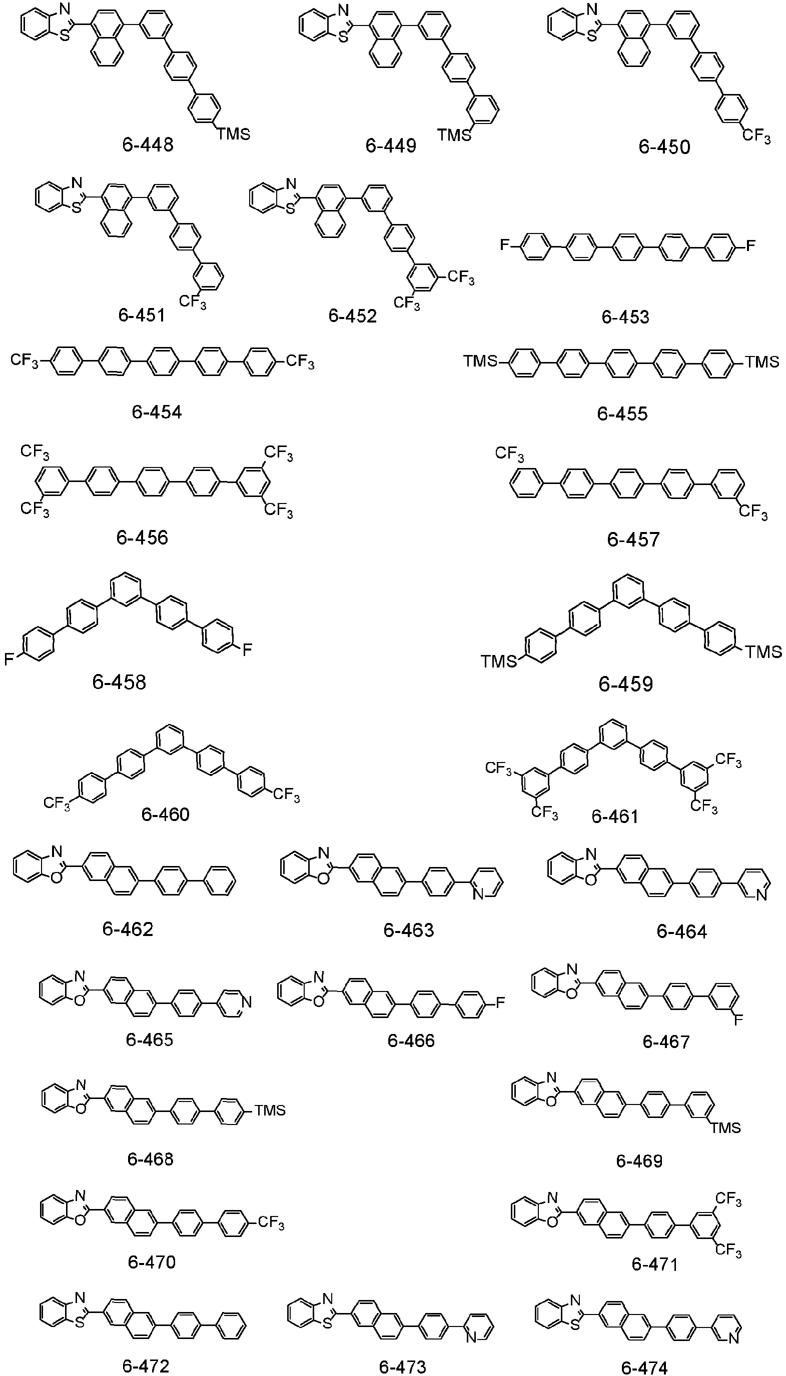

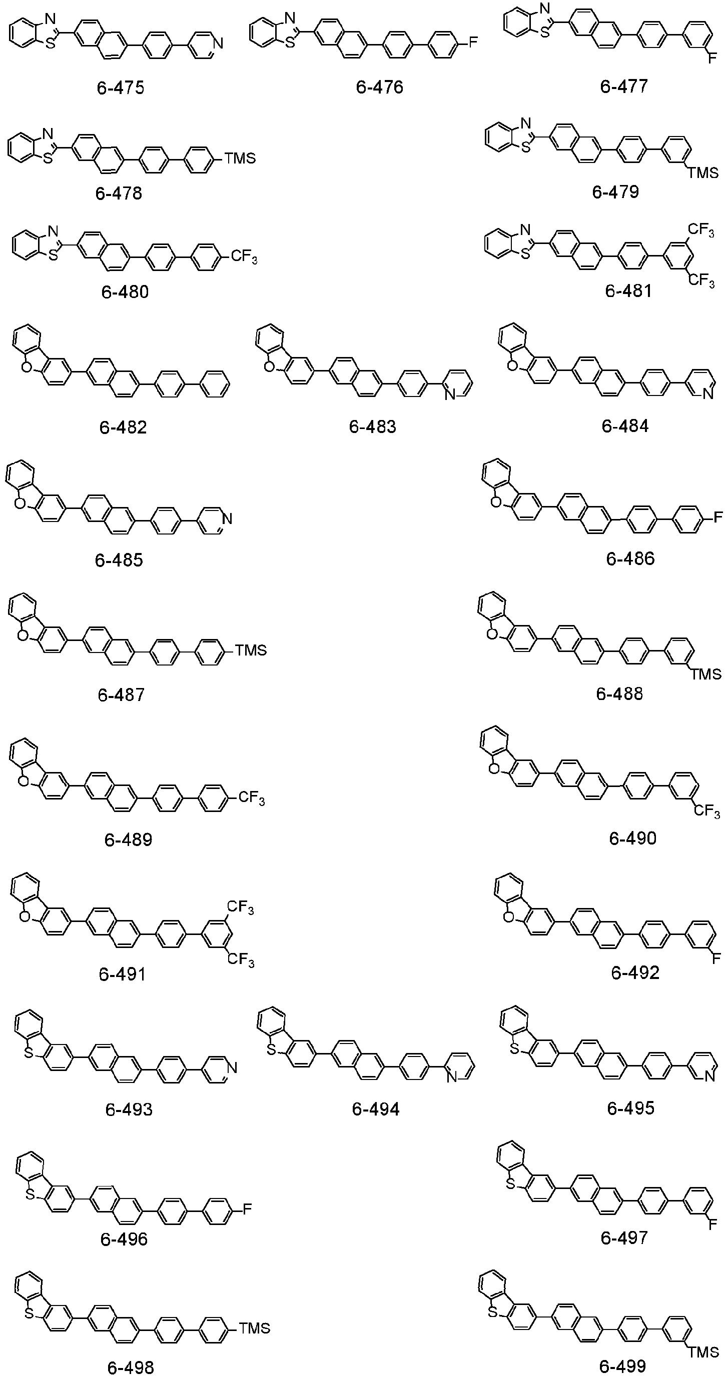

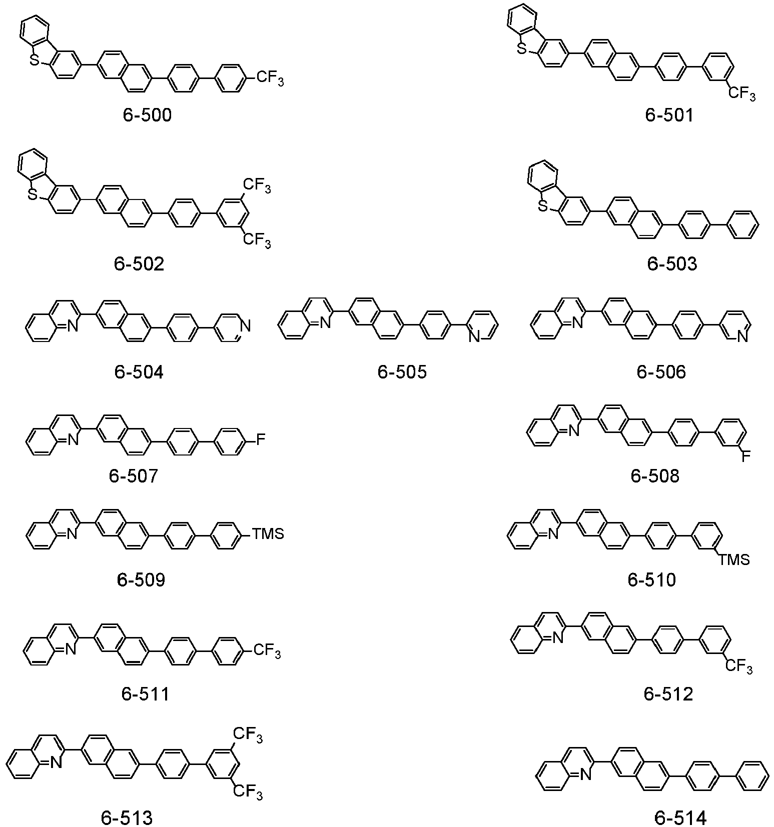

The compounds of Formulae 6 may be further substituted.

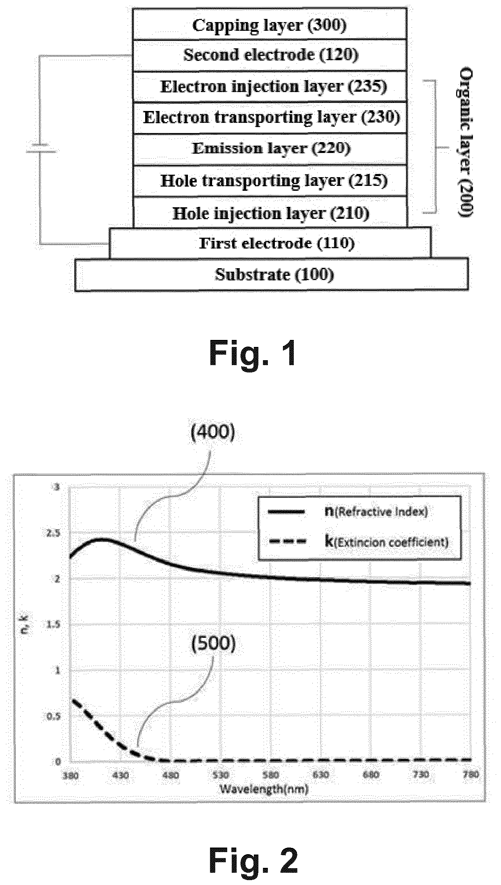

Exemplary embodiments of the present invention will now be described with reference to Figs. 1 and 2.

Fig. 1 is a schematic cross-sectional view of an organic electroluminescent device according to one embodiment of the present invention. Referring to Fig. 1, the organic electroluminescent device includes a first electrode 110, a hole injection layer 210, a hole transporting layer 215, an emission layer 220, an electron transporting layer 230, an electron injection layer 235, a second electrode 120, and a capping layer 300 stacked in this order on a substrate 100.

The first electrode 110 and the second electrode 120 are arranged opposite to each other and an organic layer 200 is arranged therebetween. The hole injection layer 210, the hole transporting layer 215, the emission layer 220, the electron transporting layer 230, and the electron injection layer 235 together form the organic layer 200.

The capping layer 300 is a functional layer deposited on the second electrode 120 and includes an organic material represented by Formula 1.

In the organic electroluminescent device illustrated in Fig. 1, the first electrode 110 is a conductive electrode and may be made of a metal alloy or a conductive material. The first electrode 110 is generally an anode but its function as an electrode is not limited.

The first electrode 110 may be prepared by depositing an electrode material on the substrate 100. Alternatively, electron beam evaporation or sputtering may be used instead of deposition. The material for the first electrode 110 may be selected from high work function materials that facilitate the injection of holes into the organic electroluminescent device.

The capping layer 300 is provided when the organic electroluminescent device is of a top emission type. In this case, a reflective electrode is used as the first electrode 110. Suitable materials for the first electrode include metals and alloys rather than oxides, for example, magnesium (Mg), aluminum (Al), aluminum-lithium (Al-Li), calcium (Ca), magnesium-indium (Mg-In), and magnesium-silver (Mg-Ag). Carbon-based flexible materials such as carbon nanotubes (CNTs) and graphene have also been used in recent years.

The organic layer 200 may consist of a plurality of layers. In this case, the organic layer 200 may include a hole transporting region 210-215 arranged on the first electrode 110, an emission layer 220 arranged on the hole transporting region, and an electron transporting region 230-235 arranged on the emission layer 220.

The capping layer 300 may include an organic compound represented by Formula 1, which will be described below.

The hole transporting region 210-215 is provided on the first electrode 110. The hole transporting region 210-215 may include a hole injection layer 210, a hole transporting layer 215, a hole buffer layer and/or an electron blocking layer (EBL). The hole transporting region 210-215 serves to ensure smooth hole injection and transport into the emission layer. The hole transporting region 210-215 is generally larger in thickness than the electron transporting region because the hole mobility is larger than the electron mobility.

The hole transporting region 210-215 may have a monolayer structure composed of a single material, a monolayer structure composed of different materials or a multilayer structure consisting of a plurality of layers composed of different materials.

For example, the hole transporting region 210-215 may have a monolayer structure consisting of a hole injection layer 210 or a hole transporting layer 215. Alternatively, the hole transporting region 210-215 may have a monolayer structure composed of a hole injection material and a hole transporting material. Alternatively, the hole transporting region 210-215 may have a monolayer structure composed of a plurality of different materials. Alternatively, the hole transporting region 210-215 may have a multilayer structure consisting of a hole injection layer 210 and a hole transporting layer 215 sequentially stacked on the first electrode 110. Alternatively, the hole transporting region 210-215 may have a multilayer structure consisting of a hole injection layer 210, a hole transporting layer 215, and a hole buffer layer sequentially stacked on the first electrode 110. Alternatively, the hole transporting region 210-215 may have a multilayer structure consisting of a hole injection layer 210, a hole buffer layer, a hole transporting layer 215, and a hole buffer layer sequentially stacked on the first electrode 110. Alternatively, the hole transporting region 210-215 may have a multilayer structure consisting of a hole injection layer 210, a hole transporting layer 215, and an electron blocking layer (EBL) sequentially stacked on the first electrode 110. However, the hole transporting region is not limited to the above structures.

The hole injection layer 210 of the hole transporting region 210-215 may be formed on the anode by any suitable method such as vacuum deposition, spin coating, casting or LB deposition. For example, vacuum deposition for the formation of the hole injection layer 210 may be performed at a rate of about 1 Å/s at 100 to 500 °C. The vacuum deposition conditions are not particularly limited and can be freely controlled depending on the kind of a material for the hole injection layer 210 and the desired structure and thermal properties of the hole injection layer 210. Alternatively, the hole injection layer 210 may be formed by spin coating. The coating conditions may vary depending on the kind of a material for the hole injection layer 210 and the characteristics of layers forming interfaces with the hole injection layer 210. An appropriate coating speed and a suitable thermal process for solvent removal after coating are required to make the hole injection layer 210 even.

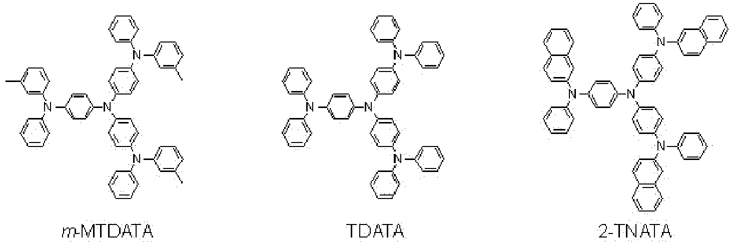

For example, the hole transporting region 210-215 may include one or more of m-MTDATA, TDATA, 2-TNATA, NPB, β-NPB, TPD, spiro-TPD, spiro-NPB, methylated-NPB, TAPC, HMTPD, 4,4',4"-tris(N-carbazolyl)triphenylamine (TCTA), polyaniline/dodecylbenzenesulfonic acid (Pani/DBSA), poly(3,4-ethylenedioxythiophene)/poly(4-styrenesulfonate) (PEDOT/PSS), polyaniline/camphor sulfonic acid (Pani/CSA), and polyaniline/poly(4-styrenesulfonate) (Pani/PSS).

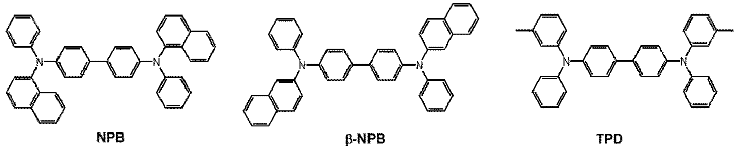

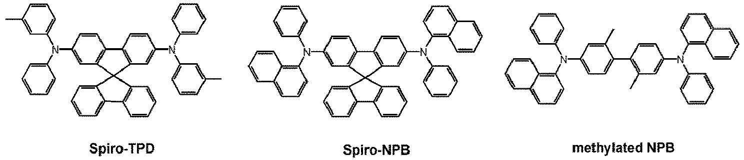

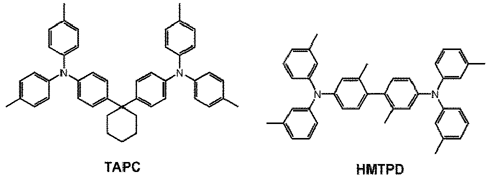

The hole transporting region 210-215 may have a thickness of 100 to 10,000 Å and the constituent organic layers of the hole transporting region 210-215 do not need to have the same thickness. For example, the hole injection layer 210 may have a thickness of 50 Å, the hole transporting layer 215 may have a thickness of 1000 Å, and the electron blocking layer may have a thickness of 500 Å. The thickness of the hole transporting region 210-215 can be determined to such a degree that the efficiency and lifetime of the organic electroluminescent device are satisfactory without an excessive increase in the driving voltage of the organic electroluminescent device.

The organic layer 200 may include one or more layers selected from the group consisting of a hole injection layer 210, a hole transporting layer 215, a functional layer having functions of injecting and transporting holes, a buffer layer, an electron blocking layer, an emission layer 220, a hole blocking layer, an electron transporting layer 230, an electron injection layer 235, and a functional layer having functions of transporting and injecting electrons.

The hole transporting region 210-215 may be doped with a charge generating material to improve the characteristics of the organic electroluminescent device, like the emission layer 220. This doping into the hole transporting region 210-215 can improve the electrical properties of the organic electroluminescent device.

The charge generating material is generally a material with very low HOMO and LUMO energy levels. For example, the LUMO energy level of the charge generating material is similar to the HOMO energy level of a material for the hole transporting layer 215. The low LUMO energy level facilitates hole transfer to the adjacent hole transporting layer 215 based on the electron vacancy in the LUMO, achieving improved electrical properties.

The charge generating material may be, for example, a p-type dopant. Non-limiting examples of such p-type dopants include: quinone derivatives such as tetracyanoquinodimethane (TCNQ) and 2,3,5,6-tetrafluoro-tetracyano-1,4-benzoquinodimethane (F4-TCNQ); metal oxides such as tungsten oxides and molybdenum oxides; and cyano group-containing compounds.

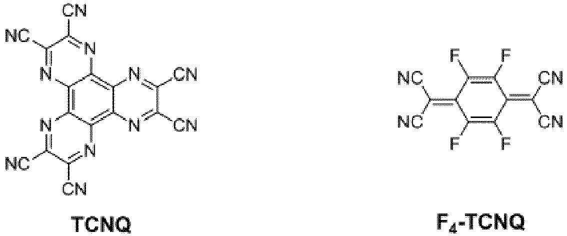

The hole transporting region 210-215 may further include a charge generating material to achieve improved conductivity, in addition to the aforementioned materials.

The charge generating material may be uniformly or non-uniformly dispersed in the hole transporting region 210-215. The charge generating material may be, for example, a p-type dopant. Non-limiting examples of such p-type dopants include: quinone derivatives such as tetracyanoquinodimethane (TCNQ) and 2,3,5,6-tetrafluoro-tetracyano-1,4-benzoquinodimethane (F4-TCNQ); metal oxides such as tungsten oxides and molybdenum oxides; and cyano group-containing compounds.

As described above, the hole transporting region 210-215 may further include a hole buffer layer and/or an electron blocking layer, in addition to the hole injection layer 210 and the hole transporting layer 215. The hole buffer layer may compensate for a resonance distance of light emitted from the emission layer 220 depending on the wavelength of the light to enhance the efficiency of light emission. The hole buffer layer may include the same material as the hole transporting region 210-215.

The electron blocking layer serves to prevent the injection of electrons from the electron transporting region 230-235 into the hole transporting region 210-215. The electron blocking layer may be formed using a material having a high T1 value that can not only block the migration of electrons to the hole transporting region but also can prevent excitons formed in the emission layer 220 from diffusing into the hole transporting region 210-215. For example, a host with a high T1 value for the emission layer 220 is usually used as a material for the electron blocking layer.

The emission layer 220 is provided on the hole transporting region 210-215. For example, the emission layer 220 may have a thickness of 100 Å to 1000 Å or 100 Å to 300 Å. The emission layer 220 may have a monolayer structure composed of a single material, a monolayer structure composed of different materials or a multilayer structure consisting of a plurality of layers composed of different materials.

The emission layer 220 is a region where holes recombine with electrons to form excitons. Materials for the emission layer 220 should have appropriate energy band gaps such that excellent luminescent properties are obtained and a desired color of light is emitted. The materials for the emission layer 220 are typically a host and a dopant but are not limited thereto.

The host may include at least one of TPBi, TBADN, ADN (also called "DNA"), CBP, CDBP, TCP, and mCP but is not limited thereto.

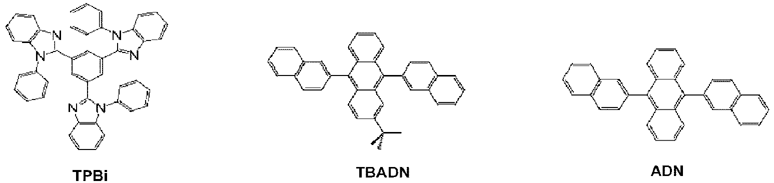

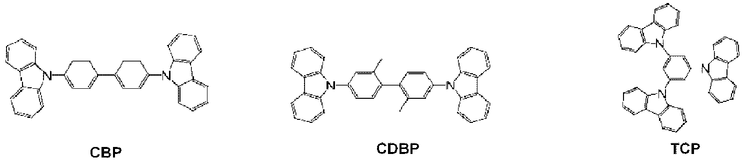

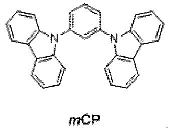

In one embodiment, the dopant may be an organometallic complex. The content of the dopant may generally be selected from 0.01 to 20% but is not limited thereto.

The electron transporting region 230-235 is provided on the emission layer 220. The electron transporting region 230-235 may include a hole blocking layer, an electron transporting layer 230, and/or an electron injection layer 235 but is not limited thereto.

The electron transporting region 230-235 may have a monolayer structure composed of a single material, a monolayer structure composed of different materials or a multilayer structure consisting of a plurality of layers composed of different materials.

For example, the electron transporting region 230-235 may have a monolayer structure consisting of an electron injection layer 235 or an electron transporting layer 230. Alternatively, the electron transporting region 230-235 may have a monolayer structure composed of an electron injection material and an electron transporting material. Alternatively, the electron transporting region 230-235 may have a monolayer structure composed of a plurality of different materials. Alternatively, the electron transporting region 230-235 may have a multilayer structure consisting of an electron transporting layer 230 and an electron injection layer 235 sequentially stacked on the emission layer 220. Alternatively, the electron transporting region 230-235 may have a multilayer structure consisting of a hole blocking layer, an electron transporting layer 230, and an electron injection layer 235 sequentially stacked on the emission electrode 220. However, the electron transporting region is not limited to the above structures. The thickness of the electron transporting region 230-235 may be, for example, 1000 Å to 1500 Å.

The electron transporting region 230-235 may be formed by any suitable method such as vacuum deposition, spin coating, casting, Langmuir-Blodgett (LB) deposition, inkjet printing, laser printing or laser induced thermal imaging (LITI).

The electron transporting layer 230 of the electron transporting region 230-235 may include an anthracene-based compound. Non-limiting examples of suitable materials for the electron transporting layer 230 include, but are not limited to, tris(8-hydroxyquinolinato)aluminum (Alq3), 1,3,5-tri((3-pyridyl)-phen-3-yl)benzene, 2,4,6-tris(3'-(pyridin-3-yl)biphenyl-3-yl)-1,3,5-triazine, 2-(4-(N-phenylbenzoimidazol-1-ylphenyl)-9,10-dinaphthylanthracene, 1,3,5-tri(1-phenyl-1H-benzo[d]imidazol-2-yl)phenyl) (TPBi), 2,9-dimethyl-4,7-diphenyl-1,10- phenanthroline (BCP), 4,7-diphenyl-1,10-phenanthroline (Bphen), 3-(4-biphenylyl)-4-phenyl-5-tert-butylphenyl-1,2,4-triazole (TAZ), 4-(naphthalen-1-yl)-3,5-diphenyl-4H-1,2,4-triazole (NTAZ), 2-(4-biphenylyl)-5-(4-tert-butylphenyl)-1,3,4-oxadiazole (tBu-PBD), bis(2-methyl-8-quinolinolato-N1,O8)-(1,1'-biphenyl-4-olato)aluminum (BAlq), beryllium bis(benzoquinolin-10-olate) (Bebq2), 9,10-di(naphthalene-2-yl)anthracene (ADN). These materials may be used alone or as a mixture thereof.

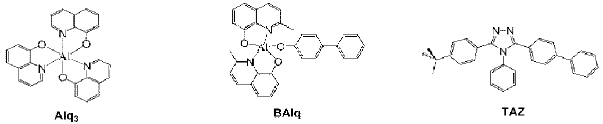

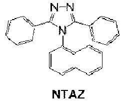

The material for the electron transporting layer 230 may be selected from materials with either high or low electron mobility. The selection varies depending on the structure of the organic light emitting device. The electron transporting layer 230 may be optionally doped with Liq or Li.

The thickness of the electron transporting layer 230 may be in the range of 100 Å to 1000 Å, for example, 150 Å to 500 Å. Within this range, satisfactory electron transporting properties can be obtained without a substantial increase in driving voltage.

The electron injection layer 235 of the electron transporting region 230-235 may include a metal material that facilitates electron injection. Examples of suitable metal materials for the electron injection layer 235 include, but are not limited to, LiF, lithium quinolate (LiQ), Li₂O, BaO, NaCl, CsF, lanthanide metals such as Yb, and metal halides such as RbCl and RbI.

The electron injection layer 235 may be formed of a mixture of an electron transporting material and an insulating organometallic salt. The organometallic salt may be a material having an energy band gap of approximately 4 eV or more. Specific examples of suitable organometallic salts for the electron injection layer include metal acetates, metal benzoates, metal acetoacetates, metal acetylacetonates, and metal stearates. The thickness of the electron injection layer 235 is in the range of 1 Å to 100 Å, preferably 3 Å to 90 Å. Within this range, satisfactory electron injection properties can be obtained without a substantial increase in driving voltage.

As mentioned previously, the electron transporting region 230-235 may include a hole blocking layer. For example, the hole blocking layer may include at least of 2,9-dimethyl-4,7-diphenyl-1,10-phenanthroline (BCP), 4,7-diphenyl-1,10-phenanthroline (Bphen), and Balq. However, the material for the hole blocking layer is not limited.

The second electrode 120 is provided on the electron transporting region 230-235. The second electrode 120 may be a common electrode or a cathode. The second electrode 120 may be a transmissive or transflective electrode. Unlike the first electrode 110, the second electrode 120 may be made of a combination of a relatively low work function metal, an electrically conductive compound, an alloy, etc.

The second electrode 120 is a transflective or reflective electrode. The second electrode 120 may include lithium (Li), magnesium (Mg), aluminum (Al), aluminum-lithium (Al-Li), calcium (Ca), magnesium-indium (Mg-In), magnesium-silver (Mg-Ag), or a compound or mixture containing the metal or alloy (e.g., a mixture of Ag and Mg). Alternatively, the second electrode 120 may have a multilayer structure consisting of a reflective or transflective film and a transparent conductive film. The material for the reflective or transflective film is the same as that described above for the transflective or reflective electrode. For example, the transparent conductive film may be formed of indium tin oxide (ITO), indium zinc oxide (IZO), zinc oxide (ZnO) or indium tin zinc oxide (ITZO).

Although not shown, the second electrode 120 may be connected to an auxiliary electrode. This connection can reduce the resistance of the second electrode 120.

The substrate 100 on which the electrodes and the organic layer are disposed may be made of a rigid or flexible material. For example, the rigid material may be soda-lime glass, alkali-free glass or aluminosilicate glass and the flexible material may be polycarbonate (PC), polyethersulfone (PES), a cyclic olefin copolymer (CEC), polyethylene terephthalate (PET) or polyethylene naphthalate (PEN).

When a voltage is applied to the first and second electrodes 110 and 120 of the organic electroluminescent device, holes are injected from the first electrode 110 and migrate to the emission layer 220 through the hole transporting region 210-215 and electrons are injected from the second electrode 120 and migrate to the emission layer 220 through the electron transporting region 230-235. The electrons recombine with the holes in the emission layer 220 to form excitons, which fall from the excited state to the ground state to emit light.

The path of light generated in the emission layer 220 tends to vary depending on the refractive indices of the organic/inorganic materials constituting the organic electroluminescent device. Only rays of light incident on the second electrode 120 at angles smaller than the critical angle of the second electrode 120 can pass through the second electrode 120. Rays of light coming into contact with the second electrode 120 at angles larger than the critical angle of the second electrode 120 are totally reflected or reflected, and as a result, they cannot be emitted outside the organic electroluminescent device.

A high refractive index of the capping layer 300 reduces the total reflection or reflection of light entering the capping layer 300, contributing to an improvement in luminescent efficiency. An appropriate thickness of the capping layer 300 maximizes the number of micro-cavities, contributing to high efficiency and improved color purity.

The capping layer 300 is placed at the outermost position of the organic electroluminescent device and has a great influence on the characteristics of the device without affecting the driving of the device at all. Therefore, the capping layer 300 is important from both viewpoints of protecting the inside of the organic electroluminescent device and improving the characteristics of the device. The organic materials absorb light energy in specific wavelength ranges depending on their energy band gaps. When the energy band gaps are adjusted such that light in the UV region capable of affecting the organic materials present in the organic electroluminescent device is absorbed, the capping layer 300 containing the organic materials can be used to improve the optical properties of the organic electroluminescent device while protecting the organic electroluminescent device.

The organic light emitting device of the present disclosure may be of a top emission type, a bottom emission type or a dual emission type depending on materials used.

### Mode for Carrying out the Invention

Organic electroluminescent devices and organic compounds according to exemplary embodiments of the present invention will be specifically explained with reference to the following examples, including comparative examples. However, these examples are provided for illustrative purposes to assist in understanding the present invention and are not intended to limit the scope of the present invention is not limited thereto.

### [PREPARATIVE EXAMPLES]

### Intermediate Synthesis Example 1: Synthesis of Intermediate 3

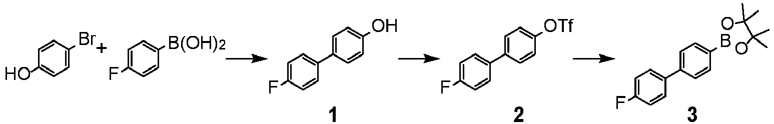

### (Synthesis of Intermediate 1)

50.0 g (289.0 mmol) of 4-bromophenol, 67.3 g (346.8 mmol) of (4-fluorophenyl)boronic acid, 10.0 g (8.7 mmol) of Pd(PPh₃)₄, 119.8 g (867.0 mmol) of K₂CO₃, 1.5 L of toluene, 400 mL of ethanol, and 400 mL of water were mixed in a 3 L four-necked flask. Then, the mixture was stirred at 80 °C for 16 h. After completion of the reaction, the reaction mixture was cooled to room temperature and extracted with ethyl acetate. The organic layer was dried over anhydrous magnesium sulfate, filtered, and distilled under reduced pressure. The residue was dissolved in ethyl acetate, filtered through a pad of celite, and solidified with a mixed solution (Hex/EA) to afford 48.1 g (yield: 88.4%) of Intermediate **1** as an off-white solid.

### (Synthesis of Intermediate 2)

48.1 g (255.6 mmol) of Intermediate **1** was dissolved in 1.2 L of dichloromethane in a 2 L one-necked flask and 41.3 mL (511.2 mmol) of pyridine was added dropwise thereto. Thereafter, the mixture was cooled to 0 °C. After slow dropwise addition of 64.5 mL (383.4 mmol) of Tf₂O, the temperature was raised to room temperature. The reaction was allowed to proceed for 4 h. The reaction mixture was added with purified water, extracted with dichloromethane, and dried over anhydrous magnesium sulfate. The solvent was removed under reduced pressure. The residue was solidified with a mixed solution (MC/MeOH) to afford 47.0 g (yield: 57.4%) of Intermediate **2** as a colorless oil.

### (Synthesis of Intermediate 3)

40.0 g (124.9 mmol) of Intermediate **2**, 38.1 g (149.9 mmol) of bis(pinacolato)diboron, 5.1 g (6.2 mmol) of Pd(dppf)Cl₂-CH₂Cl₂, 36.8 g (374.7 mmol) of KOAc, and 600 mL of 1,4-dioxane were mixed in a 2 L two-necked flask. The mixture was stirred under reflux for 16 h. After completion of the reaction, the reaction mixture was cooled to room temperature, added with purified water, extracted with ethyl acetate, and dried over anhydrous magnesium sulfate. The solvent was removed under reduced pressure. The residue was purified by silica gel column chromatography (toluene) and solidified with hexane at a low temperature to afford 26.3 g (yield: 70.5%) of Intermediate **3** as a white solid.

### Intermediate Synthesis Example 2: Synthesis of Intermediate 5

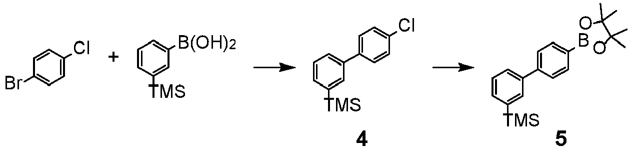

### (Synthesis of Intermediate 4)

30.0 g (156.7 mmol) of 1-bromo-4-chlorobenzene, 31.9 g (164.5 mmol) of (3-(trimethylsilyl)phenyl)boronic acid, 5.4 g (4.7 mmol) of Pd(PPh₃)₄, 65.0 g (470.1 mmol) of K₂CO₃, 500 mL of toluene, 150 mL of ethanol, and 150 mL of distilled water were mixed in a 2 L one-necked flask. Then, the mixture was stirred under reflux for 12 h. After completion of the reaction, the reaction mixture was cooled to room temperature, added with water, extracted with ethyl acetate, and concentrated. The concentrate was purified by column chromatography (Hex) to afford 38.7 g (yield: 94.7%) of Intermediate **4** as a white solid.

### (Synthesis of Intermediate 5)

38.7 g (148.4 mmol) of Intermediate **4, 50.0** g (192.9 mmol) of bis(pinacolato)diboron, 13.6 g (14.8 mmol) of Pd₂(dba)₃, 14.2 g (29.7 mmol) of X-phos, 43.7 g (445.1 mmol) of KOAc, and 600 mL of toluene were mixed in a 2 L one-necked flask. Then, the mixture was stirred at 110 °C for 12 h. After completion of the reaction, the reaction mixture was cooled to room temperature, passed through a pad of celite, and concentrated under reduced pressure. The concentrate was purified by silica gel column chromatography (Hex:CHCl₃) to afford 40.1 g (yield: 76.7%) of Intermediate **5** as a yellow solid.

### Intermediate Synthesis Example 3: Synthesis of Intermediate 6

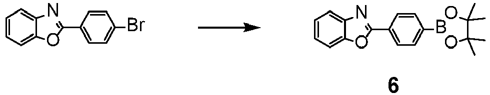

50.0 g (182.4 mmol) of 2-(4-bromophenyl)benzo[d]oxazole, 69.5 g (273.6 mmol) of bis(pinacolato)diboron, 7.5 g (9.1 mmol) of Pd(dppf)Cl₂-CH₂Cl₂, 54.0 g (547.2 mmol) of KOAc, and 730 mL of 1,4-dioxane were mixed in a 2 L one-necked flask. Then, the mixture was stirred at 100 °C for 12 h. After completion of the reaction, the reaction mixture was cooled to room temperature, passed through a pad of celite, and concentrated under reduced pressure. The concentrate was purified by silica gel column chromatography (CHCl₃) and solidified with a mixed solvent (DCM/EtOH) to afford 52.5 g (yield: 89.6%) of Intermediate **6** as a white solid.

### Intermediate Synthesis Example 4: Synthesis of Intermediate 8

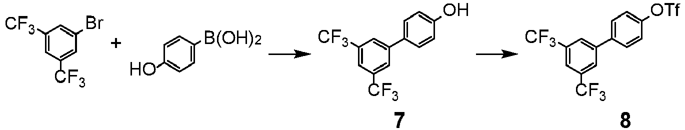

### (Synthesis of Intermediate 7)

12.7 g (92.2 mmol) of 4-hydroxyphenylboronic acid, 18.0 g (61.4 mmol) of 1-bromo-3,5-bis(trifluoromethyl)benzene, 3.6 g (3.1 mmol) of Pd(PPh₃)₄, 25.5 g (18.4 mmol) of K₂CO₃, 240 mL of toluene, 120 mL of ethanol, and 90 mL of distilled water were mixed in a 1 L four-necked flask. Then, the mixture was stirred at 80 °C for 16 h. After completion of the reaction, the reaction mixture was cooled to room temperature and extracted with ethyl acetate. The organic layer was dried over anhydrous magnesium sulfate, filtered, and distilled under reduced pressure. The residue was dissolved in ethyl acetate, filtered through a pad of celite, and solidified with a mixed solution (Hex/EA) to afford 18.8 g (yield: 90.3%) of Intermediate 7 as an off-white solid.

### (Synthesis of Intermediate 8)

18.8 g (61.4 mmol) of Intermediate **7** was dissolved in 170 mL of dichloromethane in a 1 L one-necked flask and 14.9 mL (184.2 mmol) of pyridine was added dropwise thereto. Thereafter, the mixture was cooled to 0 °C. After slow dropwise addition of 15.5 mL (92.1 mmol) of Tf₂O, the temperature was raised to room temperature. The reaction was allowed to proceed for 4 h. The reaction mixture was added with purified water, extracted with dichloromethane, and dried over anhydrous magnesium sulfate. The solvent was removed under reduced pressure. The residue was solidified with a mixed solution (MC/MeOH) to afford 17.7 g (yield: 66.1%) of Intermediate **8** as a colorless oil.

### Intermediate Synthesis Example 5: Synthesis of Intermediate 10

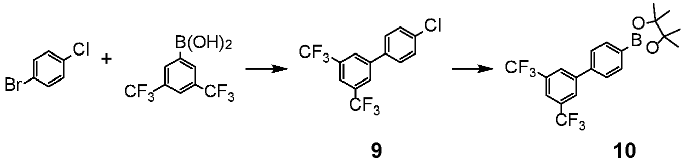

### (Synthesis of Intermediate 9)

30.0 g (156.7 mmol) of 1-bromo-4-chlorobenzene, 40.4 g (156.7 mmol) of (3,5-bis(trifluoromethyl)phenyl)boronic acid, 5.4 g (4.7 mmol) of Pd(PPh₃)₄, 65.0 g (470.1 mmol) of K₂CO₃, 500 mL of toluene, 150 mL of ethanol, and 150 mL of water were mixed in a 2 L one-necked flask. Then, the mixture was stirred under reflux for 12 h. After completion of the reaction, the reaction mixture was cooled to room temperature, added with water, extracted with ethyl acetate, and concentrated. The concentrate was purified by column chromatography (Hex) to afford 50.8 g (yield: 99.9%) of Intermediate **9** as a white liquid.

### (Synthesis of Intermediate 10)

50.8 g (156.5 mmol) of Intermediate **9**, 51.6 g (203.4 mmol) of bis(pinacolato)diboron, 14.3 g (15.7 mmol) of Pd(dba)₂, 14.9 g (31.3 mmol) of X-phos, 46.1 g (436.6 mmol) of KOAc, and 650 mL of toluene were mixed in a 2 L one-necked flask. Then, the mixture was stirred at 110 °C for 12 h. After completion of the reaction, the reaction mixture was cooled to room temperature, passed through a pad of celite, and concentrated under reduced pressure. The concentrate was purified by silica gel column chromatography (Hex:EA) to afford 54.6 g (yield: 83.8%) of Intermediate **10** as a yellow solid.

### Intermediate Synthesis Example 6: Synthesis of Intermediate 11

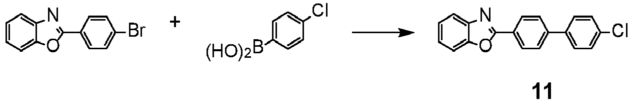

30.0 g (109.4 mmol) of 2-(4-bromophenyl)benzo[d]oxazole, 17.1 g (109.4 mmol) of (4-chlorophenyl)boronic acid, 6.3 g (5.5 mmol) of Pd(PPh₃)₄, 37.8 g (273.6 mmol) of K₂CO₃, 300 mL of toluene, 100 mL of ethanol, and 100 mL of water were mixed together. Then, the mixture was stirred under reflux for 12 h. After completion of the reaction, the reaction mixture was cooled to room temperature. The resulting solid was collected by filtration, washed with water and ethanol, dried, purified by silica gel column chromatography (CHCl₃), and solidified with methanol to afford 15.7 g (yield: 46.9%) of Intermediate **11** as a white solid.

### Intermediate Synthesis Example 7: Synthesis of Intermediate 14

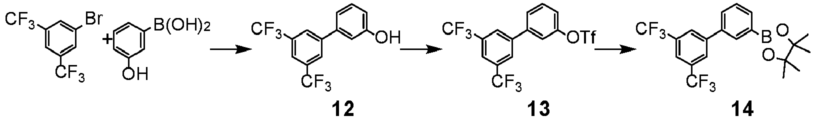

### (Synthesis of Intermediate 12)

7.1 g (51.2 mmol) of (4-hydroxyphenyl)boronic acid, 10.0 g (34.1 mmol) of 1-bromo-3,5-bis(trifluoromethyl)benzene, 2.0 g (1.7 mmol) of Pd(PPh₃)₄, 14.2 g (102.0 mmol) of K₂CO₃, 130 mL of toluene, 70 mL of ethanol, and 50 mL of distilled water were mixed in a 500 mL two-necked flask. Then, the mixture was stirred at 80 °C for 16 h. After completion of the reaction, the reaction mixture was cooled to room temperature and extracted with ethyl acetate. The organic layer was dried over anhydrous magnesium sulfate, filtered, and distilled under reduced pressure. The residue was dissolved in ethyl acetate, filtered through a pad of celite, and solidified with a mixed solution (Hex/EA) to afford 10.4 g (yield: 92.0%) of Intermediate **12** as a pale yellow solid.

### (Synthesis of Intermediate 13)

10.4 g (34.0 mmol) of Intermediate **12** was dissolved in 100 mL of dichloromethane in a 250 mL one-necked flask and 8.2 mL (102.0 mmol) of pyridine was added dropwise thereto. Thereafter, the mixture was cooled to 0 °C. After slow dropwise addition of 8.6 mL (50.9 mmol) of Tf₂O, the temperature was raised to room temperature. The reaction was allowed to proceed for 4 h. The reaction mixture was added with purified water, extracted with dichloromethane, and dried over anhydrous magnesium sulfate. The solvent was removed under reduced pressure. The residue was solidified with a mixed solution (MC/MeOH) to afford 14.7 g (yield: 98.7%) of Intermediate **13** as a white solid.

### (Synthesis of Intermediate 14)

14.7 g (33.5 mmol) of Intermediate **13,** 10.2 g (40.3 mmol) of bis(pinacolato)diboron, 1.4 g (1.7 mmol) of Pd(dppf)Cl₂-CH₂Cl₂, 9.9 g (101.0 mmol) of KOAc, and 130 mL of 1,4-dioxane were mixed in a 500 mL one-necked flask. Then, the mixture was stirred at 110 °C for 12 h. After completion of the reaction, the reaction mixture was cooled to room temperature, passed through a pad of celite, and concentrated under reduced pressure. The concentrate was purified by silica gel column chromatography (Hex:EA) to afford 13.0 g (yield: 92.9%) of Intermediate **14** as a light yellow solid.

### Intermediate Synthesis Example 8: Synthesis of Intermediate 17

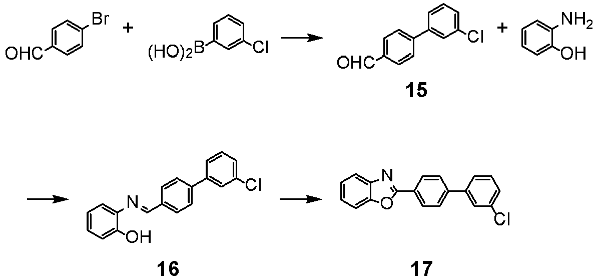

### (Synthesis of Intermediate 15)

40.0 g (216.2 mmol) of 4-bromobenzaldehyde, 40.6 g (259.4 mmol) of 3-chlorophenylboronic acid, 7.5 g (6.5 mmol) of Pd(PPh₃)₄, 60.0 g (432.4 mmol) of K₂CO₃, 400 mL of toluene, 200 mL of ethanol, and 200 mL of distilled water were mixed in a 500 mL two-necked flask. Then, the mixture was stirred at 80 °C for 16 h. After completion of the reaction, the reaction mixture was cooled to room temperature and extracted with ethyl acetate. The organic layer was dried over anhydrous magnesium sulfate, filtered, and distilled under reduced pressure. The residue was dissolved in ethyl acetate, filtered through a pad of celite, and solidified with a mixed solution (Hex/EA) to afford 40.0 g (yield: 85.4%) of Intermediate **15** as a pale yellow solid.

### (Synthesis of Intermediate 16)

40.0 g (183.3 mmol) of Intermediate **15** and 20.0 g (183.3 mmol) of 2-aminophenol were mixed in 900 mL of toluene in a 2 L one-necked flask. Then, the mixture was stirred at 110 °C for 6 h. After completion of the reaction, the reaction mixture was cooled to room temperature and distilled under reduced pressure to afford 56.4 g (yield: 100%) of Intermediate **16** as a yellow solid.

### (Synthesis of intermediate 17)

56.4 g (183.3 mmol) of Intermediate **16** was dissolved in 900 mL of dichlorobenzene in a 2 L one-necked flask and 49.9 g (219.9 mmol) of DDQ was added thereto. Thereafter, the mixture was stirred at room temperature for 3 h. The reaction mixture was filtered through Celite (CHCl₃) and concentrated under reduced pressure. The concentrate was solidified with a mixed solution (DCM/EtOH), filtered, and dried to afford 48.4 g (yield: 86.4%) of Intermediate **17** as a white solid.

### Intermediate Synthesis Example 9: Synthesis of Intermediate 19

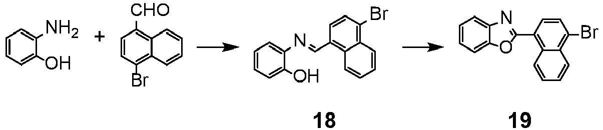

### (Synthesis of Intermediate 18)

25.0 g (229.1 mmol) of 2-aminophenol and 53.8 g (229.1 mmol) of 4-bromo-1-naphthylaldehyde were mixed in 760 mL of toluene in a 2 L one-necked flask. Then, the mixture was stirred at 120 °C for 12 h. After completion of the reaction, the reaction mixture was cooled to room temperature and distilled under reduced pressure to afford 74.7 g (yield: 100%) of Intermediate **18** as a yellow solid.

### (Synthesis of Intermediate 19)

74.7 g (229.1 mmol) of Intermediate **18** was dissolved in 760 mL of dichlorobenzene in a 2 L one-necked flask and 62.4 g (274.9 mmol) of DDQ was added thereto. Thereafter, the mixture was stirred at room temperature for 12 h. The reaction mixture was filtered through Celite (CHCl₃) and concentrated under reduced pressure. The concentrate was solidified with a mixed solution (DCM/EtOH) and filtered to afford 67.8 g (yield: 91.3%) of Intermediate **19** as a gray solid.

### Intermediate Synthesis Example 10: Synthesis of Intermediate 21

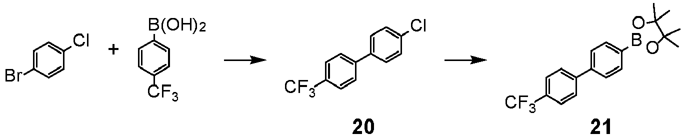

### (Synthesis of Intermediate 20)

10.0 g (52.7 mmol) of 4-(trifluoromethyl)phenyl)boronic acid, 10.1 g (52.7 mmol) of 1-bromo-4-chlorobenzene, 1.8 g (1.6 mmol) of Pd(PPh₃)₄, 14.6 g (105.3 mmol) of K₂CO₃, 120 mL of toluene, 70 mL of purified water, and 70 mL of ethanol were mixed in a 1000 mL two-necked flask. Then, the reaction was allowed to proceed at 110 °C for 5 h. After completion of the reaction, the reaction mixture was cooled to room temperature, added with purified water, and extracted with ethyl acetate. The solvent was removed under reduced pressure. The residue was dissolved in chloroform and filtered through silica gel. The solvent was removed by concentration under reduced pressure. The concentrate was solidified with methanol/hexane to afford 10.1 g (yield: 74.7%) of Intermediate **20** as a white solid.

### (Synthesis of Intermediate 21)

10.1 g (39.4 mmol) of Intermediate **20,** 12.0 g (47.2 mmol) of bis(pinacolato)diboron, 1.9 g (3.4 mmol) of Pd(dba)₂, 3.2 g (6.7 mmol) of X-Phos, 7.7 g (78.7 mmol) of KOAc, and 120 mL of toluene were mixed in a 250 mL two-necked flask. Then, the mixture was stirred under reflux at 110 °C for 5 h. After completion of the reaction, the reaction mixture was cooled to room temperature, added with purified water, and extracted with ethyl acetate. The solvent was removed under reduced pressure. The residue was purified by silica gel column chromatography (Hex:CH₂Cl₂) and solidified with methanol/hexane to afford 10.2 g (yield: 74.4%) of Intermediate **21** as a white solid.

### Intermediate Synthesis Example 11: Synthesis of Intermediate 22

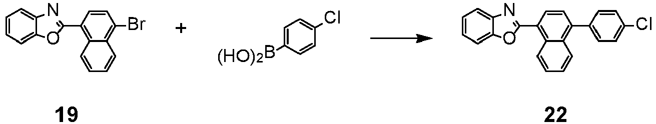

20.0 g (61.7 mmol) of Intermediate **19,** 8.5 g (61.7 mmol) of (4-chlorophenyl)boronic acid, 3.6 g (3.1 mmol) of Pd(PPh₃)₄, 93.0 mL (185.1 mmol) of 2 M K₂CO₃, 200 mL of toluene, and 90 mL of ethanol were mixed together. Then, the mixture was stirred under reflux for 12 h. After completion of the reaction, the reaction mixture was cooled to room temperature, added with water, and extracted with chloroform. Thereafter, the solvent was removed under reduced pressure. The residue was purified by silica gel column chromatography (CHCl₃) and solidified with a mixed solution (DCM/MeOH) to afford 19.3 g (yield: 92.7%) of Intermediate **22** as a white solid.

### Intermediate Synthesis Example 12: Synthesis of Intermediate 24

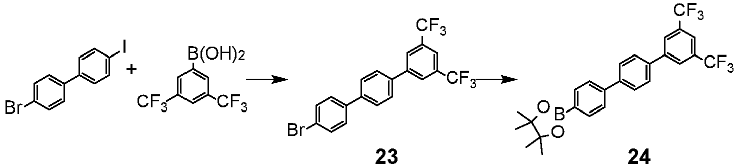

### (Synthesis of intermediate 23)

20.0 g (55.7 mmol) of 4-bromo-4'-iodo-1,1'-biphenyl, 14.3 g (55.7 mmol) of (3,5-bis(trifluoromethyl)phenyl)boronic acid, 1.9 g (1.7 mmol) of Pd(PPh₃)₄, 19.2 g (138.9 mmol) of K₂CO₃, 300 mL of toluene, 150 mL of ethanol, and 150 mL of distilled water were mixed in a 1 L one-necked flask. Then, the mixture was stirred under reflux for 17 h. After completion of the reaction, the reaction mixture was cooled to room temperature, washed with DCM and water, purified by silica gel column chromatography (DCM:Hex), and solidified with a mixed solution (DCM/MeOH) to afford 17.1 g (yield: 68.9%) of Intermediate **23** as an orange solid.

### (Synthesis of Intermediate 24)

10.0 g (22.5 mmol) of Intermediate **23,** 11.4 g (44.9 mmol) of bis(pinacolato)diboron, 550.2 mg (673.8 µmol) of Pd(dppf)₂Cl₂-CH₂Cl₂, 4.4 g (44.9 mmol) of KOAc, and 300 mL of dioxane were mixed in a 1 L one-necked flask. Then, the mixture was stirred at 120 °C for 48 h. After completion of the reaction, the reaction mixture was cooled to room temperature, passed through a pad of celite, and concentrated under reduced pressure. The concentrate was purified by silica gel column chromatography (DCM) and solidified with MeOH to afford 9.6 g (yield: 87.1%) of Intermediate **24** as a white solid.

### Intermediate Synthesis Example 13: Synthesis of Intermediate 25

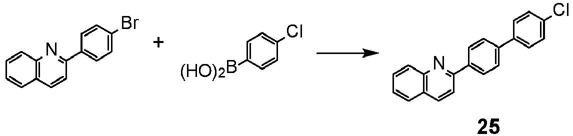

20.0 g (70.4 mmol) of 2-(4-bromophenyl)quinoline, 11.0 g (70.4 mmol) of (4-chlorophenyl)boronic acid, 4.1 g (3.5 mmol) of Pd(PPh₃)₄, 29.2 g (211.2 mmol) of K₂CO₃, 230 mL of toluene, 60 mL of ethanol, and 60 mL of distilled water were mixed in a 1 L one-necked flask. Then, the mixture was stirred under reflux for 12 h. After completion of the reaction, the reaction mixture was cooled to room temperature, added with water, extracted with chloroform, and concentrated. The concentrate was purified by column chromatography (CHCl₃) and solidified with a mixed solvent (DCM/MeOH) to afford 16.1 g (yield: 72.4%) of Intermediate **25** as a white solid.

### Intermediate Synthesis Example 14: Synthesis of Intermediate 27

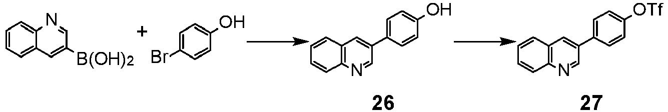

### (Synthesis of Intermediate 26)

50.0 g (289.0 mmol) of 4-bromophenol, 65.0 g (375.7 mmol) of quinolin-3-ylboronic acid, 10.0 g (8.7 mmol) of Pd(PPh₃)₄, 119.8 g (867.0 mmol) of K₂CO₃, 1500 mL of toluene, 360 mL of ethanol, and 360 mL of distilled water were mixed in a 3 L two-necked flask. Then, the reaction was allowed to proceed at 90 °C for 16 h. After completion of the reaction, the reaction mixture was cooled to room temperature, washed with distilled water, and extracted with ethyl acetate. The organic layer was separated, concentrated, and solidified with a mixed solution (acetone:methanol) to afford 55.2 g (yield: 86.3%) of Intermediate **26** as a light brown solid.

### (Synthesis of Intermediate 27)

54.2 g (245.0 mmol) of Intermediate **26** was dissolved in 1.2 L of dichloromethane in a 2 L one-necked flask and 39.6 mL (490.0 mmol) of pyridine was added dropwise thereto. Thereafter, the mixture was cooled to 0 °C. After slow dropwise addition of 53.6 mL (318.5 mmol) of Tf₂O, the temperature was raised to room temperature. The reaction was allowed to proceed for 3 h. The reaction mixture was washed with water and extracted with dichloromethane. The organic layer was separated, dried over anhydrous magnesium sulfate, filtered, and concentrated. The concentrate was purified by silica gel column chromatography (Hex:EA), solidified with a mixed solution (Hex/EA), and filtered to afford 59.3 g (yield: 68.5%) of Intermediate **27** as a light yellow solid.

### Intermediate Synthesis Example 15: Synthesis of Intermediate 28

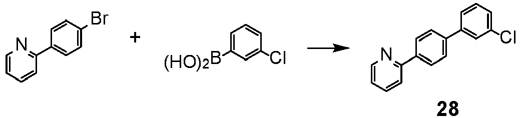

20.0 g (85.4 mmol) of 2-(4-bromophenyl)pyridine, 16.0 g (102.5 mmol) of (3-chlorophenyl)boronic acid, 3.0 g (2.6 mmol) of Pd(PPh₃)₄, 35.4 g (256.3 mmol) of K₂CO₃, 400 mL of toluene, 100 mL of ethanol, and 100 mL of distilled water were mixed in a 3 L two-necked flask. Then, the mixture was stirred at 90 °C for 4 h. After completion of the reaction, the reaction mixture was cooled to room temperature, extracted with ethyl acetate, dried over anhydrous magnesium sulfate, filtered, distilled under reduced pressure, and dissolved in hot toluene. The solution was filtered through a pad of silica gel and solidified with hexane to afford 19.8 g (yield: 87.2%) of Intermediate **28** as a white solid.

### Intermediate Synthesis Example 16: Synthesis of Intermediate 29

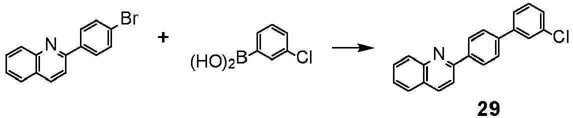

20.0 g (70.4 mmol) of 2-(4-bromophenyl)quinoline, 11.0 g (70.4 mmol) of (3-chlorophenyl)boronic acid, 4.1 g (3.5 mmol) of Pd(PPh₃)₄, 29.2 g (211.2 mmol) of K₂CO₃, 230 mL of toluene, 60 mL of ethanol, and 60 mL of distilled water were mixed in a 1 L one-necked flask. Then, the mixture was stirred under reflux for 12 h. After completion of the reaction, the reaction mixture was cooled to room temperature, added with water, extracted with chloroform, and concentrated. The concentrate was purified by column chromatography (CHCl₃) and solidified with a mixed solvent (DCM/MeOH) to afford 10.3 g (yield: 46.3%) of Intermediate **29** as a white solid.

### Intermediate Synthesis Example 17: Synthesis of Intermediate 32

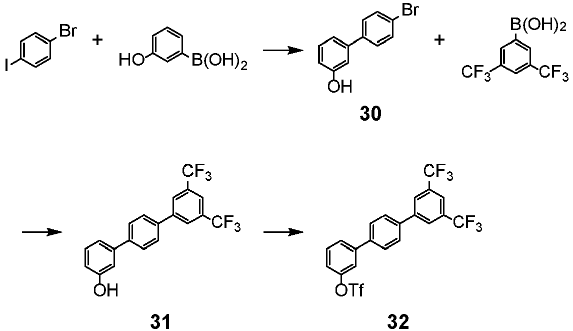

### (Synthesis of intermediate 30)

3.2 g (23.2 mmol) of 3-hydroxyphenylboronic acid, 9.8 g (34.8 mmol) of 1-bromo-4-iodobenzene, 1.3 g (1.2 mmol) of Pd(PPh₃)₄, 35 mL (69.6 mmol) of 2 M K₂CO₃, 120 mL of toluene, and 60 mL of ethanol were stirred under reflux in a 250 mL one-necked flask for 3 h. The reaction mixture was cooled to room temperature and extracted with ethyl acetate. Water and the solvent were removed. The residue was dissolved in dichloromethane, purified by silica gel column chromatography (DCM:HEX), and filtered with hexane to afford 3.9 g (yield: 67.1%) of Intermediate **30** as a white solid.

### (Synthesis of Intermediate 31)

3.9 g (15.6 mmol) of Intermediate **30,** 4.8 g (18.7 mmol) of 3,5-bis(trifluoromethyl)phenylboronic acid, 0.9 g (0.8 mmol) of Pd(PPh₃)₄, 24 mL (46.7 mmol) of 2 M K₂CO₃, 80 mL of toluene, and 40 mL of ethanol were stirred under reflux in a 250 mL one-necked flask all day. The reaction mixture was cooled to room temperature and extracted with ethyl acetate. Water and the solvent were removed. The residue was dissolved in dichloromethane and purified by silica gel column chromatography (DCM:HEX) to afford 5.6 g (yield: 93.8%) of Intermediate **31** as a yellow solid.

### (Synthesis of Intermediate 32)

5.6 g (14.6 mmol) of Intermediate **31** and 3.6 mL (43.8 mmol) of pyridine were added to and stirred in 60 mL of dichloromethane in a 250 mL one-necked flask, and 3.7 mL (21.9 mmol) of trifluoromethanesulfonic anhydride was slowly added dropwise thereto at 0 °C. Thereafter, the mixture was stirred at room temperature for 2 h. After completion of the reaction, distilled water was added to quench the reaction. The reaction mixture was extracted with dichloromethane and water was removed. The residue was purified by silica gel column chromatography (DCM:HEX) to afford 7.6 g (yield: 100%) of Intermediate **32** as a colorless solid.

### Intermediate Synthesis Example 18: Synthesis of Intermediate 33

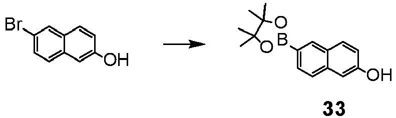

120.0 g (537.9 mmol) of 6-bromonaphthalen-2-ol, 150.3 g (591.7 mmol) of bis(pinacolato)diboron, 17.6 g (21.5 mmol) of Pd(dppf)Cl₂, 211.2 g (1.1 mol) of potassium acetate (KOAc), and 2000 mL of dioxane were placed together in a 3000 mL one-necked flask. The mixture was refluxed under a nitrogen atmosphere at 100 °C all day. After completion of the reaction, the solvent was removed by evaporation. The reaction mixture was added with water and extracted with CHCl₃. The organic layer was dried over anhydrous MgSO₄ and purified by column chromatography (CHCl₃:Hex) to afford 102.2 g (yield: 70.3%) of Intermediate **33** as a slightly yellow solid.

### Intermediate Synthesis Example 19: Synthesis of Intermediate 37

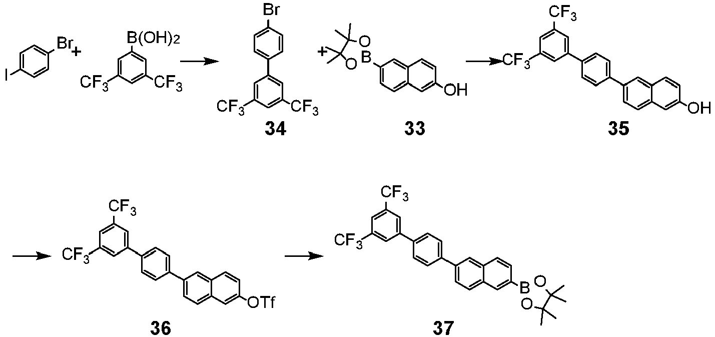

### (Synthesis of Intermediate 34)

20.0 g (70.7 mmol) of 1-bromo-4-iodobenzene, 18.2 g (70.7 mmol) of 3,5-bis(trifluoromethyl)phenylboronic acid, 2.5 g (2.1 mmol) of Pd(PPh₃)₄, 14.7 g (106.1 mmol) of K₂CO₃, 150 mL of toluene, 70 mL of ethanol, and 70 mL of distilled water were placed in a 500 ml one-necked flask. The mixture was heated to reflux with stirring all day. After completion of the reaction, the reaction mixture was cooled to room temperature and the solvent was removed. The residue was passed through a pad of celite with CHCl₃ and the solvent was then distilled off under reduced pressure. Purification by column chromatography (CHCl₃:Hex) afforded 10.4 g (yield: 53.1%) of Intermediate **34** as a pale brown solid.

### (Synthesis of Intermediate 35)

10.3 g (37.9 mmol) of Intermediate **33,** 14.0 g (37.9 mmol) of Intermediate **34,** 1.3 g (1.1 mmol) of Pd(PPh₃)₄, 10.5 g (75.9 mmol) of K₂CO₃, 150 mL of toluene, 70 mL of ethanol, and 70 mL of distilled water were placed in a 500 mL one-necked flask. The mixture was heated to reflux with stirring for 10 h. After completion of the reaction, the reaction mixture was cooled to room temperature and the solvent was removed. The residue was added with distilled water and extracted with DCM. The organic layer was separated, dried over anhydrous MgSO₄, and purified by column chromatography (CHCl₃:Hex) to afford 11.5 g (yield: 70.1%) Intermediate **35** as a white solid.

### (Synthesis of Intermediate 36)

11.5 g (26.6 mmol) of Intermediate **35** and 300 mL of CHCl₃ were placed together in a 500 mL one-necked flask and 3.2 g (39.9 mmol) of pyridine was added thereto under stirring. After slow addition of 11.3 g (39.9 mmol) of trifluoromethanesulfonic anhydride at 0 °C, the temperature was raised to room temperature. The mixture was stirred all day. After the completion of the reaction was confirmed, the reaction mixture was added with distilled water and CHCl₃ and stirred. The organic layer was separated, dried over anhydrous MgSO₄, and purified by column chromatography (CHCl₃:Hex) to afford 7.6 g (yield: 50.3%) of Intermediate **36** as a brown solid.

### (Synthesis of Intermediate 37)

7.6 g (13.5 mmol) of Intermediate **36**, 3.8 g (14.8 mmol) of bis(pinacolato)diboron, 0.4 g (0.5 mmol) of Pd(dppf)Cl₂, 2.6 g (26.9 mmol) of potassium acetate (KOAc), and 150 mL of dioxane were placed together in a 250 mL one-necked flask. The mixture was refluxed under a nitrogen atmosphere at 100 °C all day. After completion of the reaction, the solvent was removed. The reaction mixture was added with distilled water and extracted with CHCl₃. The organic layer was dried over anhydrous MgSO₄ and purified by column chromatography (CHCl₃:Hex) to afford 5.6 g (yield: 76.4%) of Intermediate **37** as a white solid.

### Intermediate Synthesis Example 20: Synthesis of Intermediate 40

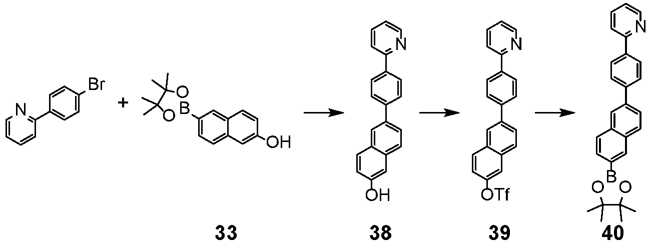

### (Synthesis of Intermediate 38)

10.0 g (42.7 mmol) of 2-(4-bromophenyl)pyridine, 11.5 g (42.7 mmol) of Intermediate **33**, 2.5 g (2.1 mmol) of Pd(PPh₃)₄, 43 mL (85.4 mmol) of 2 M K₂CO₃, 80 mL of toluene, and 40 mL of ethanol were stirred under reflux in a 250 mL one-necked flask for 3 h. The reaction mixture was cooled to room temperature and extracted with ethyl acetate. Water and the solvent were removed. The residue was dissolved in dichloromethane, purified by silica gel column chromatography (DCM:HEX), and filtered with hexane to afford 9.8 g (yield: 77.1%) of Intermediate **38** as a white solid.

### (Synthesis of Intermediate 39)

9.8 g (33.0 mmol) of Intermediate **38** was dissolved in 165 mL of dichloromethane in a 250 mL one-necked flask and 7.8 g (98.9 mmol) of pyridine was added dropwise thereto. Thereafter, the mixture was cooled to 0 °C. After slow dropwise addition of 11.2 g (39.6 mmol) of Tf₂O, the temperature was raised to room temperature. The reaction was allowed to proceed for 4 h. The reaction mixture was added with purified water, extracted with dichloromethane, and dried over anhydrous magnesium sulfate. The solvent was removed under reduced pressure. The residue was solidified with a mixed solution (MC/MeOH) to afford 12.3 g (yield: 86.9%) of Intermediate **39** as a white solid.

### (Synthesis of Intermediate 40)

12.3 g (28.6 mmol) of Intermediate **39,** 8.7 g (34.4 mmol) of bis(pinacolato)diboron, 1.2 g (1.4 mmol) of Pd(dppf)Cl₂, 5.1 g (51.7 mmol) of potassium acetate (KOAc), and 140 mL of dioxane were placed together in a 250 mL one-necked flask. The mixture was refluxed under a nitrogen atmosphere at 100 °C all day. After completion of the reaction, the solvent was removed. The reaction mixture was added with distilled water and extracted with CHCl₃. The organic layer was dried over anhydrous MgSO₄ and purified by column chromatography (CHCl₃:Hex) to afford 10.0 g (yield: 85.7%) of Intermediate **40** as a white solid.

The synthesized intermediates were used to synthesize various organic compounds as follows.

### Synthesis Example 1: Synthesis of Compound 6-9 (LT20-30-477)

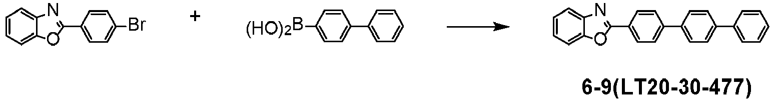

5.0 g (18.2 mmol) of 2-(4-bromophenyl)benzo[d]oxazole, 3.6 g (18.2 mmol) of (1,1'-biphenyl)-4-ylboronic acid, 1.0 g (0.9 mmol) of Pd(PPh₃)₄, 5.0 g (36.5 mmol) of K₂CO₃, 60 mL of toluene, 25 mL of purified water, and 20 mL of ethanol were mixed in a 250 mL two-necked flask. Then, the reaction was allowed to proceed at 90 °C for 3 h. After completion of the reaction, the reaction mixture was cooled to room temperature. Thereafter, the resulting solid was collected by filtration, dissolved in 300 mL of hot MCB, filtered through silica gel, and solidified with chloroform/methanol to give 1.9 g (yield: 30.5%) of Compound **6-9 (LT20-30-477)** as a white solid.

### Synthesis Example 2: Synthesis of compound 6-10 (LT20-30-445)

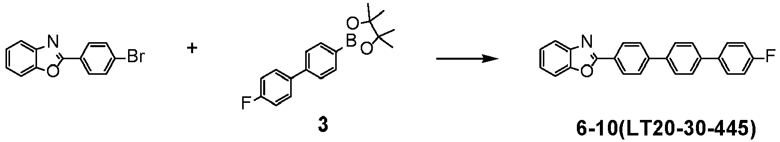

4.0 g (14.6 mol) of 2-(4-bromophenyl)benzo[d]oxazole, 4.9 g (14.6 mmol) of Intermediate **3**, 0.8 g (0.7 mmol) of Pd(PPh₃)₄, 4.1 g (29.2 mmol) of K₂CO₃, 48 mL of toluene, 20 mL of purified water, and 16 mL of ethanol were mixed in a 250 mL two-necked flask. Then, the reaction was allowed to proceed at 90 °C for 5 h. After completion of the reaction, the reaction mixture was cooled to room temperature. Thereafter, the resulting solid was collected by filtration, and dissolved in chloroform. The solution was filtered through silica gel and solidified with chloroform/2-propanol to give 2.9 g (yield: 54.7%) of Compound **6-10 (LT20-30-445)** as a white solid.

### Synthesis Example 3: Synthesis of Compound 6-13 (LT20-30-439)

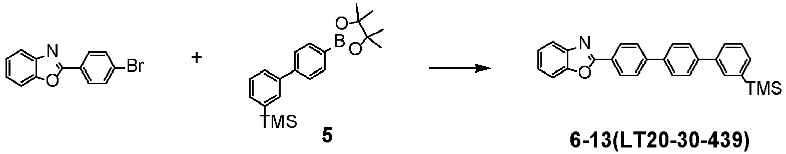

3.5 g (12.8 mol) of 2-(4-bromophenyl)benzo[d]oxazole, 4.9 g (12.8 mmol) of Intermediate **5,** 0.7 g (0.6 mmol) of Pd(PPh₃)₄, 4.4 g (25.5 mmol) of K₂CO₃, 42 mL of toluene, 17 mL of purified water, and 14 mL of ethanol were mixed in a 250 mL two-necked flask. Then, the reaction was allowed to proceed at 90 °C for 5 h. After completion of the reaction, the reaction mixture was cooled to room temperature. Thereafter, the resulting solid was collected by filtration, and dissolved in chloroform. The solution was filtered through silica gel and solidified with chloroform/methanol to give 3.2 g (yield: 60.6%) of Compound **6-13 (LT20-30-439)** as a white solid.

### Synthesis Example 4: Synthesis of Compound 6-16 (LT20-30-360)

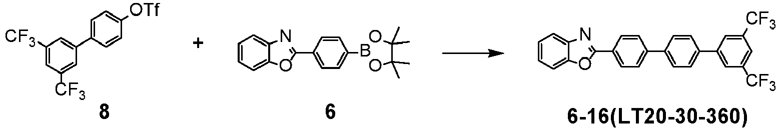

2.0 g (6.2 mmol) of Intermediate **6,** 4.5 g (9.3 mmol) of Intermediate **8**, 0.3 g (0.3 mmol) of Pd(PPh₃)₄, 6.2 mL (12.4 mmol) of 2 M K₂CO₃, 31 mL of toluene, and 15 mL of ethanol were mixed in a 250 mL one-necked flask. The mixture was stirred under reflux for 40 min. After completion of the reaction, the reaction mixture was cooled to room temperature and dichloromethane and distilled water were added thereto. The organic layer was separated, dried over anhydrous magnesium sulfate, and filtered through Celite. The filtrate was concentrated under reduced pressure, washed with dichloromethane, and filtered through silica (only DCM) to give 1.1 g (yield: 36.7%) of Compound **6-16 (LT20-30-360)** as a white solid.

### Synthesis Example 5: Synthesis of Compound 6-24 (LT20-30-454)

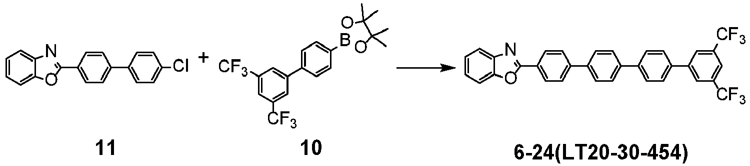

3.5 g (11.5 mmol) of Intermediate **11,** 6.2 g (14.9 mmol) of Intermediate **10,** 1.1 g (2.29 mmol) of Pd₂(dba)₃, 1.1 g (1.1 mmol) of X-Phos, 73 g (34.3 mmol) of K₃PO₄, and 60 mL of xylene were mixed in a 250 mL one-necked flask. Then, the mixture was stirred under reflux for 12 h. After completion of the reaction, the reaction mixture was cooled to room temperature. The resulting solid was collected by filtration, washed with water, methanol, and ethyl acetate, dried, and dissolved by boiling in monochlorobenzene. Thereafter, the solution was passed through a pad of celite and concentrated under reduced pressure. The concentrate was added with and boiled in monochlorobenzene for complete dissolution. Thereafter, the solution was solidified at 100 °C to give 2.6 g (yield: 41.1%) of Compound **6-24 (LT20-30-454)** as a white solid.

### Synthesis Example 6: Synthesis of Compound 6-56 (LT20-30-358)

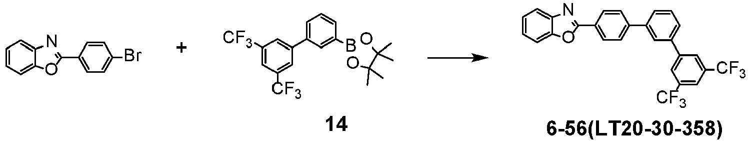

2.8 g (10.2 mmol) of 2-(4-bromophenyl)benzoxazole, 6.4 g (15.3 mmol) of Intermediate **14,** 0.6 g (0.5 mmol) of Pd(PPh₃)₄, 15 mL (30.6 mmol) of 2 M K₂CO₃, 50 mL of toluene, and 25 mL of ethanol were stirred under reflux in a 250 mL one-necked flask all day. The reaction mixture was cooled to room temperature and extracted with ethyl acetate. Water and the solvent were removed. The residue was dissolved in dichloromethane, purified by silica gel column chromatography (DCM:HEX), and filtered with a mixed solution (dichloromethane/ethanol) to give 3.1 g (yield: 63.0%) of Compound **6-56 (LT20-30-358)** as a white solid.

### Synthesis Example 7: Synthesis of Compound 6-61 (LT20-30-451)

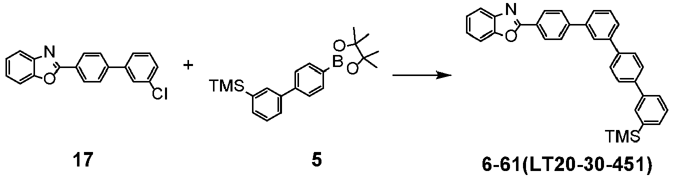

3.5 g (11.4 mol) of Intermediate **17,** 4.0 g (11.4 mmol) of Intermediate **5,** 0.7 g (1.1 mmol) of Pd(dba)₂, 1.0 g (2.3 mmol) of S-Phos, 7.3 g (34.3 mmol) of K₃PO₄, and 50 mL of xylene were mixed in a 250 mL two-necked flask. Then, the reaction was allowed to proceed at 125 °C for 3 h. After completion of the reaction, the reaction mixture was cooled to room temperature. Thereafter, the resulting solid was collected by filtration and dissolved in chloroform. The solution was filtered through silica gel and solidified with chloroform/2-propanol to give 3.6 g (yield: 63.8%) of Compound **6-61 (LT20-30- 451)** as a white solid.

### Synthesis Example 8: Synthesis of Compound 6-64 (LT20-30-457)

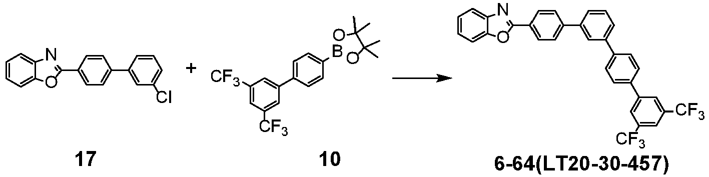

3.5 g (11.5 mmol) of Intermediate **17,** 6.2 g (14.9 mmol) of Intermediate **10,** 1.1 g (2.29 mmol) of Pd₂(dba)₃, 1.1 g (1.1 mmol) of X-Phos, 7.3 g (34.3 mmol) of K₃PO₄, and 60 mL of xylene were mixed in a 250 mL one-necked flask. The mixture was stirred under reflux for 12

h. After completion of the reaction, the reaction mixture was added with water, extracted with chloroform, dried, and concentrated under reduced pressure. The concentrate was purified by silica gel column chromatography (Hex:CHCl₃) and solidified with a mixed solvent (DCM/EA/MeOH) to give 2.8 g (yield: 43.7%) of Compound **6-64 (LT20-30-457)** as a white solid.

### Synthesis Example 9: Synthesis of Compound 6-74 (LT20-30-052)

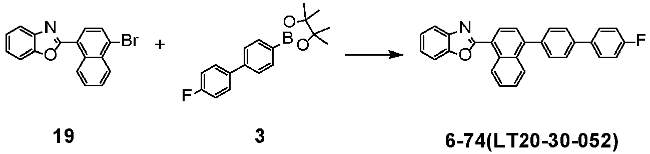

3.3 g (10.2 mmol) of Intermediate **19,** 3.0 g (10.2 mmol) of Intermediate **3,** 588.0 mg (509.0 µmol) of Pd(PPh₃)₄, 3.5 g (25.5 mmol) of K₂CO₃, 30 mL of toluene, 10 mL of ethanol, and 10 mL of distilled water were mixed in a 250 mL one-necked flask. Then, the mixture was stirred under reflux for 12 h. After completion of the reaction, the reaction mixture was cooled to room temperature. The resulting solid was collected by filtration, washed with water and methanol, dried, and dissolved in chloroform. The solution was filtered through a silica pad (CHCl₃) and solidified with a mixed solution (DCM/Acetone) to give 1.7 g (yield: 40.9%) of Compound **6-74 (LT20-30-052)** as a white solid.

### Synthesis Example 10: Synthesis of Compound 6-78 (LT20-30-046)

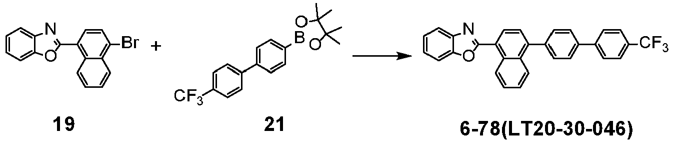

3.0 g (9.3 mmol) of Intermediate **19,** 3.2 g (9.3 mmol) of Intermediate **21,** 534.0 mg (462.7 µmol) of Pd(PPh₃)₄, 3.2g (23.1 mmol) of K₂CO₃, 30 mL of toluene, 10 mL of ethanol, and 10 mL of distilled water were mixed in a 250 mL one-necked flask. The mixture was stirred under reflux for 12 h. After completion of the reaction, the reaction mixture was cooled to room temperature. The resulting solid was collected by filtration, washed with water and methanol, dried, and dissolved in chloroform. The solution was filtered through a silica pad (CHCl₃) and solidified with a mixed solution (DCM/Acetone) to give 2.7 g (yield: 62.2%) of Compound **6-78 (LT20-30-046)** as a white solid.

### Synthesis Example 11: Synthesis of compound 6-80 (LT20-30-072)

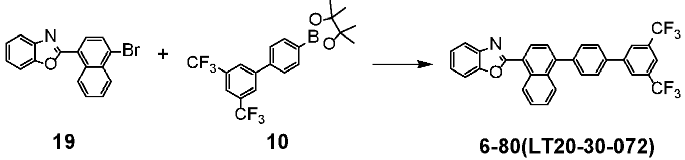

8.9 g (21.6 mmol) of Intermediate **19,** 3.5 g (10.8 mmol) of Intermediate **10,** 374.3 mg (323.9 µmol) of Pd(PPh₃)₄, 3.7 g (27.0 mmol) of K₂CO₃, 80 mL of toluene, 40 mL of ethanol, and 40 mL of distilled water were mixed in a 250 mL one-necked flask. The mixture was stirred under reflux for 19 h. After completion of the reaction, the reaction mixture was cooled to room temperature, washed with CHCl₃ and water, purified by silica gel column chromatography (Hex:CHCl₃), and solidified with methanol to give 2.0 g (yield: 76.5%) of Compound **6-80 (LT20-30-072)** as a white solid.

### Synthesis Example 12: Synthesis of Compound 6-82 (LT20-30-142)

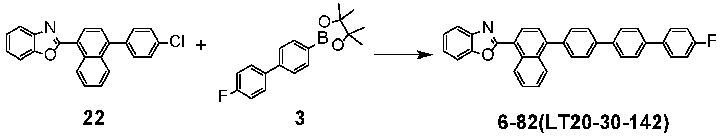

20.0 g (61.7 mmol) of Intermediate **22,** 8.5 g (61.7 mmol) of Intermediate **3**, 3.6 g (3.1 mmol) of Pd(PPh₃)₄, 93.0 mL (185.1 mmol) of 2 M K₂CO₃, 200 mL of toluene, and 90 mL of ethanol were mixed together. Then, the mixture was stirred under reflux for 12 h. After completion of the reaction, the reaction mixture was cooled to room temperature, added with water, and extracted with chloroform. Thereafter, the solvent was removed under reduced pressure. The residue was purified by silica gel column chromatography (CHCl₃) and solidified with a mixed solution (DCM/MeOH) to give 19.3 g (yield: 92.7%) of Compound **6-82 (LT20-30-142)** as a white solid.

### Synthesis Example 13: Synthesis of Compound 6-86 (LT20-30-053)

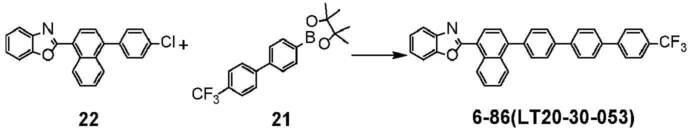

3.0 g (8.4 mmol) of Intermediate **22,** 3.5 g (10.1 mmol) of Intermediate **21,** 485.0 mg (509.0 µmol) of Pd(dba)₂, 804.0 mg (1.7 mmol) of X-Phos, 5.4 g (25.3 mmol) of K₃PO₄, and 45 mL of xylene were mixed in a 250 mL one-necked flask. Then, the mixture was stirred under reflux for 12 h. After completion of the reaction, the reaction mixture was cooled to room temperature. The resulting solid was collected by filtration, washed with water and methanol, dried, and dissolved in chloroform. Thereafter, the solution was filtered through a silica pad (CHCl₃) and solidified with chloroform to give 2.6 g (yield: 56.7%) of Compound **6-86 (LT20-30-053)** as a yellow solid.

### Synthesis Example 14: Synthesis of Compound 6-88 (LT20-30-129)

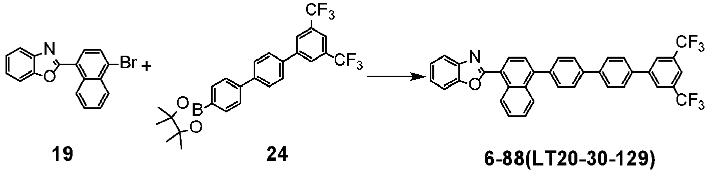

4.0 g (8.1 mmol) of Intermediate **24,** 2.6 g (8.1 mmol) of Intermediate **19,** 281.0 mg (243.0 µmol) of Pd(PPh₃)₄, 2.8 g (20.3 mmol) of K₂CO₃, 80 mL of toluene, 40 mL of ethanol, and 40 mL of distilled water were mixed in a 250 mL one-necked flask. The mixture was stirred under reflux for 19 h. After completion of the reaction, the reaction mixture was cooled to room temperature, washed with CHCl₃ and water, dried, and dissolved in chloroform. Thereafter, the solution was filtered through a silica pad (CHCl₃) and solidified with methanol to give 2.5 g (yield: 51.3%) of Compound **6-88 (LT20-30-129)** as a yellow solid.

### Synthesis Example 15: Synthesis of Compound 6-117 (LT20-30-473)

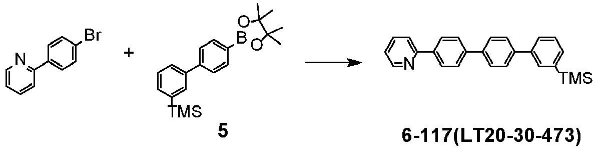

3.0 g (12.8 mmol) of 2-(4-bromophenyl)pyridine, 5.4 g (15.4 mmol) of Intermediate **5,** 444.3 mg (384.5 µmol) of Pd(PPh₃)₄, 5.3 g (38.4 mmol) of K₂CO₃, 64 mL of toluene, 16 mL of ethanol, and 16 mL of distilled water were mixed in a 250 mL one-necked flask. Then, the mixture was stirred at 90 °C for one day. After completion of the reaction, the reaction mixture was cooled to room temperature. The resulting solid was collected by filtration, washed with water, methanol, and hexane, dried, and dissolved in hot toluene. The solution was filtered through a pad of silica gel and solidified with a mixed solution (Hex:EA) to give 1.9 g (yield: 40.0%) of Compound **6-117 (LT20-30-473)** as a white solid.

### Synthesis Example 16: Synthesis of Compound 6-120 (LT20-30-484)

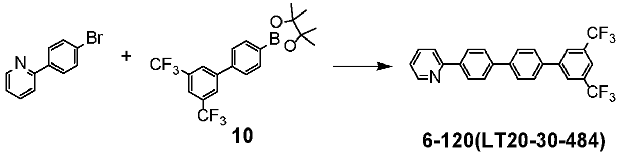

3.0 g (12.8 mmol) of 2-(4-bromophenyl)pyridine, 6.4 g (15.4 mmol) of Intermediate **10,** 444.3 mg (384.5 µmol) of Pd(PPh₃)₄, 5.3 g (38.4 mmol) of K₂CO₃, 64 mL of toluene, 16 mL of ethanol, and 16 mL of distilled water were mixed in a 250 mL one-necked flask. Then, the mixture was stirred at 90 °C for one day. After completion of the reaction, the reaction mixture was cooled to room temperature. The resulting solid was collected by filtration, washed with water, methanol, and hexane, dried, and dissolved in hot toluene. The solution was filtered through a pad of silica gel and solidified with a mixed solution (Hex:EA) to give 2.4 g (yield: 43.1%) of Compound **6-120 (LT20-30-484)** as a white solid.

### Synthesis Example 17: Synthesis of compound 6-141 (LT20-30-435)

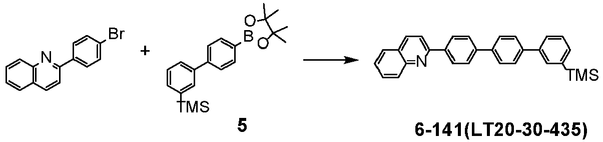

3.5 g (12.3 mmol) of 2-(4-bromophenyl)quinoline, 5.2 g (14.8 mmol) of Intermediate **5,** 712 mg (615.9 µmol) of Pd(PPh₃)₄, 5.1 g (37.0 mmol) of K₂CO₃, 40 mL of toluene, 10 mL of ethanol, and 10 mL of distilled water were mixed in a 250 mL one-necked flask. Then, the mixture was stirred under reflux for 12 h. After completion of the reaction, the reaction mixture was cooled to room temperature. The resulting solid was collected by filtration, washed with distilled water and ethanol, dried, and dissolved by boiling in monochlorobenzene. Thereafter, the solution was passed through a pad of celite and concentrated under reduced pressure. The concentrate was added with and boiled in monochlorobenzene for complete dissolution. Thereafter, the solution was solidified at room temperature to give 2.6 g (yield: 49.7%) of Compound **6-141 (LT20-30-435)** as a white solid.

### Synthesis Example 18: Synthesis of Compound 6-149 (LT20-30-438)

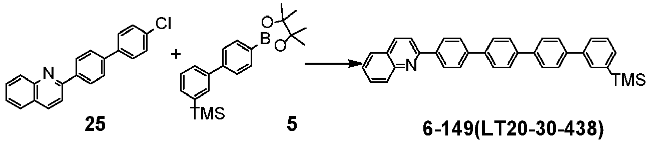

3.5 g (11.1 mmol) of Intermediate **25,** 5.1 g (14.4 mmol) of Intermediate **5,** 1.0 g (14.4 mmol) of Pd₂(dba)₃, 1.1 g (2.2 mmol) of X-Phos, 7.1 g (33.3 mmol) of K₃PO₄, and 60 mL of xylene were mixed together. Then, the mixture was stirred under reflux for one day. After completion of the reaction, the reaction mixture was cooled to room temperature. The resulting solid was collected by filtration, washed with water, ethanol, and ethyl acetate, dried, and dissolved by boiling in monochlorobenzene. Thereafter, the solution was passed through a pad of celite and concentrated under reduced pressure. The concentrate was added with and boiled in monochlorobenzene for dissolution. The solution was stirred at 100 °C and solidified to give 2.1 g (yield: 36.9%) of Compound **6-149 (LT20-30-438)** as a white solid.

### Synthesis Example 19: Synthesis of compound 6-152 (LT20-30-434)

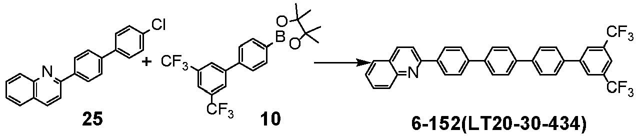

3.5 g (11.1 mmol) of Intermediate **25**, 6.0 g (14.4 mmol) of Intermediate **10,** 1.0 g (14.4 mmol) of Pd₂(dba)₃, 1.1 g (2.2 mmol) of X-Phos, 7.1 g (33.3 mmol) of K₃PO₄, and 60 mL of xylene were mixed together. Then, the mixture was stirred under reflux for one day. After completion of the reaction, the reaction mixture was cooled to room temperature. The resulting solid was collected by filtration, washed with water, ethanol, and ethyl acetate, dried, and dissolved by boiling in monochlorobenzene. Thereafter, the solution was passed through a pad of celite and concentrated under reduced pressure. The concentrate was added with and boiled in monochlorobenzene for dissolution. The solution was stirred at 100 °C and solidified to give 2.9 g (yield: 45.9%) of Compound **6-152 (LT20-30-434)** as a white solid.

### Synthesis Example 20: Synthesis of Compound 6-192 (LT20-30-466)

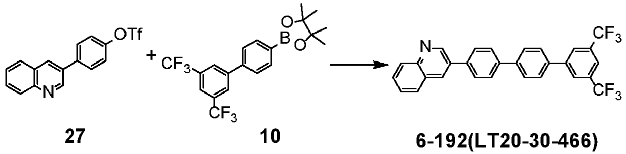

3.5 g (9.9 mmol) of Intermediate **27,** 5.4 g (12.9 mmol) of Intermediate **10,** 572.0 mg (615.9 µmol) of Pd(PPh₃)₄, 13.0 mL (24.8 mmol) of 2 M K₂CO₃, 30 mL of toluene, and 10 mL of ethanol were mixed in a 250 mL one-necked flask. Then, the mixture was stirred under reflux for 12 h. After completion of the reaction, the reaction mixture was cooled to room temperature. The resulting solid was collected by filtration, washed with water and methanol, dried, purified by column chromatography (CHCl₃), and solidified with a mixed solvent (DCM/MeOH) to give 2.9 g (yield: 58.9%) of Compound **6-192 (LT20-30-466)** as a white solid.

### Synthesis Example 21: Synthesis of Compound 6-212 (LT20-30-478)

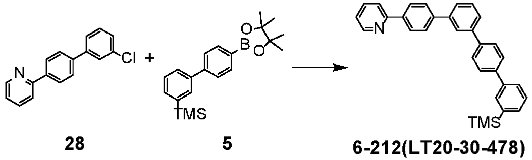

4.0 g (15.10 mmol) of Intermediate **28**, 6.3 g (18.1 mmol) of Intermediate **5,** 689.2 mg (752.6 µmol) of Pd₂(dba)₃, 1.4 g (3.0 mmol) of X-Phos, 9.6 g (45.2 mmol) of K₃PO₄, and 75 mL of xylene were mixed in a 250 mL two-necked flask. Then, the reaction was allowed to proceed at 130 °C for one day. After completion of the reaction, the reaction mixture was cooled to room temperature, filtered with distilled water, washed with distilled water, methanol, and ethyl acetate, and dissolved in hot toluene. The solution was filtered through a pad of celite and solidified with ethyl acetate to give 4.3 g (yield: 62.7%) of Compound **6-212 (LT20-30-478)** as a white solid.

### Synthesis Example 22: Synthesis of Compound 6-227 (LT20-30-450)

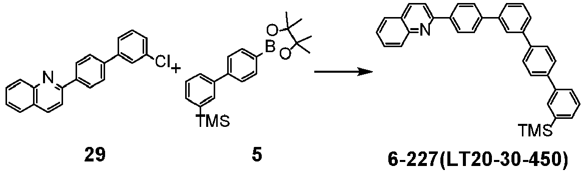

3.5 g (11.1 mmol) of Intermediate **29,** 5.1 g (14.4 mmol) of Intermediate **5,** 1.0 g (14.4 mmol) of Pd₂(dba)₃, 1.1 g (2.2 mmol) of X-Phos, 7.1 g (33.3 mmol) of K₃PO₄, and 60 mL of xylene were mixed together. Then, the mixture was stirred under reflux for one day. After completion of the reaction, the reaction mixture was cooled to room temperature. The resulting solid was collected by filtration, washed with water, ethanol, and ethyl acetate, dried, and dissolved by boiling in monochlorobenzene. Thereafter, the solution was passed through a pad of celite and concentrated under reduced pressure. The concentrate was added with and boiled in monochlorobenzene for dissolution. The solution was stirred at 40 °C and solidified to give 2.1 g (yield: 37.3%) of Compound **6-227 (LT20-30-450)** as a white solid.

### Synthesis Example 23: Synthesis of Compound 6-230 (LT20-30-443)

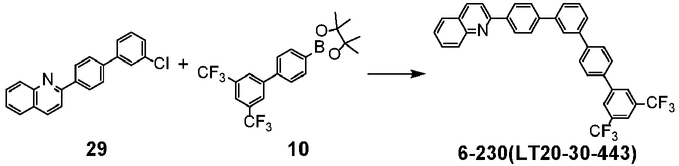

3.5 g (11.1 mmol) of Intermediate **29**, 6.0 g (14.4 mmol) of Intermediate **10,** 1.0 g (14.4 mmol) of Pd₂(dba)₃, 1.1 g (2.2 mmol) of X-Phos, 7.1 g (33.3 mmol) of K₃PO₄, and 60 mL of xylene were mixed together. Then, the mixture was stirred under reflux for one day. After completion of the reaction, the reaction mixture was cooled to room temperature, added with water, extracted with chloroform, and concentrated. The concentrate was purified by column chromatography (Hex:CHCl₃) and solidified with a mixed solvent (DCM/EA) to give 1.7 g (yield: 27.3%) of Compound **6-230 (LT20-30-443)** as a white solid.

### Synthesis Example 24: Synthesis of Compound 6-243 (LT20-30-486)

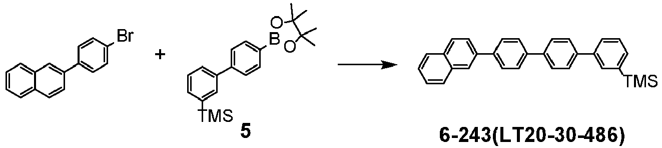

4.2 g (14.9 mmol) of 2-(4-bromophenyl)naphthalene, 5.0 g (14.2 mmol) of Intermediate 5, 0.8 g (0.7 mmol) of Pd(PPh₃)₄, 3.9 g (28.4 mmol) of K₂CO₃, 60 mL of toluene, 25 mL of purified water, and 20 mL of ethanol were mixed in a 250 mL two-necked flask. Then, the reaction was allowed to proceed at 90 °C for 3 h. After completion of the reaction, the reaction mixture was cooled to room temperature. Thereafter, the resulting solid was collected by filtration and dissolved in chloroform. The solution was filtered through silica gel and solidified with methanol to give 2.8 g (yield: 46.4%) of Compound **6-243 (LT20-30-486)** as a white solid.

### Synthesis Example 25: Synthesis of Compound 6-246 (LT20-30-514)

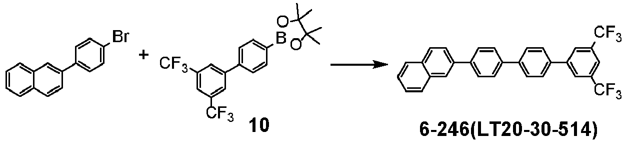

3.0 g (10.6 mmol) of 2-(4-bromophenyl)naphthalene, 5.3 g (12.7 mmol) of Intermediate **10,** 367.3 mg (317.8 µmol) of Pd(PPh₃)₄, 4.4 g (31.8 mmol) of K₂CO₃, 55 mL of toluene, 14 mL of ethanol, and 14 mL of distilled water were mixed in a 250 mL one-necked flask. The mixture was stirred at 90 °C for one day. After completion of the reaction, the reaction mixture was cooled to room temperature. The resulting solid was collected by filtration, washed with water, methanol, and hexane, dried, and dissolved in hot toluene. The solution was filtered through a pad of silica gel and solidified with hexane and monochlorobenzene to give 4.0 g (yield: 76.7%) of Compound **6-246 (LT20-30-514)** as a white solid.

### Synthesis Example 26: Synthesis of Compound 6-268 (LT20-30-442)

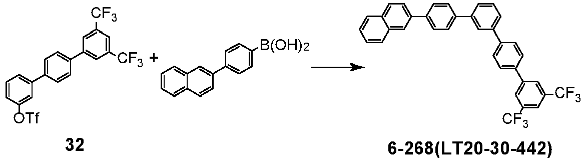

4.0 g (7.8 mmol) of Intermediate **32**, 2.3 g (9.3 mmol) of 4-(2-naphthyl)phenylboronic acid, 0.5 g (0.4 mmol) of Pd(PPh₃)₄, 12 mL (23.3 mmol) of 2 M K₂CO₃, 30 mL of toluene, and 15 mL of ethanol were stirred under reflux in a 250 mL one-necked flask all day. The reaction mixture was cooled to room temperature and extracted with chloroform. Water and the solvent were removed. The residue was dissolved in chloroform, purified by silica gel column chromatography (CHCl₃:HEX), solidified with a mixed solution (chloroform/2-propanol), and filtered to give 3.5 g (yield: 78.2%) of Compound **6-268 (LT20-30-442)** as a white solid.

### Synthesis Example 27: Synthesis of Compound 6-284 (LT20-35-003)

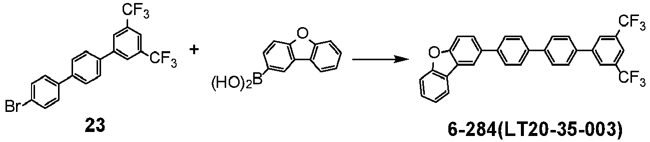

3.0 g (6.7 mmol) of Intermediate **23,** 1.7 g (8.1 mmol) of dibenzo[b,d]furan-2-ylboronic acid, 0.4 g (0.3 mmol) of Pd(PPh₃)₄, 6.7 mL (13.5 mmol) of 2 M K₂CO₃, 20 mL of toluene, and 10 mL of ethanol were stirred under reflux in a 100 mL one-necked flask all day. The mixture was cooled to room temperature and extracted with chloroform. Water and the solvent were removed. The residue was dissolved in chloroform, purified by silica gel column chromatography (CHCl₃:HEX), solidified with a mixed solution (chloroform/acetone), and filtered to give 2.1 g (yield: 58.5%) of Compound **6-284 (LT20-35-003)** as a white solid.

### Synthesis Example 28: Synthesis of Compound 6-340 (LT20-35-005)

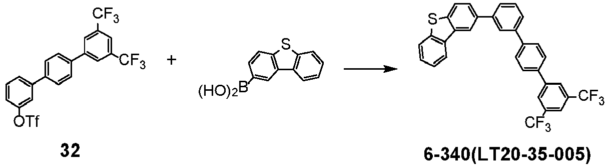

3.0 g (5.8 mmol) of Intermediate **32,** 1.6 g (7.0 mmol) of dibenzo[b,d]thiophen-2-ylboronic acid, 337.0 mg (291.6 µmol) of Pd(PPh₃)₄, 5.8 mL (24.8 mmol) of 2 M K₂CO₃, 30 mL of toluene, and 10 mL of ethanol were mixed in a 250 mL one-necked flask. Then, the mixture was stirred under reflux for 12 h. After completion of the reaction, the reaction mixture was cooled to room temperature. The resulting solid was collected by filtration, washed with water and methanol, dried, purified by column chromatography (CHCl₃), and solidified with a mixed solvent (DCM/MeOH) to give 1.3 g (yield: 40.6%) of Compound **6-340 (LT20-35-005)** as a white solid.

### Synthesis Example 29: Synthesis of compound 6-364 (LT20-30-461)

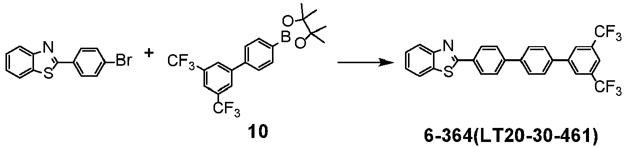

3.0 g (10.3 mmol) of 2-(4-bromophenyl)benzo[d]thiazole, 5.6 g (13.4 mmol) of Intermediate **10,** 597.0 mg (519.9 µmol) of Pd(PPh₃)₄, 4.3 g (31.0 mmol) of K₂CO₃, 30 mL of toluene, 10 mL of ethanol, and 10 mL of distilled water were mixed in a 250 mL one-necked flask. Then, the mixture was stirred under reflux for 12 h. After completion of the reaction, the reaction mixture was cooled to room temperature. The resulting solid was collected by filtration, washed with water and ethanol, dried, and dissolved by boiling in monochlorobenzene. Thereafter, the solution was passed through a pad of celite and concentrated under reduced pressure. The concentrate was added with and boiled in monochlorobenzene for complete dissolution. Thereafter, the solution was solidified at 100 °C to give 3.6 g (yield: 69.9%) of Compound **6-364 (LT20-30-461)** as a white solid.

### Synthesis Example 30: Synthesis of Compound 6-461 (LT18-30-479)

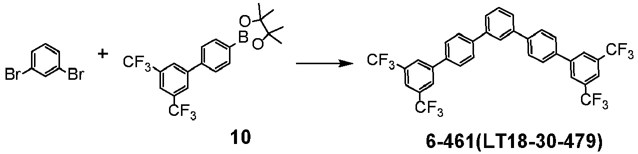

1.8 g (7.63 mmol) of 1,3-dibromobenzene, 8.6 g (20.6 mmol) of Intermediate **10,** 0.9 g (0.8 mmol) of Pd(PPh₃)₄, 38 mL of toluene, 19 mL of ethanol, and 19 mL of 2 M K₂CO₃ (38.2 mmol) were stirred in a 250 mL one-necked flask at 80 °C all day. After the reaction was confirmed, the reaction mixture was extracted with EA. After removal of water and the solvent, the residue was purified by silica gel column chromatography (MC:HEX). The resulting solid was boiled in dichloromethane for complete dissolution. Then, the solution was cooled at room temperature. The resulting solid was collected by filtration and washed thoroughly with methanol to give 2.5 g (yield: 49.8%) of Compound **6-461 (LT18-30-479)** as a white solid.

### Synthesis Example 31: Synthesis of Compound 6-463 (LT20-35-987)

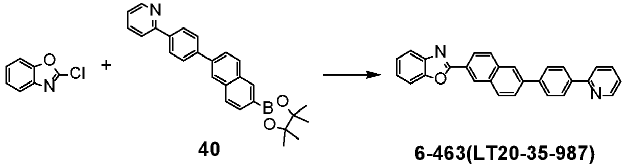

5.0 g (12.3 mmol) of Intermediate **40,** 2.8 g (18.4 mmol) of 2-chlorobenzooxazole, 0.7 g (0.6 mmol) of Pd(PPh₃)₄, 3.4 g (24.6 mmol) of K₂CO₃, 80 mL of toluene, 40 mL of ethanol, and 40 mL of distilled water were placed in a 250 mL one-necked flask. The mixture was heated to reflux with stirring all day. After completion of the reaction, the reaction mixture was cooled to room temperature and the solvent was removed. The residue was added with distilled water and extracted with DCM. The organic layer was separated, dried over anhydrous MgSO₄, and purified by column chromatography (CHCl₃:Hex) to give 2.4 g (yield: 49.1%) of Compound **6-463 (LT20-35-987)** as a white solid.

### Synthesis Example 32: Synthesis of Compound 6-471 (LT19-30-167)

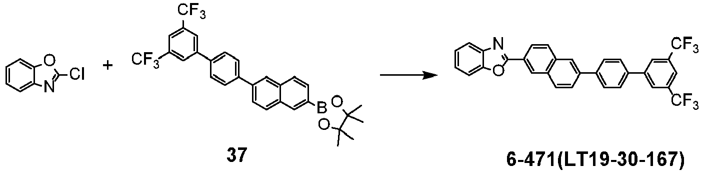

5.6 g (10.3 mmol) of Intermediate **37**, 2.4 g (15.5 mmol) of 2-chlorobenzooxazole, 0.4 g (0.3 mmol) of Pd(PPh₃)₄, 2.1 g (15.5 mmol) of K₂CO₃, 80 mL of toluene, 40 mL of ethanol, and 40 mL of distilled water were placed in a 250 ml one-necked flask. The mixture was heated to reflux with stirring all day. After completion of the reaction, the reaction mixture was cooled to room temperature and the solvent was removed. The residue was added with distilled water and extracted with DCM. The organic layer was separated, dried over anhydrous MgSO₄, and purified by column chromatography (CHCl₃:Hex) to give 3.0 g (yield: 54.5%) of Compound **6-471 (LT19-30-167)** as a white solid.

### Synthesis Example 33: Synthesis of Compound 6-491 (LT20-35-985)

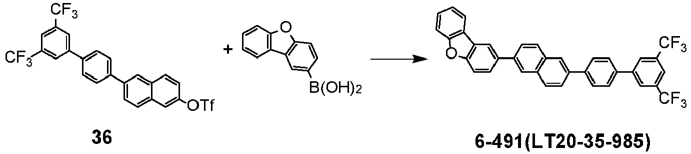

5.0 g (8.9 mmol) of 4-dibenzofuran boronic acid, 1.9 g (8.9 mmol) of Intermediate **36,** 0.5 g (0.4 mmol) of Pd(PPh₃)₄, 2.5 g (17.7 mmol) of K₂CO₃, 120 mL of toluene, 60 mL of purified water, and 60 mL of ethanol were mixed together. Then, the reaction was allowed to proceed at 110 °C for 5 h. After completion of the reaction, the reaction mixture was cooled to room temperature, added with purified water, and extracted with ethyl acetate. The solvent was removed under reduced pressure. The residue was dissolved in chloroform, filtered through silica gel, and concentrated under reduced pressure to remove the solvent. The concentrate was solidified with methanol/hexane to give 2.6 g (yield: 50.4%) of Compound **6-491 (LT20-35-985)** as a white solid.

### Synthesis Example 34: Synthesis of Compound 6-505 (LT20-35-988)

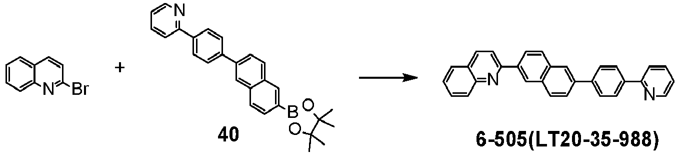

2.6 g (12.3 mmol) of 2-bromoquinoline, 5.0 g (12.3 mmol) of Intermediate **40,** 0.7 g (0.6 mmol) of Pd(PPh₃)₄, 3.4 g (24.6 mmol) of potassium carbonate, 150 mL of toluene, 30 mL of ethanol, and 30 mL of water were mixed together. Then, the mixture was stirred under reflux for 12 h. After completion of the reaction, the reaction mixture was cooled to room temperature, added with water, and extracted with ethyl acetate. The solvent was removed under reduced pressure. The residue was purified by silica gel column chromatography (CHCl₃) and solidified with a mixed solution (DCM/Hex) to give 2.0 g (yield: 40.0%) of Compound **6-505 (LT20-35-988)** as a white solid.

### Synthesis Example 35: Synthesis of Compound 6-513 (LT20-35-984)

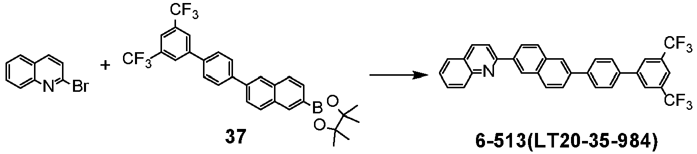

1.9 g (12.3 mmol) of 2-bromoquinoline, 5.0 g (12.3 mmol) of Intermediate **37,** 0.5 g (0.5 mmol) of Pd(PPh₃)₄, 2.6 g (18.4 mmol) of potassium carbonate, 150 mL of toluene, 30 mL of ethanol, and 30 mL of water were mixed together. Then, the mixture was stirred under reflux for 12 h. After completion of the reaction, the reaction mixture was cooled to room temperature, added with water, and extracted with ethyl acetate. The solvent was removed under reduced pressure. The residue was purified by silica gel column chromatography (CHCl₃) and solidified with a mixed solution (DCM/Hex) to give 2.1 g (yield: 41.9%) of Compound **6-513 (LT20-35-984)** as a white solid.

### <TEST EXAMPLES>

The refractive indices (n) and extinction coefficients (k) of the inventive compounds were measured using an ellipsometer (J.A. WOOLLAM).

### Preparation of monolayer films for test examples:

A glass substrate (0.7T) was washed with ethanol, DI water, and acetone (each for 10 min) and each of the inventive compounds was deposited to a thickness of 800 Å on the glass substrate to form a monolayer film, which was used to measure the optical properties of the compound.

### Preparation of monolayer films for comparative test examples (Glass/REF01 (80 nm)):

REF01 (80 nm) was deposited on glass to fabricate a device for evaluating its optical properties. Before deposition of the compound, the glass was treated with a 125 W oxygen plasma at 2 × 10⁻² Torr for 2 min. The compound was deposited at a rate of 1 Å/sec and a degree of vacuum of 9 × 10⁻⁷ Torr to prepare a monolayer film.

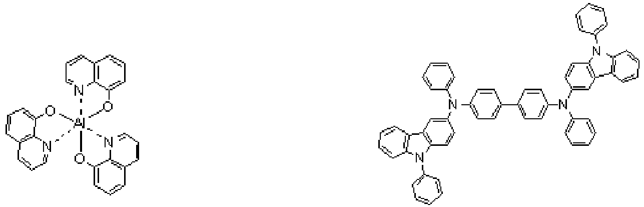

### Comparative Test Example 1 (Alq3) Comparative Test Example 2 (REF01)

### <Test Examples 1-35>

Monolayer films were prepared in the same manner as in Comparative Test Example 2, except that the corresponding compounds shown in Table 1 were used instead of REF01.

The optical properties of the compounds used in Comparative Test Examples 1-2 and Test Examples 1-35 are shown in Table 1.

The optical properties were refractive indices at wavelengths of 460 nm and 620 nm.

**Table 1**

| Test Example No. | Compound | n (460 nm) | n (620 nm) |
|---|---|---|---|
| Comparative Test Example 1 | Alq₃ | 1.808 | 1.690 |
| Comparative Test Example 2 | REF01 | 1.986 | 1.846 |
| Test Example 1 | 6-9 (LT20-30-477) | 1.512 | 1.501 |
| Test Example 2 | 6-10 (LT20-30-445) | 1.469 | 1.458 |
| Test Example 3 | 6-13 (LT20-30-439) | 1.436 | 1.429 |
| Test Example 4 | 6-16 (LT20-30-360) | 1.398 | 1.396 |
| Test Example 5 | 6-24 (LT20-30-454) | 1.587 | 1.545 |
| Test Example 6 | 6-56 (LT20-30-358) | 1.708 | 1.643 |
| Test Example 7 | 6-61 (LT20-30-451) | 1.568 | 1.544 |
| Test Example 8 | 6-64 (LT20-30-457) | 1.469 | 1.447 |
| Test Example 9 | 6-74 (LT20-30-052) | 1.440 | 1.419 |
| Test Example 10 | 6-78 (LT20-30-046) | 1.441 | 1.423 |
| Test Example 11 | 6-80 (LT20-30-072) | 1.497 | 1.468 |
| Test Example 12 | 6-82 (LT20-30-142) | 1.517 | 1.476 |
| Test Example 13 | 6-86 (LT20-30-053) | 1.587 | 1.545 |
| Test Example 14 | 6-88 (LT20-30-129) | 1.464 | 1.439 |
| Test Example 15 | 6-117 (LT20-30-473) | 1.486 | 1.452 |
| Test Example 16 | 6-120 (LT20-30-484) | 1.425 | 1.418 |
| Test Example 17 | 6-141 (LT20-30-435) | 1.538 | 1.513 |
| Test Example 18 | 6-149 (LT20-30-438) | 1.616 | 1.589 |
| Test Example 19 | 6-152 (LT20-30-434) | 1.448 | 1.439 |
| Test Example 20 | 6-192 (LT20-30-466) | 1.466 | 1.450 |
| Test Example 21 | 6-212 (LT20-30-478) | 1.464 | 1.443 |
| Test Example 22 | 6-227 (LT20-30-450) | 1.703 | 1.637 |
| Test Example 23 | 6-230 (LT20-30-443) | 1.512 | 1.479 |
| Test Example 24 | 6-243 (LT20-30-486) | 1.585 | 1.536 |
| Test Example 25 | 6-246 (LT20-30-514) | 1.442 | 1.429 |
| Test Example 26 | 6-268 (LT20-30-442) | 1.514 | 1.483 |
| Test Example 27 | 6-284 (LT20-35-003) | 1.568 | 1.544 |
| Test Example 28 | 6-340 (LT20-35-005) | 1.484 | 1.453 |
| Test Example 29 | 6-364 (LT20-30-461) | 1.475 | 1.450 |
| Test Example 30 | 6-461 (LT18-30-479) | 1.414 | 1.400 |
| Test Example 31 | 6-463 (LT20-35-987) | 1.538 | 1.513 |
| Test Example 32 | 6-471 (LT19-30-167) | 1.616 | 1.589 |
| Test Example 33 | 6-491 (LT20-35-985) | 1.585 | 1.536 |
| Test Example 34 | 6-505 (LT20-35-988) | 1.703 | 1.637 |
| Test Example 35 | 6-513 (LT20-35-984) | 1.616 | 1.589 |

As can be seen from Table 1, the n values of the compound (Alq₃) used in Comparative Test Example 1 in the blue (460 nm) and red regions (620 nm) were 1.808 and 1.690, respectively. In contrast, most of the inventive compounds were found to have lower refractive indices (n < 1.69 at 620 nm) than the compound (Alq3) used in Comparative Test Example 1 in the blue, green, and red regions. The refractive indices of the inventive compounds were low enough to secure wide viewing angles in the blue wavelength range.

### <EXAMPLES>

### Device fabrication

A transparent ITO electrode was used as an anode, 2-TNATA was used as a material for a hole injection layer, NPB was used as a material for a hole transporting layer, αβ-ADN and Pyene-CN were used as a host and a blue fluorescent dopant for an emission layer, respectively, Liq was used as a material for an electron injection layer, and Mg:Ag was used as a material for a cathode to fabricate a device. The structures of 2-TNATA, NPB, and αβ-ADN are as follow: 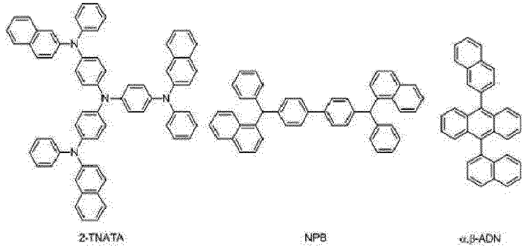
Comparative Example 1 (without capping layer): ITO/2-TNATA (60 nm)/NPB (20 nm)/αβ-ADN: 10% pyrene-CN (30 nm)/Alq₃ (30 nm)/Liq (2 nm)/Mg:Ag (1:9, 10 nm)
Comparative Example 2 (with capping layer consisting of one layer): ITO/2-TNATA (60 nm)/NPB (20 nm)/αβ-ADN: 10% pyrene-CN (30 nm)/Alq₃ (30 nm)/Liq (2 nm)/Mg:Ag (1:9, 10 nm)/Alq₃ (80 nm)
Examples 1-35 (with capping layer consisting of two layers): ITO/2-TNATA (60 nm)/NPB (20 nm)/αβ-ADN: 10% pyrene-CN (30 nm)/Alq₃ (30 nm)/Liq (2 nm)/Mg:Ag (1:9, 10 nm)/inventive compound (20 nm, low refractive index compound)/REF01 (60 nm, high refractive index compound)
ITO (180 nm), 2-TNATA (60 nm), NPB (20 nm), αβ-ADN:pyrene-CN 10% (30 nm), Alq₃ (30 nm), Liq (2 nm), Mg:Ag (1:9, 10 nm), and a capping layer were deposited in this order to fabricate a blue fluorescent organic electroluminescent device.

Before deposition of the organic materials, the ITO electrode was treated with a 125 W oxygen plasma at 2 × 10⁻² Torr for 2 min. The organic materials were deposited at a degree of vacuum of 9 × 10⁻⁷ Torr. Liq was deposited at a rate of 0.1 Å/sec, αβ-ADN and pyrene-CN were co-deposited at rates of 0.18 Å/sec and 0.02 Å/sec, respectively, and the other organic materials were all deposited at a rate of 1 Å/sec.

The device was sealed in a glove box filled with nitrogen gas to prevent contact with air and moisture. After a barrier was formed with an adhesive tape (3M), barium oxide as a moisture absorbent was placed to remove moisture and a glass plate was attached to the barrier.

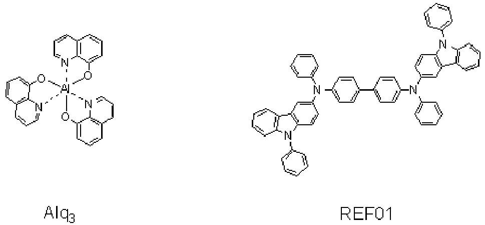

### <Examples 1-35>

Organic electroluminescent devices were fabricated in the same manner as described above, except that a capping layer having a multilayer structure consisting of a high refractive index layer (60 nm) and a low refractive index layer (20 nm) formed on the high refractive index layer was formed using REF01 for the high refractive index layer and the corresponding compound shown in Table 2 for the low refractive index layer.

The electroluminescent properties of the organic electroluminescent devices fabricated in Comparative Examples 1-2 and Examples 1-35 are shown in Table 2.

**Table 2**

| Example No. | Compound | Driving voltage (V) | Efficiency (cd/A) | Lifetime (%) |
|---|---|---|---|---|
| Comparative Example 1 | - | 4.51 | 4.22 | 89.21 |
| Comparative Example 2 | Alq₃ alone | 4.50 | 4.83 | 88.92 |
| Example 1 | 6-9 (LT20-30-477) | 4.51 | 5.31 | 95.61 |
| Example 2 | 6-10 (LT20-30-445) | 4.49 | 5.53 | 95.61 |
| Example 3 | 6-13 (LT20-30-439) | 4.40 | 5.81 | 97.32 |
| Example 4 | 6-16 (LT20-30-360) | 4.42 | 6.11 | 97.54 |
| Example 5 | 6-24 (LT20-30-454) | 4.49 | 5.43 | 95.55 |
| Example 6 | 6-56 (LT20-30-358) | 4.49 | 5.53 | 95.61 |
| Example 7 | 6-61 (LT20-30-451) | 4.40 | 6.00 | 98.11 |
| Example 8 | 6-64 (LT20-30-457) | 4.42 | 6.11 | 97.54 |
| Example 9 | 6-74 (LT20-30-052) | 4.45 | 6.55 | 97.45 |
| Example 10 | 6-78 (LT20-30-046) | 4.47 | 6.32 | 98.13 |
| Example 11 | 6-80 (LT20-30-072) | 4.42 | 6.11 | 97.54 |
| Example 12 | 6-82 (LT20-30-142) | 4.41 | 6.23 | 97.55 |
| Example 13 | 6-86 (LT20-30-053) | 4.49 | 5.53 | 95.61 |
| Example 14 | 6-88 (LT20-30-129) | 4.50 | 6.22 | 95.67 |
| Example 15 | 6-117 (LT20-30-473) | 4.52 | 6.21 | 95.52 |
| Example 16 | 6-120 (LT20-30-484) | 4.40 | 5.81 | 97.32 |
| Example 17 | 6-141 (LT20-30-435) | 4.41 | 6.23 | 97.55 |
| Example 18 | 6-149 (LT20-30-438) | 4.42 | 6.11 | 97.54 |
| Example 19 | 6-152 (LT20-30-434) | 4.47 | 6.32 | 98.13 |
| Example 20 | 6-192 (LT20-30-466) | 4.42 | 6.11 | 97.00 |
| Example 21 | 6-212 (LT20-30-478) | 4.45 | 6.13 | 97.45 |
| Example 22 | 6-227 (LT20-30-450) | 4.50 | 6.15 | 97.32 |
| Example 23 | 6-230 (LT20-30-443) | 4.41 | 6.20 | 97.75 |
| Example 24 | 6-243 (LT20-30-486) | 4.51 | 5.31 | 95.61 |
| Example 25 | 6-246 (LT20-30-514) | 4.45 | 6.13 | 96.80 |
| Example 26 | 6-268 (LT20-30-442) | 4.38 | 5.53 | 97.40 |
| Example 27 | 6-284 (LT20-35-003) | 4.51 | 5.31 | 95.61 |
| Example 28 | 6-340 (LT20-35-005) | 4.40 | 6.25 | 97.42 |
| Example 29 | 6-364 (LT20-30-461) | 4.41 | 6.23 | 97.55 |
| Example 30 | 6-461 (LT18-30-479) | 4.42 | 6.65 | 98.00 |
| Example 31 | 6-463 (LT20-35-987) | 4.50 | 6.15 | 97.32 |
| Example 32 | 6-471 (LT19-30-167) | 4.52 | 6.21 | 95.52 |
| Example 33 | 6-491 (LT20-35-985) | 4.49 | 5.53 | 95.61 |
| Example 34 | 6-505 (LT20-35-988) | 4.50 | 6.15 | 97.32 |
| Example 35 | 6-513 (LT20-35-984) | 4.52 | 6.21 | 95.52 |

The results in Table 2 reveal that the presence of the capping layer in the device of Comparative Example 2 leads to an increase in the efficiency of the device, unlike in the device without a capping layer (Comparative Example 1).

The results in Table 2 also demonstrate that the inventive organic compounds can be used as materials for low refractive index capping layers of organic electronic devices, including organic light emitting devices, and their use ensures excellent characteristics of the organic electronic devices in terms of efficiency, driving voltage, and stability.

The capping layer having a multilayer structure composed of the high refractive index compound (n > 1.69 at 620 nm) and the low refractive index compound (n < 1.69 at 620 nm) was found to increase the efficiency of the corresponding device, unlike the capping layer having a monolayer structure composed of the high refractive index compound (n > 1.69 at 620 nm). In addition, the use of the inventive compound as a material for the capping layer having a multilayer structure was found to improve the efficiency of the corresponding device compared to the use of Alq₃ as a material for the capping layer (Comparative Example 2).

These results can be explained by the refractive indices of the inventive compounds. It is obvious that the use of the capping layer having a multilayer structure composed of REF01 having a high refractive index and the inventive compound having a low refractive index ensures high efficiency of the corresponding organic electroluminescent device compared to the use of the capping layer having a monolayer structure composed of REF01.

Therefore, it can be concluded that the compound of Formula 1 has unexpectedly desirable characteristics for use as a material for a low refractive index capping layer of an OLED.

Based on its characteristics, the compound of the present invention can be used in organic electronic devices for industrial applications.

The above synthesis examples are merely illustrative and the reaction conditions may vary as required. The compounds according to exemplary embodiments of the present invention may be substituted with various substituents using suitable methods and materials known in the art. The introduction of various substituents to the core structure of the compound represented by Formula 1 allows the substituted compounds to have characteristics suitable for use in organic electroluminescent devices.

### Industrial Applicability

The organic compound of the present invention can be used as a material for an organic layer or a capping layer of an organic electroluminescent device to improve the quality of the device.

When the compound of the present invention is used in a capping layer, the optical properties of the compound ensure an improvement in the lifetime of an organic electroluminescent device using the capping layer while allowing the device to exhibit its original characteristics.

## Claims

1. An organic compound for use in an organic electroluminescent device, represented by Formula 1: 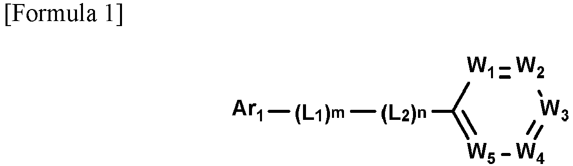 wherein W₁ to W₅ are each independently N or CRₐ, Rₐ is selected from H, F, CF₃, and Si(CH₃)₃, L₁ and L₂ are each independently selected from phenylene, pyridylene, and naphthalene groups, m is 0 or 1, n is an integer from 1 to 5, and Ar₁ is selected from a phenyl group, a phenyl group substituted with F, CF₃ or Si(CH₃)₃, a pyridyl group, a naphthyl group, a quinoline group, an isoquinoline group, a dibenzofuran group, a dibenzothiophene group, a benzoxazole group, a benzothiazole group, and a benzimidazole group.

2. The organic compound according to claim 1, wherein the compound of Formula 1 is selected from compounds represented by Formulae 2 to 5: 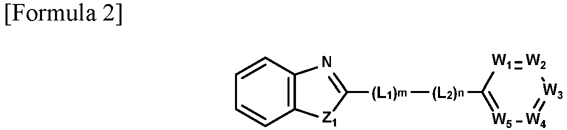
wherein Z₁ is O or S and W₁ to W₅, L₁, L₂, m, and n are as defined in Formula 1, 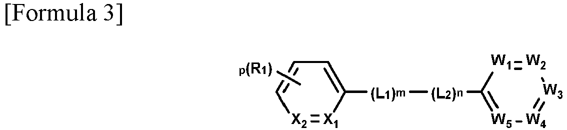
wherein X₁ and X₂ are each independently CH or N (with the proviso that at least one of X₁ and X₂ is CH), R₁ is selected from H, F, CF₃, and Si(CH₃)₃, p is an integer from 0 to 5, and W₁ to W₅, L₁, L₂, m, and n are as defined in Formula 1, 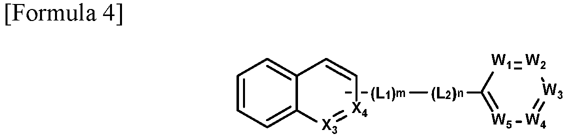
wherein X₃ and X₄ are each independently CH or N (with the proviso that at least one of X₃ and X₄ is CH) and W₁ to W₅, L₁, L₂, m, and n are as defined in Formula 1, and 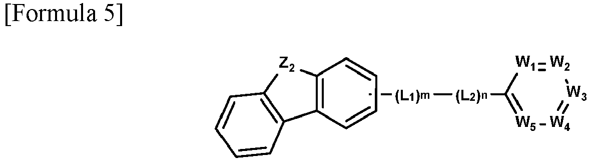
wherein Z₂ is O or S and W₁ to W₅, L₁, L₂, m, and n are as defined in Formula 1.

3. The organic compound according to claim 1, wherein the compound of Formula 1 is selected from the compounds represented by Formulae 6:

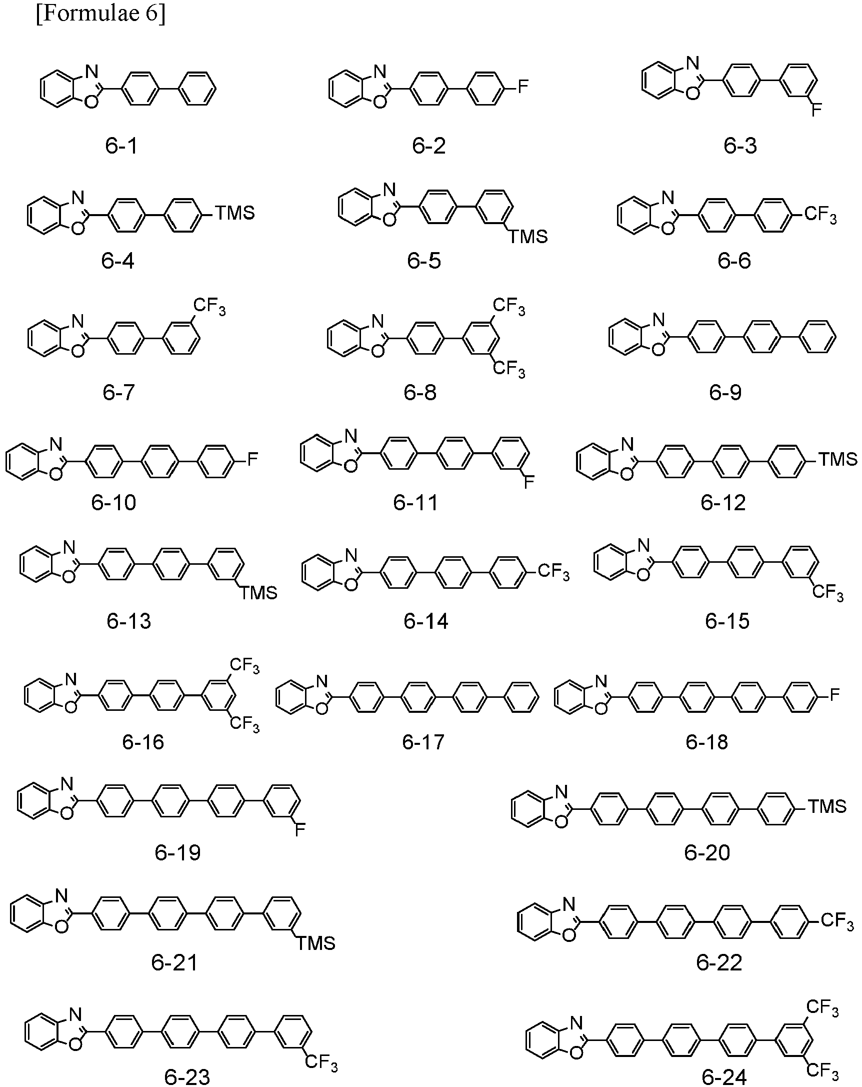

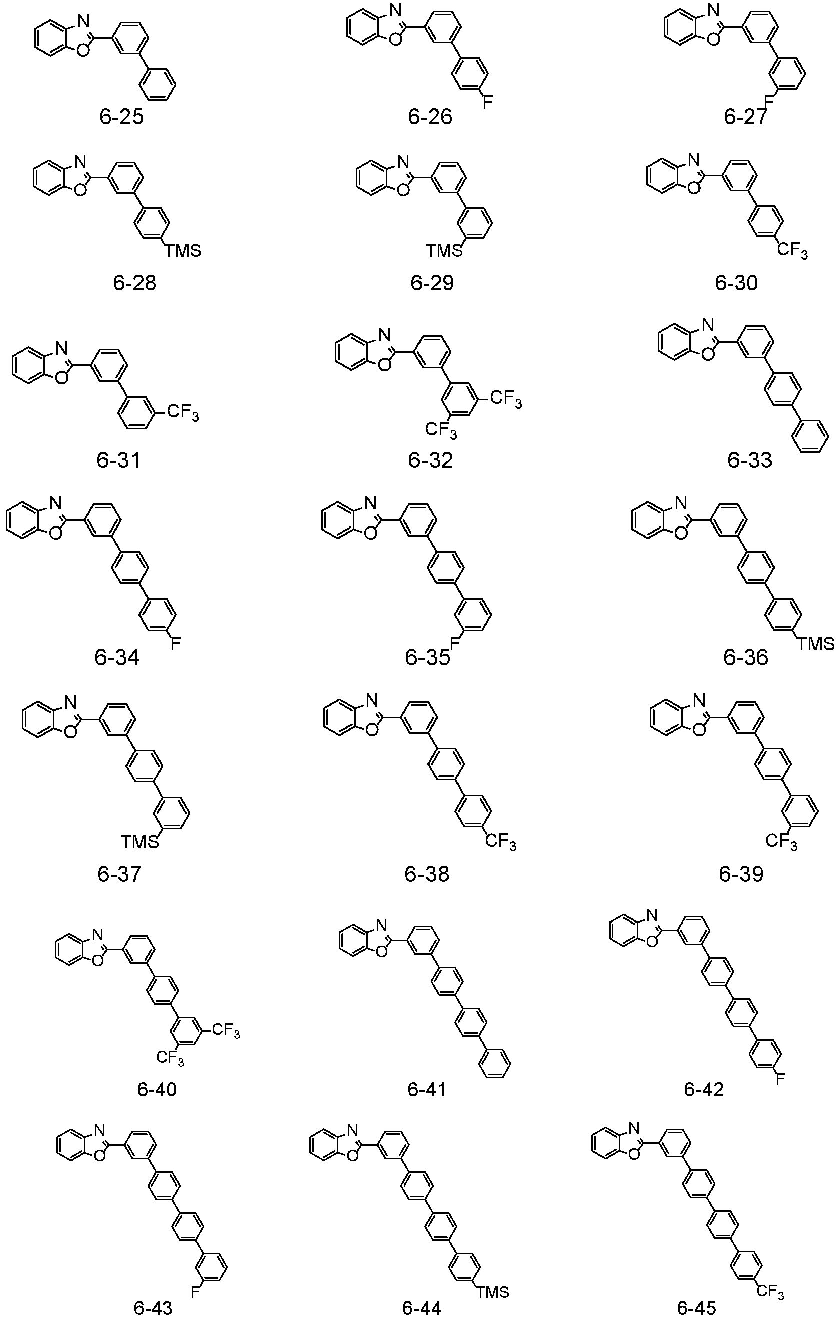

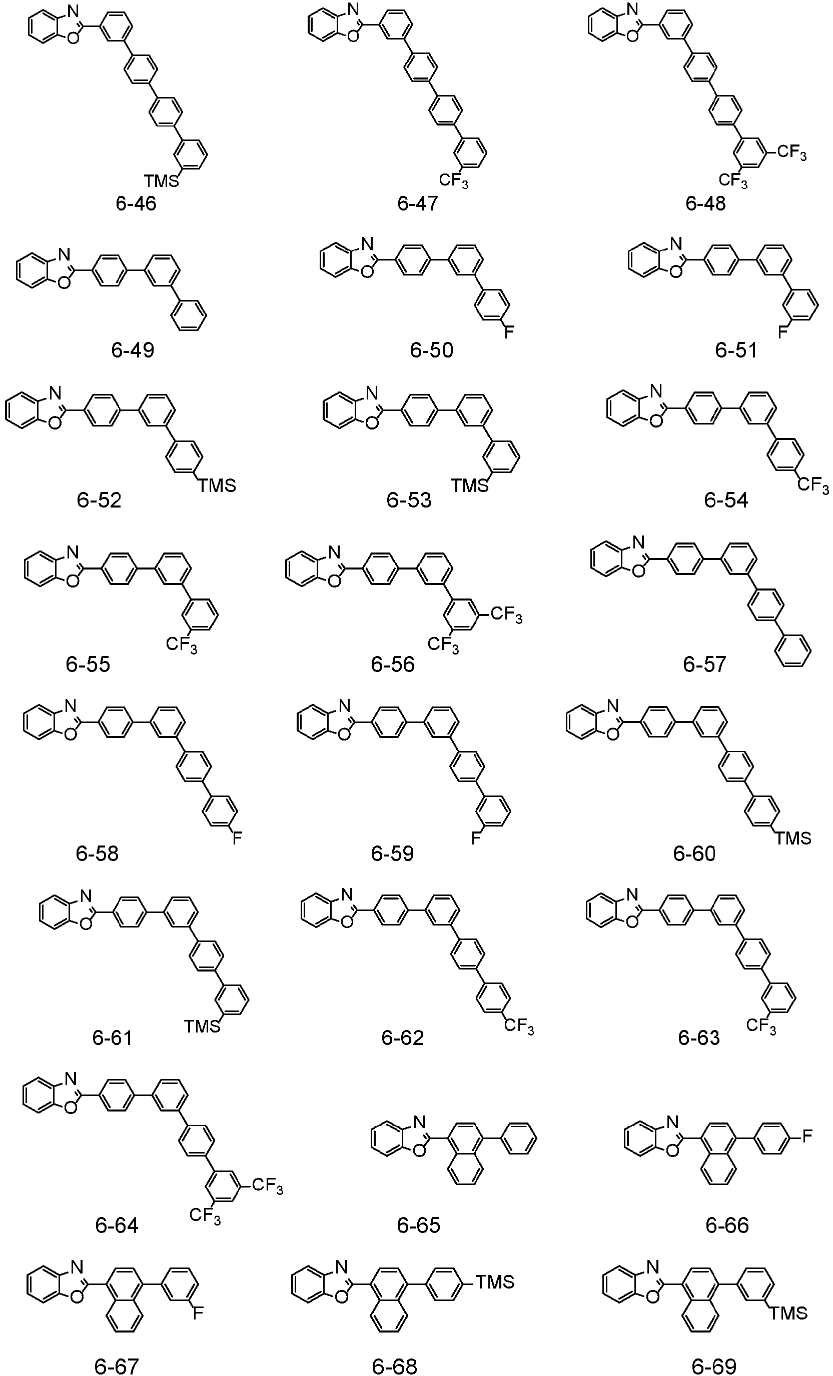

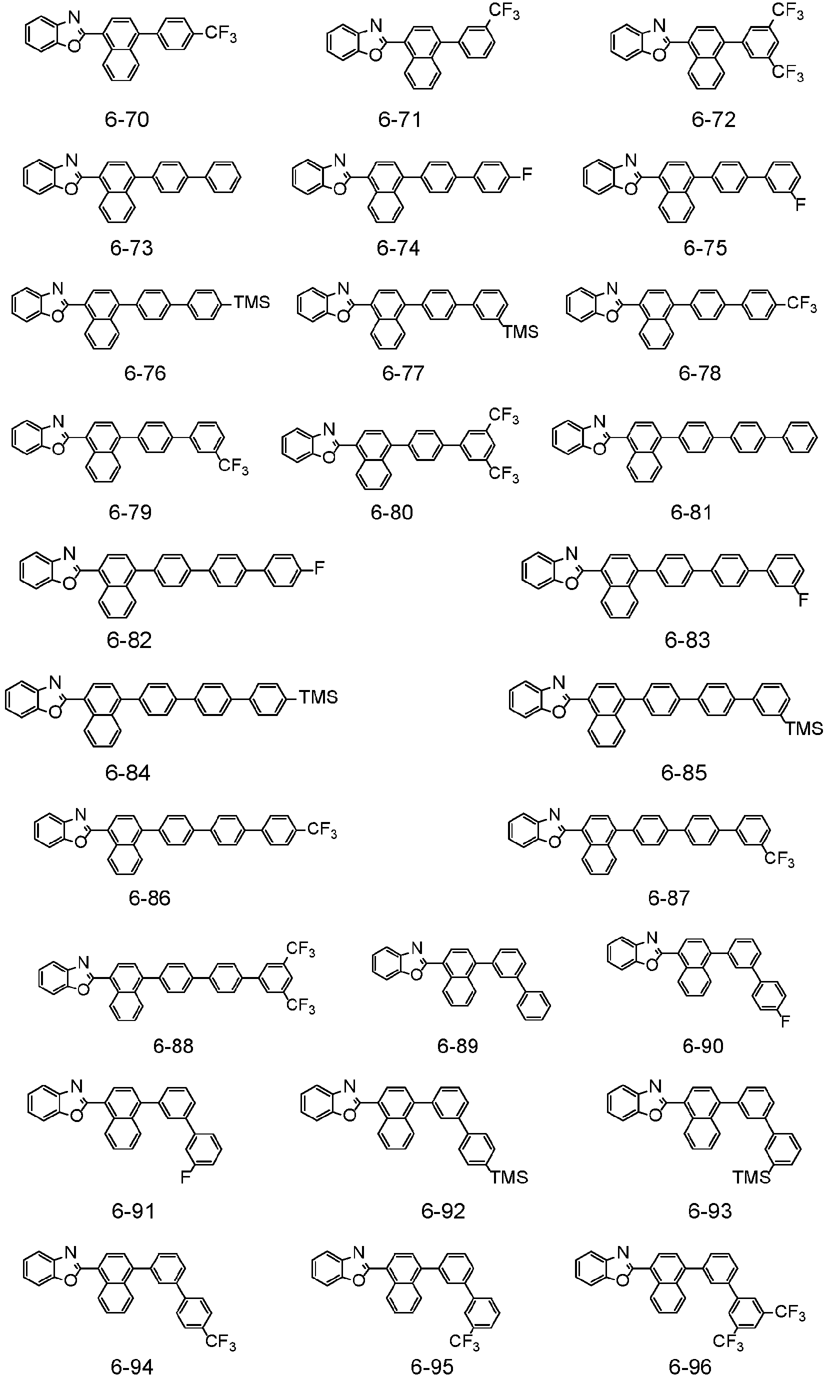

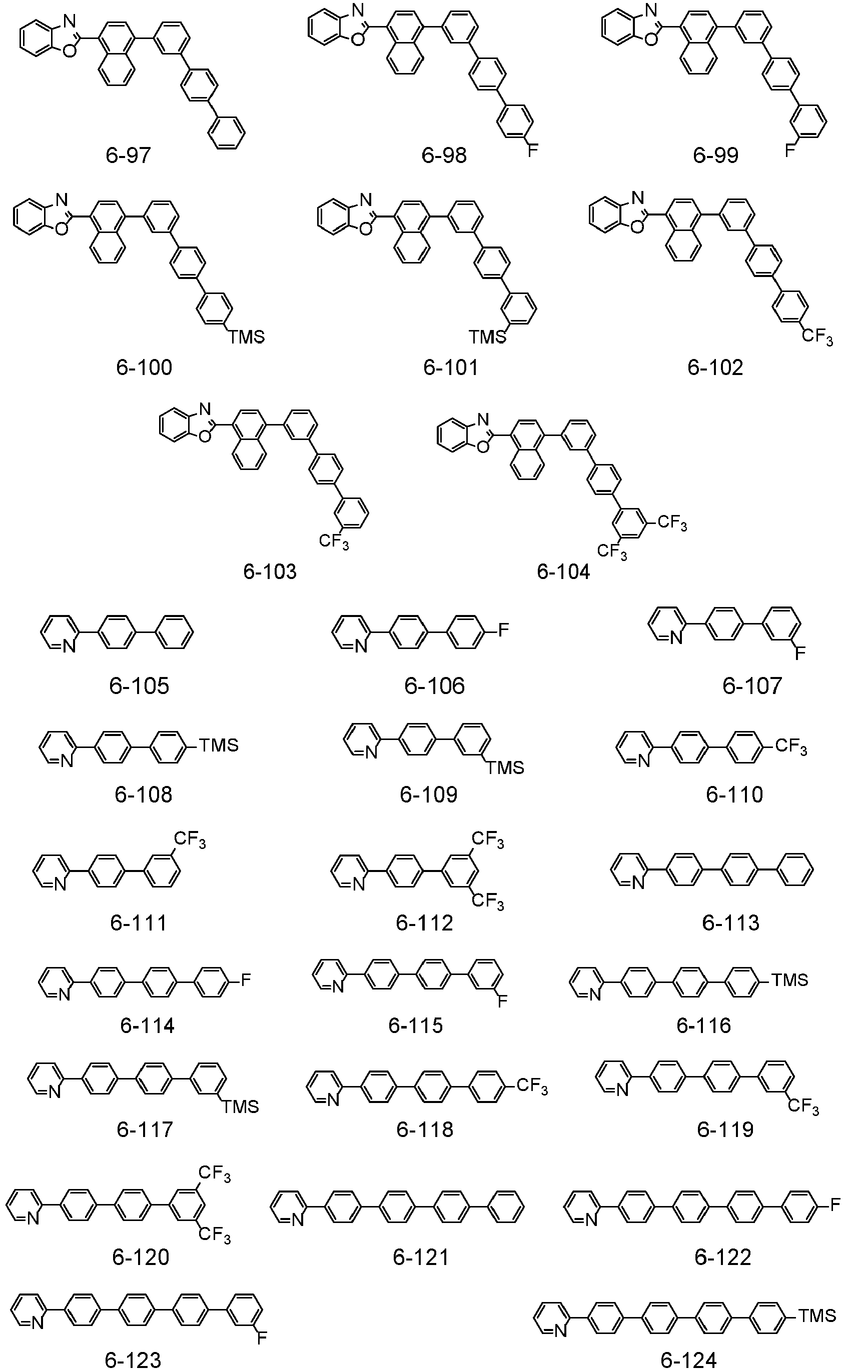

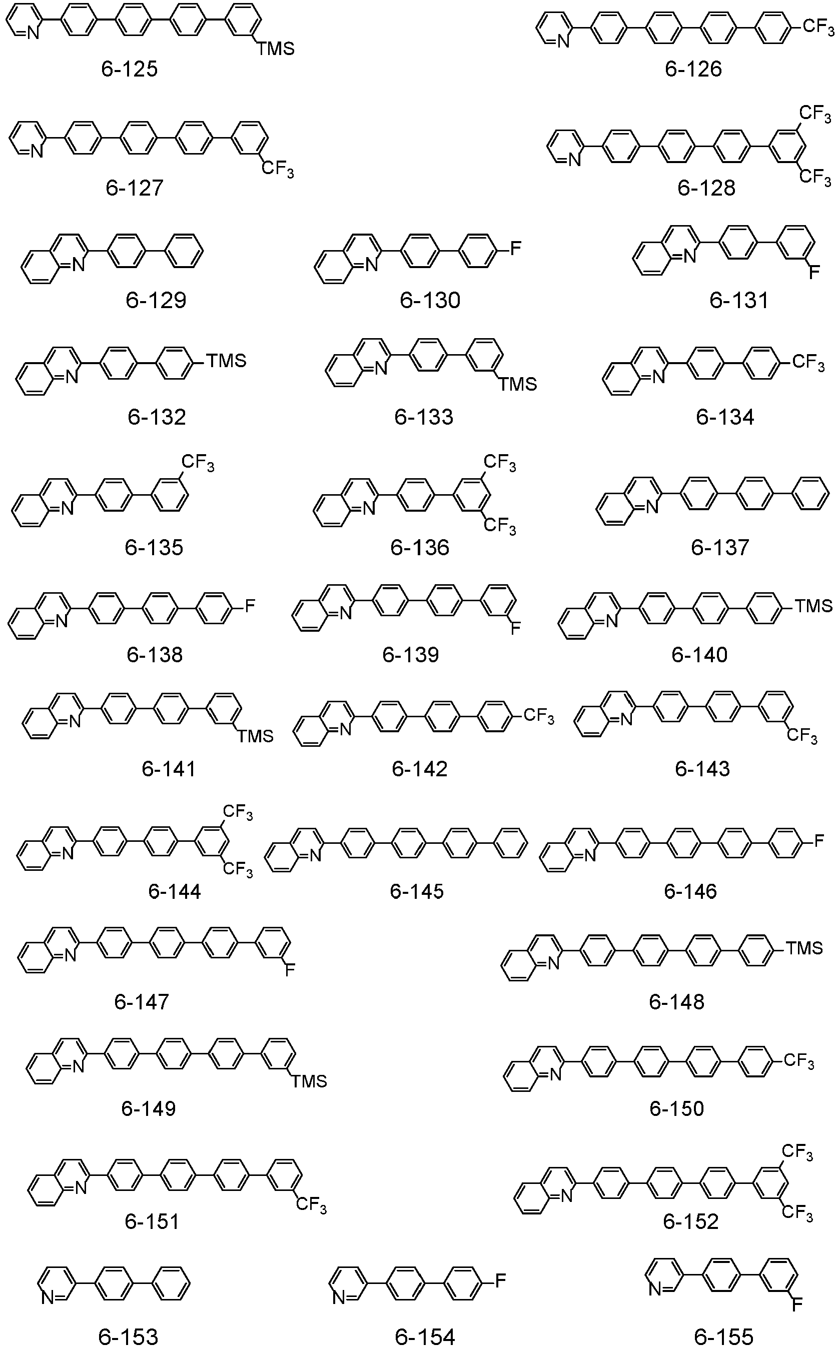

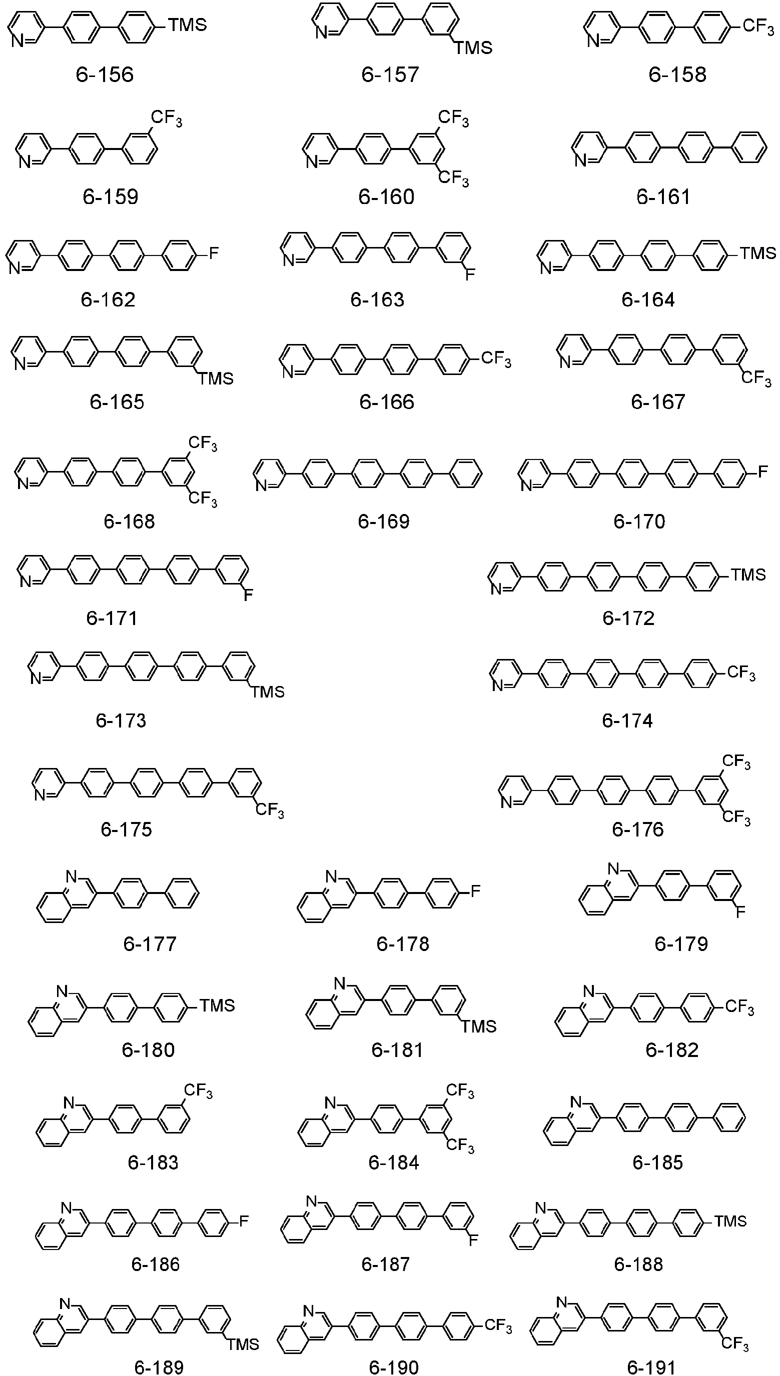

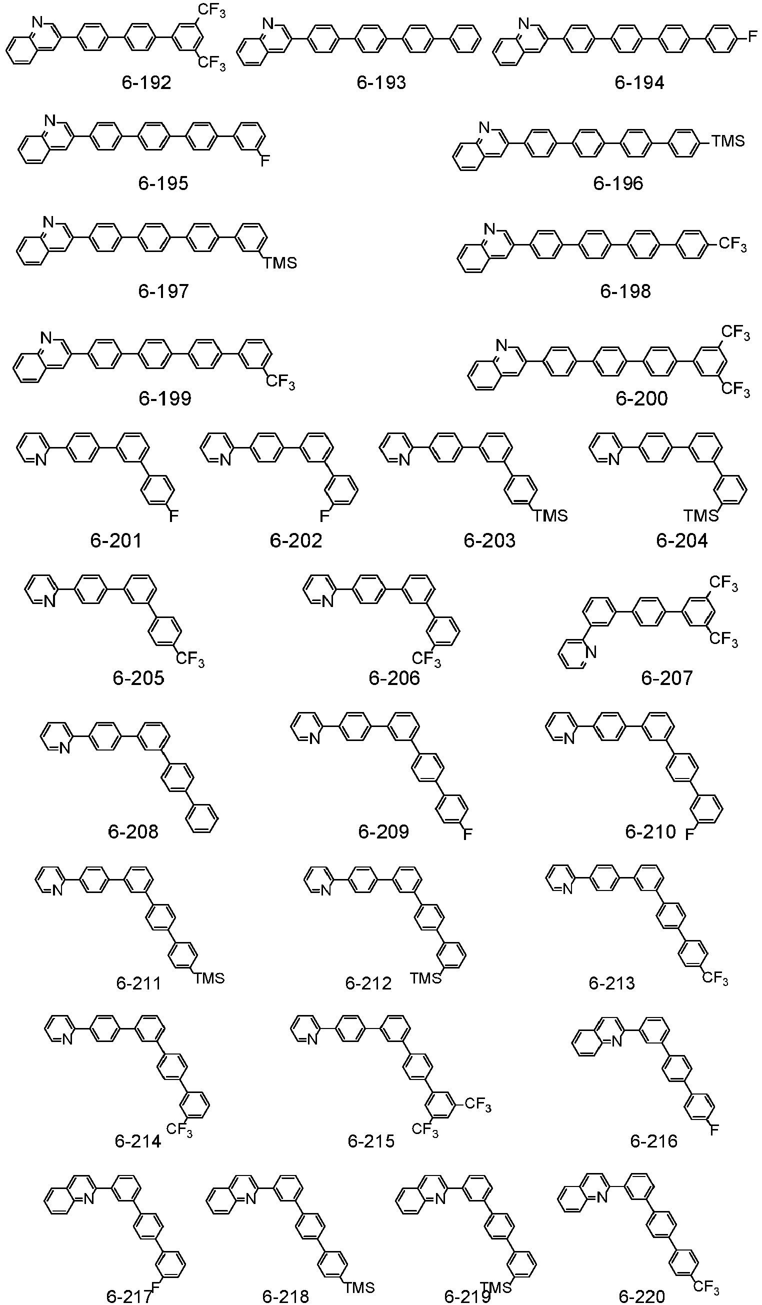

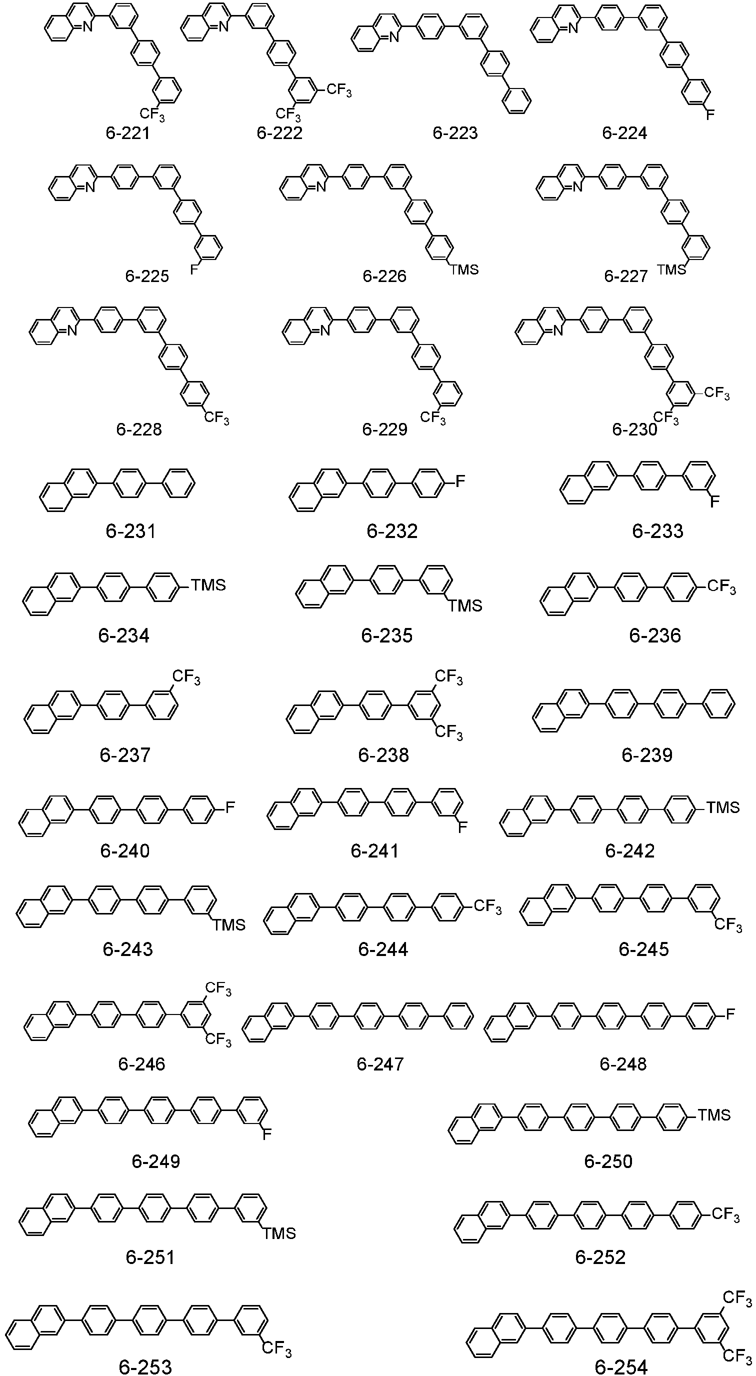

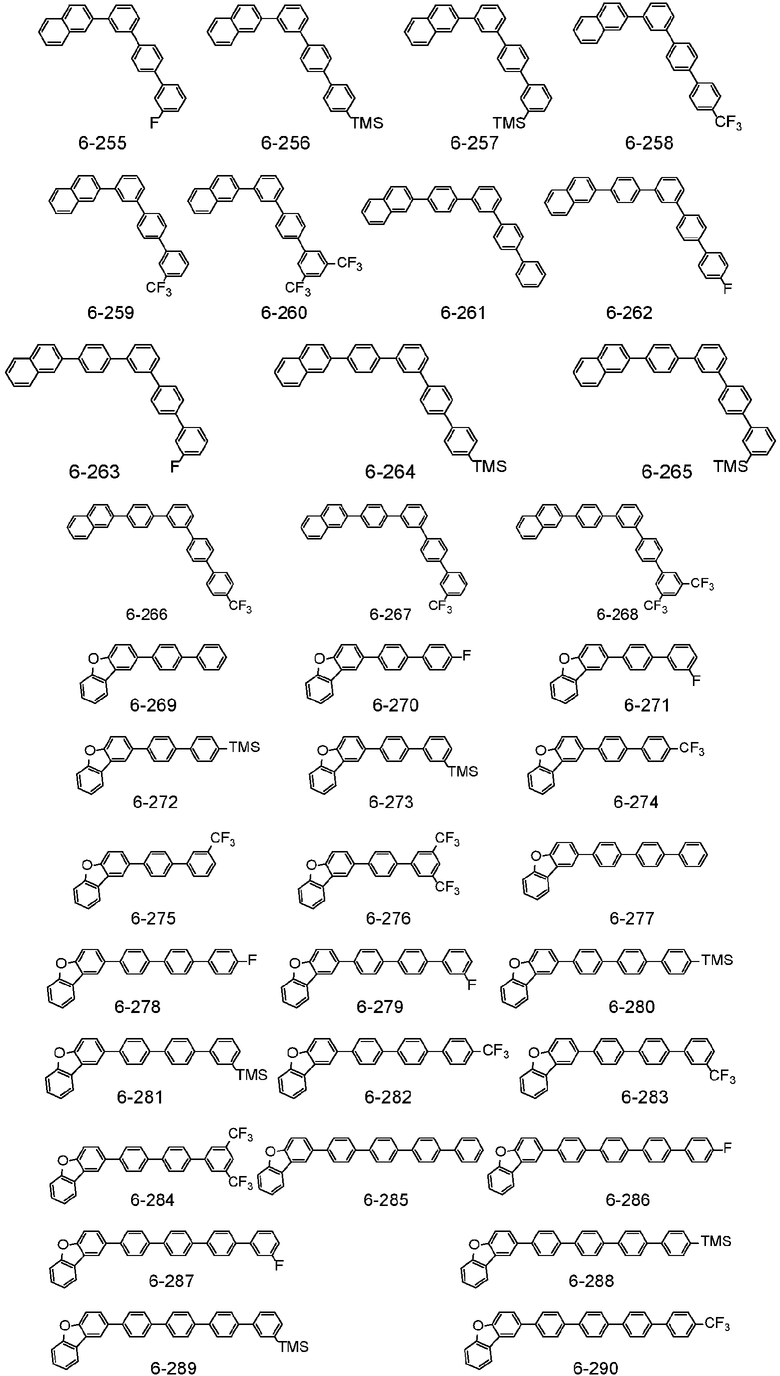

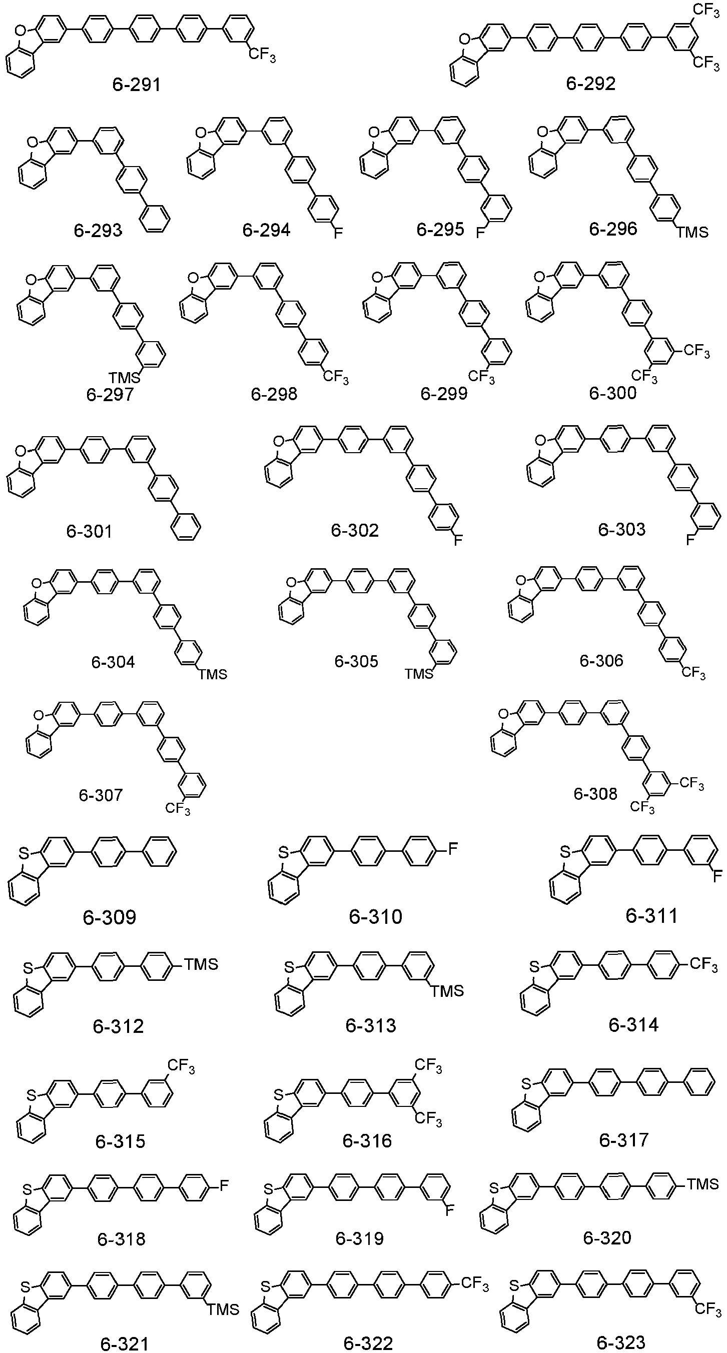

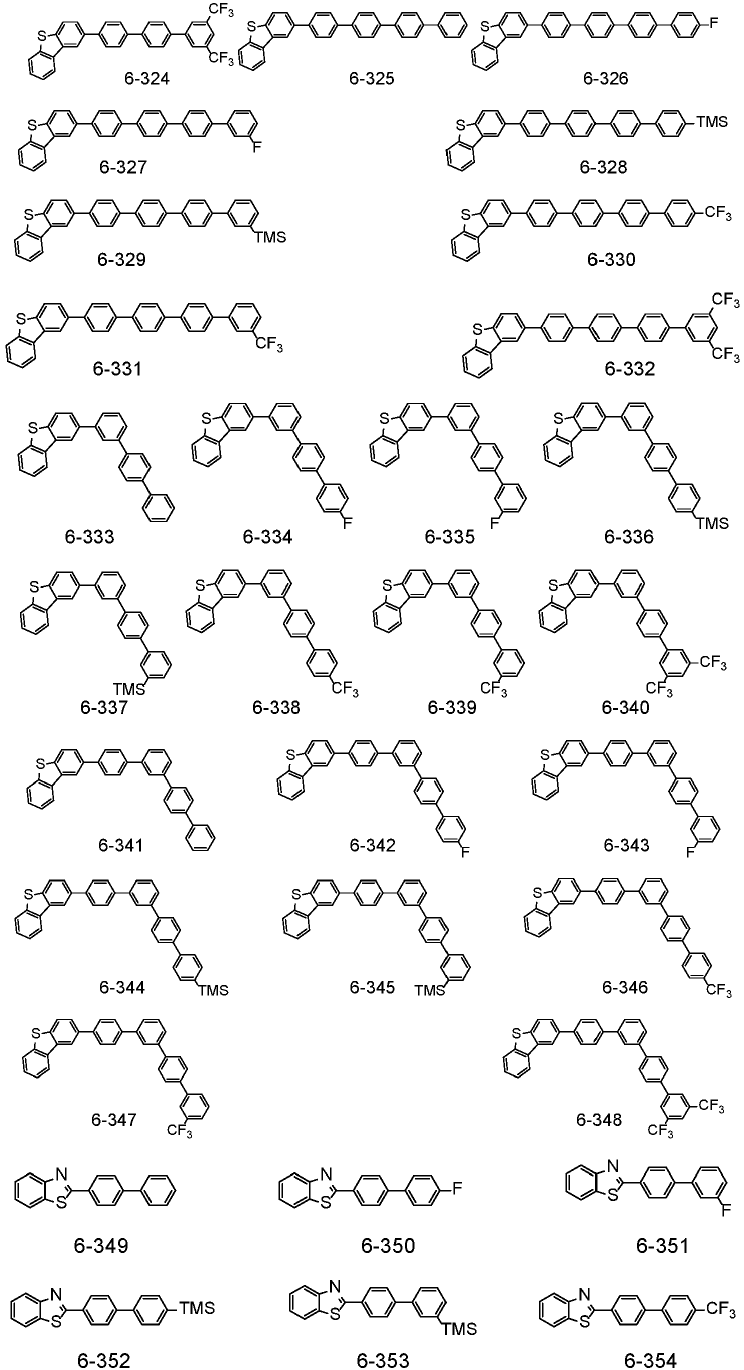

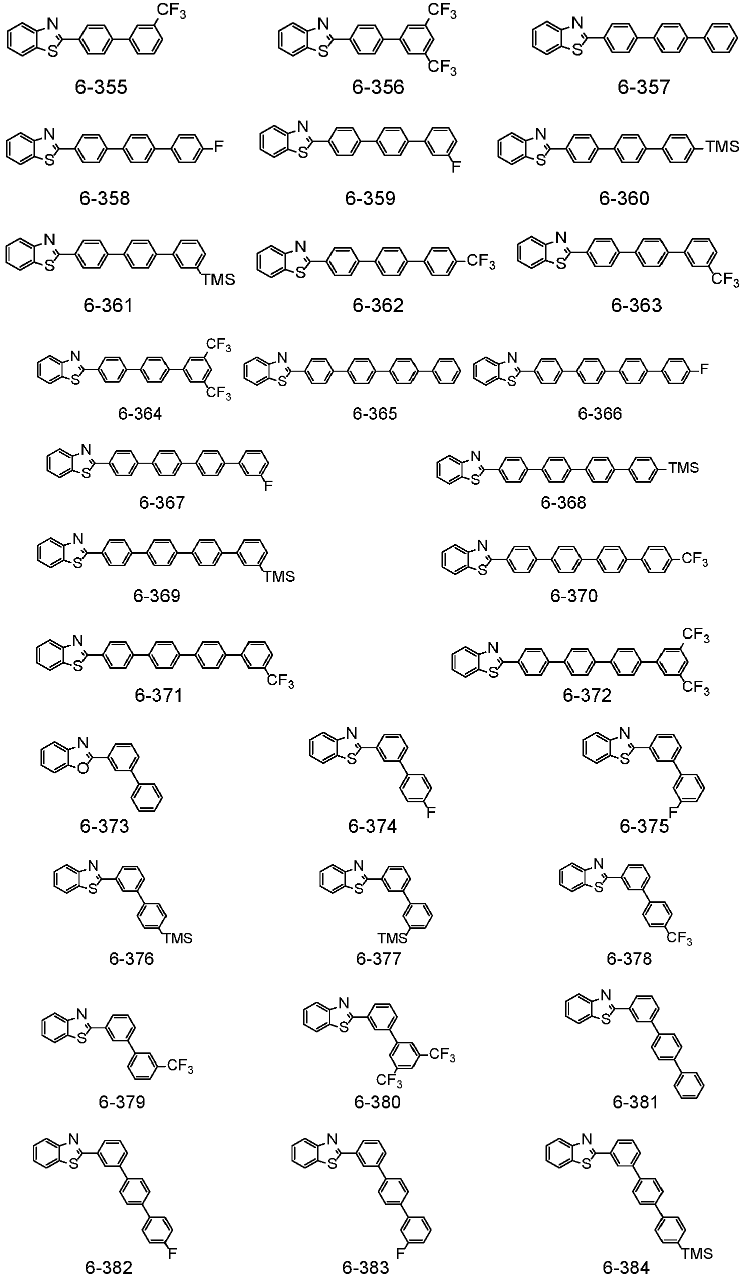

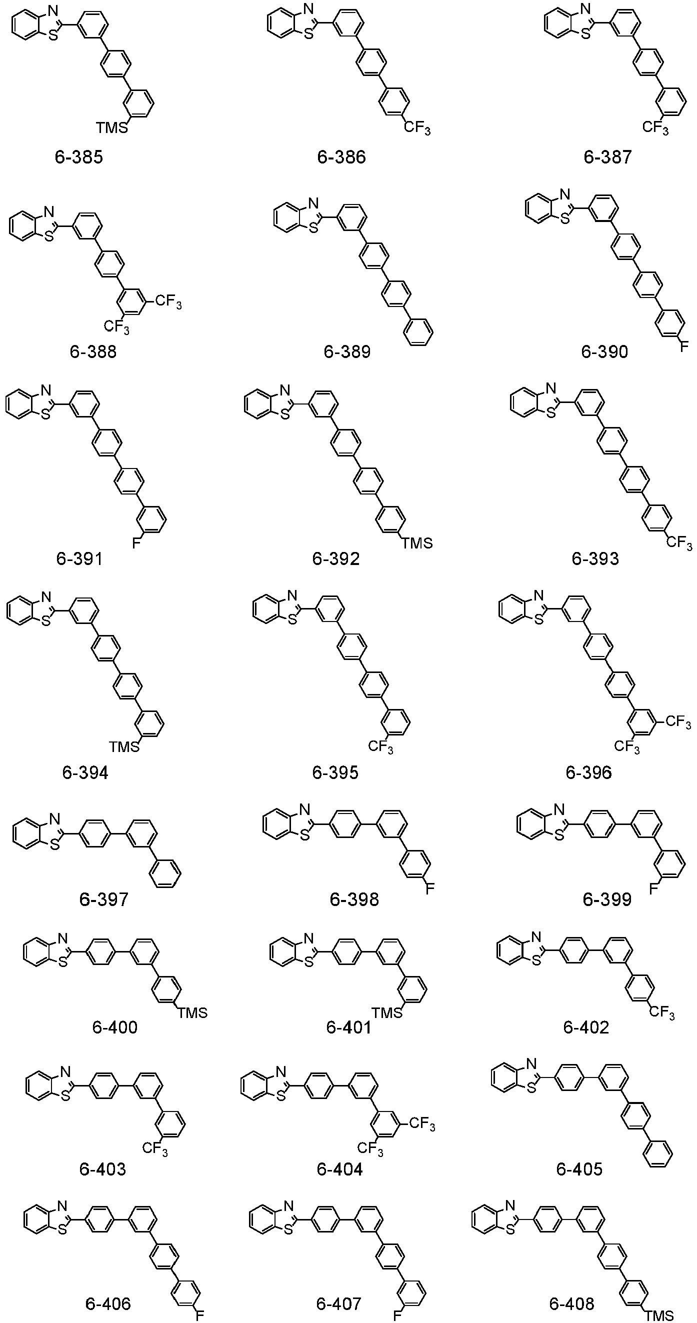

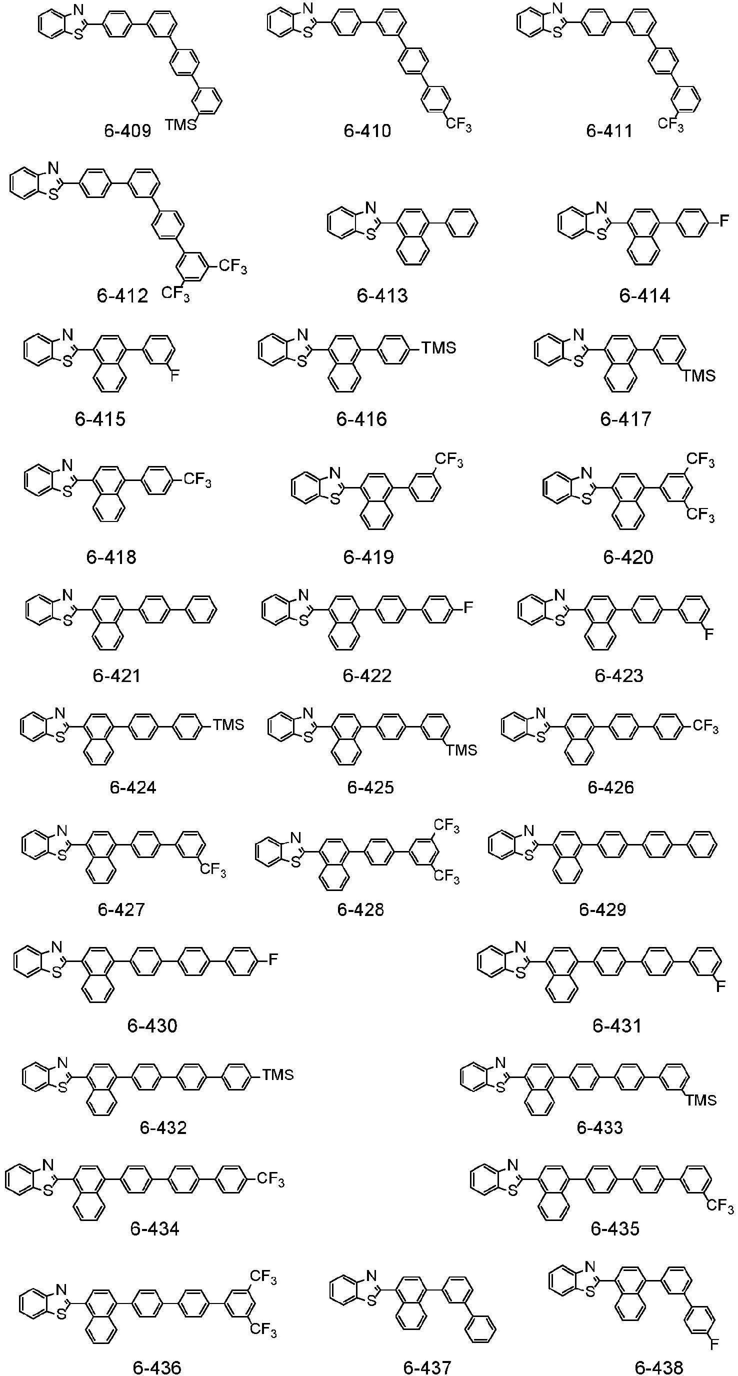

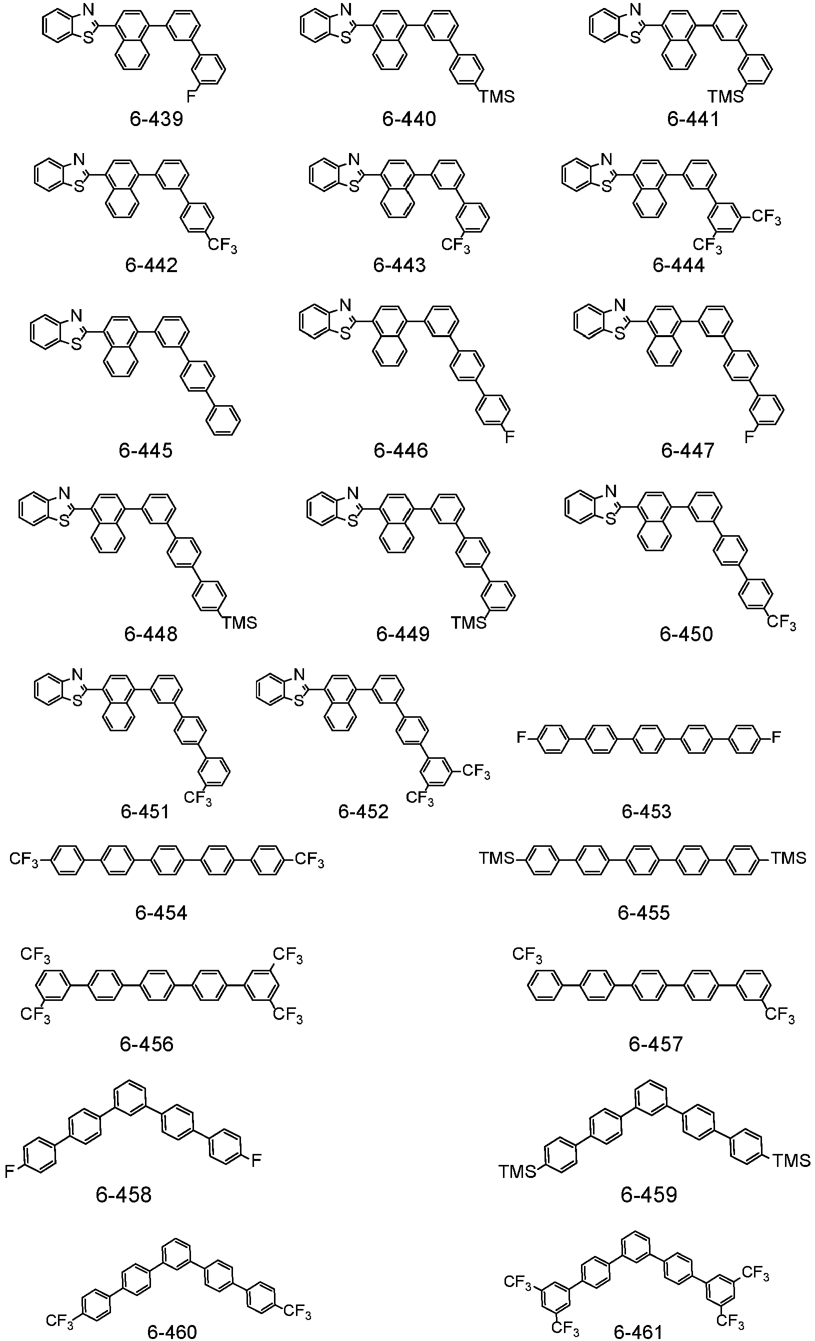

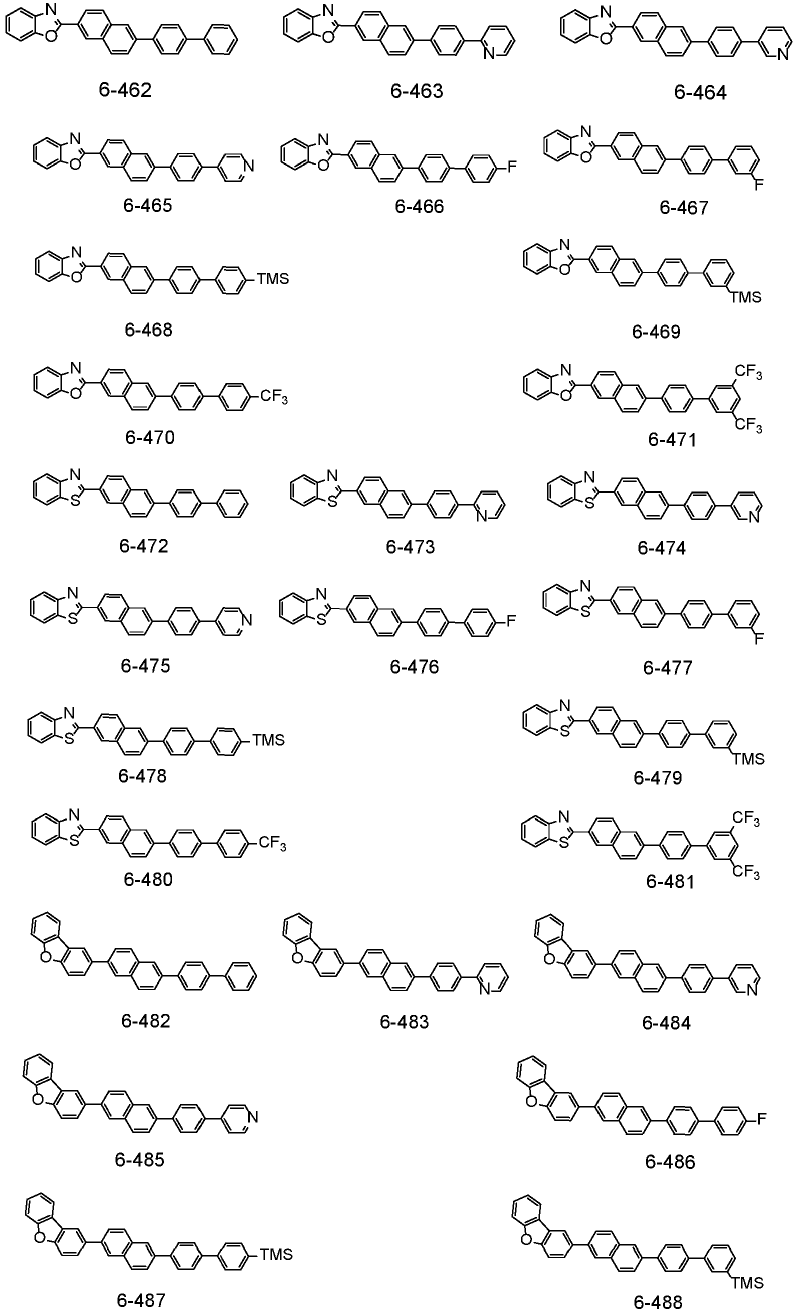

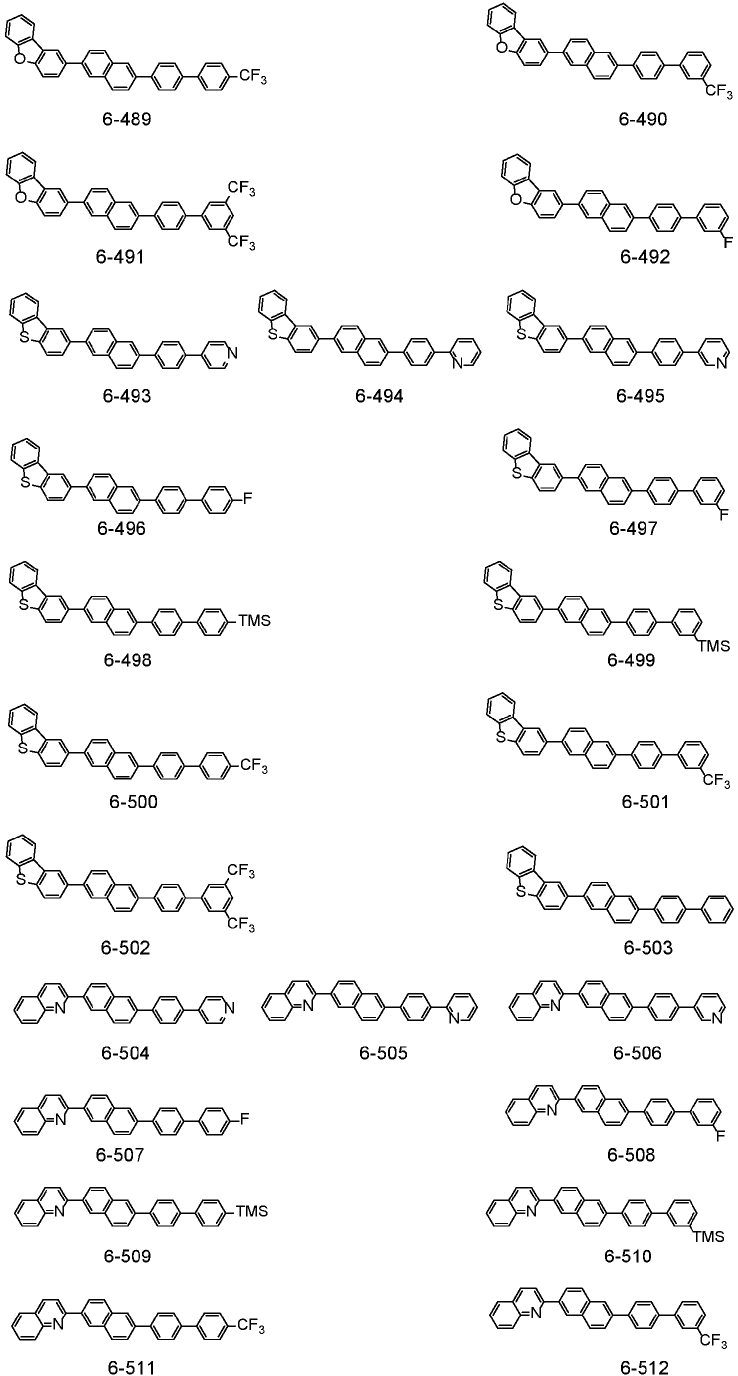

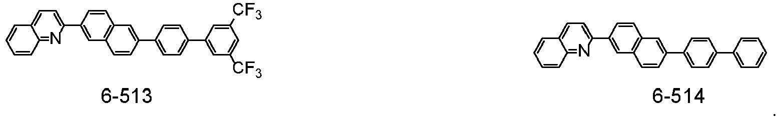

4. An organic electroluminescent device comprising a first electrode, an organic layer consisting of a plurality of layers arranged on the first electrode, a second electrode arranged on the organic layer, and a capping layer arranged on the second electrode wherein the organic layer or the capping layer comprises the organic compound according to any one of claims 1 to 3.

5. The organic electroluminescent device according to claim 4, wherein the organic layer comprises an emission layer and an electron transporting layer and wherein the electron transporting layer comprises the organic compound.
